# EUROPEAN PATENT SPECIFICATION

(11) **EP 1 475 094 B1**
(45) Date of publication and mention of the grant of the patent: **28.07.2010**
(21) Application number: 03010184.4
(22) Date of filing: 06.05.2003
(51) Int. Cl.: A61K 31/505, C07D 487/04

(54) **Pyrazolo[4,3-D]pyrimidines, process for their preparation and methods of use**
Pyrazolo[4,3-D]pyrimidines, verfahren zur ihre herstellung und deren verwendung
Pyrazolo[4,3-D]pyrimidines, procede de preparation et d'utilisation

(43) Date of publication of application: 10.11.2004
(73) Proprietor: Ustav Experimentalni Botaniky Akademie ved Ceské Republiky, 165 02 Praha 6 (CZ); Universita Palackeho v Olomouci, 771 47 Olomouc (CZ)
(72) Inventor: Moravcova, Daniela, 34101 Horzdovice (CZ); Havlicek, Libor, 14104 Praha 4 (CZ); Krystof, Vladimir, 70200 Ostrava (CZ); Lenobel, Rene, 73802 Frydek Mistek (CZ); Binarova, Pavla, 77200 Olomouc (CZ); Mlejnek, Petra, 60200 Brno (CZ); Vojtesek, Borek, 66442 Modrice (CZ); Uldrijan, Stjepan, 62100 Brno (CZ); Schmülling, Thomas, 14052 Berlin (DE); Strnad, Miroslav, 77900 Olomouc (CZ)
(74) Representative: Grund, Martin

(56) References cited:
- WO-A-01/18170
- DE-A- 10 060 388
- US-A- 1 530 747
- US-A- 4 282 361
- ELLAMES G J ET AL: "THE SYNTHESES OF ACYCLOFORMYCINS AND 5-AMINO-3-(2-HYDROXYETHOXY)-METH YLPYRAZOLO[4,3-D]PYRIMIDIN-7(6H)- ONE AN ANALOGUE OF THE ANTIVIRAL ACYCLOGUANOSINE" JOURNAL OF THE CHEMICAL SOCIETY, PERKIN TRANSACTIONS 1, CHEMICAL SOCIETY. LETCHWORTH, GB, no. 10, 1 October 1985 (1985-10-01), pages 2087-2091, XP002047056 ISSN: 0300-922X
- LONG ROBERT A ET AL: "Derivatives of the new ring system pyrazolo[4,3-d]-v-triazine and the synthesis of 5,7-disubstituted 3-methylpyrazolo[4,3- d]pyrimidines and 5,7-disubstituted 3-methylpyrazolo[4,3- d]pyrimidine 6-oxides which are structurally related to the nucleoside antibiotics formycin and formycin B" JOURNAL OF HETEROCYCLIC CHEMISTRY, HETEROCORPORATION. PROVO, US, vol. 7, no. 4, 1970, pages 863-869, XP002208252 ISSN: 0022-152X
- HECHT SM ET AL: "cytokinins: development of a potent antagonist" PROCEEDING OF THE NATIONAL ACADEMIE OF SCIENCE USA, vol. 68, no. 10, 1971, pages 2608-2610, XP009015448 USA
- HECHT SM ET AL: "question of the ribosyl moiety in the promotion of callus growth by exogenously added cytokinins" BIOCHEMISTRY, vol. 10, no. 23, 1971, pages 4224-4228, XP002250881 USA
- HECHT SM ET AL: "on the activation of cytokinins" THE JOURNAL OF BIOLOGICAL CHEMISTRY, vol. 250, no. 18, 1975, pages 7343-7351, XP002250882 USA
- SKOOG F ET AL: "cytokinin antagonists: synthesis and physiological effects of 7-substituted 3-methylpyrazolo[4,3-d]pyrimidines" PHYTOCHEMISTRY, vol. 12, 1973, pages 25-37, XP009015452 UK
- GREGORINI G ET AL: "biological effects of cytokinin antagonists 7-(pentylamino) and 7-(benzylamino)-3-methylpyrazolo[4,3-d]pyr imidines on suspension-cultured tobacco cells" PLANT PHYSIOLOGY, vol. 65, 1980, pages 363-367, XP001154399
- HECHT SM ET AL: "competitive inhibition of beef heart cyclic AMP phosphodiesterase by cytokinins and related compounds" PROCEEDING OF THE NATIONAL ACADEMIE OF SCIENCES USA, vol. 71, no. 12, 1974, pages 4670-4674, XP009015447 USA
- DIZENGREMEL P ET AL: "inhibition by adenine derivatives of the cyaninde insensitive electron transport pathway of plant mitochondria" PLANT PHYSIOLOGY, vol. 70, 1982, pages 585-589, XP001154398
- BIANCO-COLOMANS J : "effect of a cytokinin antagonist on cytokinin and light-dependent amaranthin synthesis in amaranthus tricolor seedlings" JOURNAL OF PLANT GROWTH REGULATION, vol. 2, no. 4, 1984, pages 281-287, XP009015443 Germany
- PARKER CW ET AL: "inhibitors of two enzymes which metabolize cytokinins" PHYTOCHEMISTRY, vol. 25, no. 2, 1986, pages 303-310, XP001154032 GB
- AUNG LH: "Action of cytokinins and anticytokinins on cotyledonary bud growth of lycopersicon esculentum mill" BIOLOGIA PLANTARUM, vol. 28, no. 6, 1986, pages 407-411, XP009015451 praha
- WIERZCHOWSKI J ET AL: "luminiscence studies on formycin, its aglycone, and thier N-methyl derivatives: tautomerism, sites of protonation and phototautomerism" PHOTOCHEMISTRY AND PHOTOBIOLOGY, vol. 35, 1982, pages 445-458, XP009015440 GB
- ROBINS RK ET AL: "potential purine antagonists V. synthesis of some 3-methyl-5,7-substituted pyrazolo[4,3-d]pyrimidines" THE JOURNAL OF ORGANIC CHEMISTRY, vol. 21, no. 8, 1956, pages 833-836, XP002250883 USA
- WIERZCHOWSKI J ET AL: "analogues of formycins A and B: synthesis and some properties of methyl derivatives of 7-amino and 7-keto pyrazolo[4,3-d]pyrimidines" ACTA BIOCHIMICA POLONICA, vol. 27, no. 1, 1980, pages 35-56, XP009015441 Poland
- BUCHANAN JG ET AL: "C-nucleoside studies part 18 the synthesis of C-nucleoside analogues of the antiviral agent (S)-9-(2,3-dihydroxypropyl)adenine" JOURNAL OF THE CHEMICAL SOCIETY PERKIN TRANSACTIONS I, 1985, pages 1425-1430, XP009015442 GB

## Description

This invention relates to new pyrazolo[4,3-d]pyrimidine derivatives and to their use in suitable utilities, especially in cancer therapy and agricultural practice.

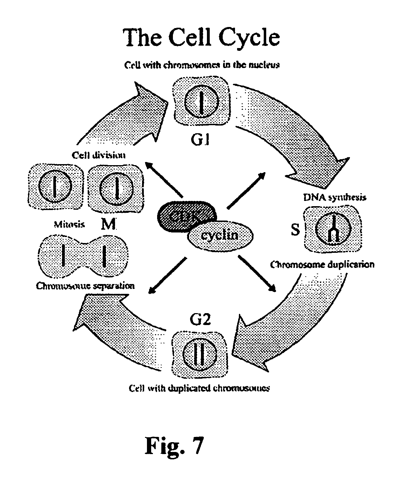

The cell division cycle is an evolutionarily conserved process in all eukaryotic cells to control growth and division. The cell cycle consists of four distinct stages illustrated in Figure 7, the G1, S, G2 and M phase. Normal cellular proliferation is initiated and tightly controlled by a series of regulatory mechanisms that either permit or prevent cell cycle progression. In every phase, there are protein complexes, the cyclins and cyclin-dependent kinases regulating and advancing the cell cycle. Figure 7 shows the cell division cycle (cdc) consisting of four phases G1, S, G2 and M. Mitosis (the actual division) occurs in M-phase. In every phase, there are specific cyclin-dependent kinase complexes present (CDK's).

Proliferative disorders such as cancer are recognised as diseases of the cell cycle. It has been found that in tumour cells, the mechanisms that normally function to restrain cell division are defective, whilst those that promote division become more active. The genes responsible for these changes in growth and proliferation are generally named "tumour suppressors" and "oncogenes". Cell-cycle regulatory compounds are pivotal in the modulation of abnormal cellular proliferation as they provide ideal targets for therapy for a range of proliferative disorders.
A series of 3-,7-disubstituted pyrazolo[4,3-d]pyrimidines are useful for inhibition of cyclin-dependent kinases (preferably p34^{cdc2}/cyclin B). Hence they can be used as antimitotic and apoptotic drugs, particularly as anticancer drugs and herbicides. Likewise, the compounds can be used as anti-fungal agents, which may have high value in the treatment of aspergillosis, penicilliosis, actinomycosis and the like. Difference in homology of insect CDK genes permit selection of compounds of this invention which discriminate between insect/mammalian CDK enzymes and thus leads to insecticides.

### Summary of the Invention

It is an object of this invention to provide antimitotic, anticancer, herbicidal, fungicidal and insecticidal compounds having improved selectivity and efficiency index, i.e. that are less toxic yet more efficacious than analogues known heretofore.

It is an object of this invention to provide 3,7-disubstituted pyrazolo[4,3-d]pyrimidines, which inhibit the cdks, cell proliferaton or block cytokinin receptors.

A further object of this invention is to provide a pharmaceutical composition, which comprises a 3,7-disubstituted pyrazolo[4,3-d]pyrimidine, and a pharmaceutically acceptable carrier.

A further object of this invention to provide a method for inhibiting cell proliferation and/or inducing apoptosis to a mammal or plant in need of an effective amount of 3,7-disubstituted pyrazolo[4,3-d]pyrimidines.

This invention further constitutes a method for inhibiting cell proliferation to a plant in need of an effective amount 3,7-disubstituted pyrazolo[4,3-d]pyrimidines.

The solution of this object are 3-, 7-disubstituted pyrazolo[4,3-d]pyrimidines represented by the general formula **I** 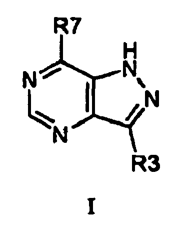 and pharmaceutically acceptable salts thereof, wherein
**R3** is selected from the group consisting of ethyl, iso-propyl, n-butyl, 3-pentyl,
cycloalkyl, cycloalkyl alkyl, arylalkyl, alkenyl and alkynyl wherein each of the groups may optionally be substituted,
**R7** is selected from the group consisting of halogen, hydroxyl, hydroxylamino, amino, carboxyl, cyano, nitro, amido, sulfo, sulfamido, carbamino, unsubstituted or **R7'-X-** wherein **X** is an -NH-, or -N(alkyl)- moiety and
**R7'** is selected from the group consisting of H, alkyl, cycloalkyl, aryl, alkylcycloalkyl, arylalkyl, heterocycle, heterocycloalkyl, substituted alkyl, substituted cycloalkyl, substituted aryl, substituted arylalkyl, substituted heterocycle, substituted heteroaryl, substituted heteroarylalkyl, substituted heteroalkyl, substituted cycloalkyl alkyl and substituted cycloheteroalkyl alkyl.
If the above groups are substituted, they are preferably substituted by halogen, hydroxyl, amino, mercapto, carboxyl, cyano, nitro, amido, sulfo, sulfamido, carbamino, alkyl, alkoxy, and/or substituted alkyl group, in particular by more than one of the above substituents, preferably by 1 to 3 substituents.

In another embodiment, this invention relates to the use of the compounds as defined in the claims for inhibiting cdks and cell proliferation and/or for inducing apoptosis in plants, comprising administering an effective amount of a composition comprising one or more compounds according to the claims to the plant. The cdk inhibiting molecules are useful for treating disorders, some of them involving cell proliferation, and thus are useful as herbicides.

In yet another embodiment, this invention is a pharmaceutical composition comprising one or more compounds according to the claims in an admixture with one or more pharmaceutical excipients.

In still another embodiment, this invention is a composition comprising one or more compounds according to the claims useful for treating fungal infections (fungi) in plants.

In another embodiment, this invention is a composition comprising one or more compounds according to the claims useful for treating insects and yeasts on plants.

3,7-disubstituted pyrazolo[4,3-d]pyrimidines result in the acquisition of extremely high potency against plant viruses on the part of the defined compounds. As used herein, and unless modified by the immediate context:
"Halogen" preferably refers to fluorine, bromine, chlorine and iodine atoms.
"Hydroxy" refers to the group -OH.
"Mercapto" refers to group -SH.
"Alkyl" preferably refers to branched or unbranched C₁-C₈ alkyl chain which is saturated or unsaturated. Thus, the term "alkyl" when used herein encompasses alkyl alkenyl and alkinyl groups. Alkenyl groups preferably have 2 to 8 carbon atoms, alkinyl groups preferably have 3 to 8 carbon atoms. Such groups as methyl, ethyl, propyl, isopropyl, n-butyl, isobutyl, tert-butyl, allyl, ethinyl, propargyl, and the like can exemplify this term.
"Substituted alkyl" preferably refers to alkyl as described above including one to six, in particular 1 to 3 substituents such as hydroxyl, mercapto, alkylthio, halogen, alkoxy, acyloxy, amino, acylamino, hydrazino, carbamoyl, amido, carboxyl, sulfo, acyl, guanidino and the like. These groups may be attached to any carbon atom of the alkyl moiety.
"Alkoxy" denotes the group -OR, where R is preferably alkyl, substituted alkyl, aryl, substituted aryl, arylalkyl, substituted arylalkyl, cycloalkyl, substituted cycloalkyl, cycloheteroalkyl or substituted cycloheteroalkyl as defined herein.
"Alkylthio" denotes the group -SR, where R is preferably as defined for "alkoxy" group.
"Sulfo" denotes the group ―SO₃R, where R is preferably H, alkyl or substituted alkyl as defined above.
"Sulfamido" denotes to the group SO₂NRR" where R and R" is preferably H, alkyl or substituted alkyl as defined above.
"Acyl" denotes groups -C(O)R, where R preferably is alkyl, substituted alkyl, aryl, substituted aryl, arylalkyl, substtituted arylalkyl, cycloalkyl, substituted cycloalkyl as defined herein.
"Aryloxy" denotes groups -OAr, where Ar is preferably an aryl, substituted aryl, heteroaryl or substituted heteroaryl group as defined herein.
"Alkylamino" denotes the group -NRR', where R and R'may independently be hydrogen, alkyl, substituted alkyl, aryl, substituted aryl, heteroaryl or substituted heteroaryl as defined herein.
"Amido" denotes the group -C(O)NRR', where R and R'may independently be hydrogen, alkyl, substituted alkyl, aryl, substituted aryl, heteroaryl, substituted heteroaryl as defined herein.
"Carboxyl" denotes the group -C(O)OR, where R is preferably hydrogen, alkyl, substituted alkyl, aryl, substituted aryl, hetaryl or substituted hetaryl as defined herein.
"Acylamino denotes the group ―NHCOR, where R may be alkyl, substituted alkyl, heterocycle, aryl, substituted aryl, heteroaryl and substituted heteroaryl as defined herein.
   Carbamoylamino denotes the group NHCOOR, where R is preferably alkyl or aryl.
"Aryl" or "Ar" refers to an aromatic carbocyclic group having at least one aromatic ring (e.g., phenyl or biphenyl) or multiple condensed rings in which at least one ring is aromatic (e.g., 1,2,3,4-tetrahydronaphthyl, naphthyl, anthryl, or phenanthryl). Preferably, the aryl group has more than six, in particular 6 to 10 carbon atoms.
"Substituted aryl" refers to aryl as described above which is optionally substituted with one or more functional groups, in particular 1 to 3 substituents, such as halogen, alkyl, hydroxy, amino, acylamino, carbamoylamino, hydrazino, mercapto, alkoxy, alyklthio, alkylamino, amido, carboxyl, nitro, sulfo and the like as defined herein.
"Heterocycle" refers to a unsaturated or aromatic carbocyclic group preferably having 1 to 3 rings and having at least one, preferably 1 to 3 and in particular 1 or 2 hetero atoms, such as N, O or S, within the ring; the ring can be single (e.g. pyranyl, pyridyl or furyl) or multiple condensed (e.g., quinazolinyl, purinyl, quinolinyl or benzofuranyl) which can optionally be unsubstituted or substituted with, e.g., halogen, amino, hydroxy, cyano, nitro, mercapto, alkoxy, alkylamino, acylamino, carbamoylamino, acyloxy, dialkylamino, alkylthio carboxyl, amido, sulfo, sulfamido, and the like as defined above. The heterocycle groups preferably has 5 to 10 ring atoms, which are either cabon atoms or hetero atoms as defined above.
"Heteroaryl" refers to a heterocycle in which at least one heterocyclic ring is aromatic.
"Substituted heteroaryl" refers to a heterocycle optionally mono or poly substituted with one or more functional groups, preferably 1 to 6, in particular 1 to 3 substituents, e.g., halogen, amino, hydroxy, cyano, nitro, mercapto, alkoxy, alkylamino, acylamino, carbamoylamino, acyloxy, dialkylamino, alkylthio carboxyl, amido, sulfo, sulfamido, and the like.
"Arylalkyl" refers to the group -R-Ar where Ar is an aryl group and R is alkyl or substituted alkyl group as defined above. The aryl groups can optionally be unsubstituted or substituted as defined above with, e.g., halogen, amino, acylamino, carbamoylamino, hydrazino, acyloxy, alkyl, hydroxyl, alkoxy, alkylthio, alkylamino, amido, carboxyl, hydroxy, aryl, nitro, mercapto, sulfo and the like.
"Heteroalkyl" refers to the group -R-Het where Het is a heterocycle group and R is a alkyl group as defined above. Heteroalkyl groups can optionally be unsubstituted or substituted as defined above with e.g., halogen, amino, hydroxy, cyano, nitro, mercapto, alkoxy, alkylamino, acylamino, carbamoylamino, acyloxy, dialkylamino, alkylthio, carboxyl, amido, sulfo, sulfamido, and the like.
"Heteroarylalkyl" refers to the group -R-HetAr where HetAr is an heteroaryl group and R is alkyl or substituted alkyl as defined above. Heteroarylalkyl groups can optionally be unsubstituted or substituted as defined above with, e.g., halogen, alkyl, substituted alkyl, alkoxy, alkylthio, nitro, mercapto, sulfo and the like.
"Cycloalkyl" refers to a divalent cyclic or polycyclic alkyl group containing preferably 3 to 15 carbon atoms.
"Substituted cycloalkyl" refers to a cycloalkyl group comprising one or more substituents as defined above with, e.g., halogen, amino, hydroxy, cyano, nitro, mercapto, alkoxy, alkylamino, acylamino, carbamoylamino, acyloxy, dialkylamino, alkylthio, carboxyl, amido, sulfo, sulfamido, and the like.
"Cycloheteroalkyl" refers to a cycloalkyl group as defined above wherein one or more, preferably 1 to 3, of the ring methylene group is replaced with a heteroatom (e.g., NH, O, S)
"Substituted cycloheteroalkyl" refers to a cycloheteroalkyl group as herein defined which contains one or more substituents as defined above, such as halogen, amino, hydroxy, cyano, nitro, mercapto, alkoxy, alkylamino, acylamino, carbamoylamino, acyloxy, dialkylamino, alkylthio, carboxyl, amido, sulfo, sulfamido and the like.
"Cycloalkyl alkyl" denotes the group -R-cycloalkyl where cycloalkyl is a cycloalkyl group as defined above and R is an alkyl or substituted alkyl as defined above. Cycloalkyl groups can optionally be unsubstituted or substituted as defined above with e.g., halogen, amino, hydroxy, cyano, nitro, mercapto, alkoxy, alkylamino, acylamino, carbamoylamino, acyloxy, dialkylamino, alkylthio, carboxyl, amido, sulfo, sulfamido and the like.
"Cycloheteroalkyl alkyl" denotes he group -R-cycloheteroalkyl where R is a alkyl or substituted alkyl as defined above and cycloheteroalkyl as as defined above. Cycloheteroalkyl groups can optionally be unsubstituted or substituted as defined above with e.g. halogen, amino, hydroxy, cyano, nitro, mercapto, alkoxy, alkylamino, acylamino, carbamoylamino, acyloxy, dialkylamino, alkylthio, carboxyl, amido, sulfo, sulfamido, and the like.

In a preferred embodiment the invention relates to 3-, 7-disubstituted pyrazolo[4,3-d]pyrimidines, which inhibit the cyclin-dependent and MAP kinases having formula **I** 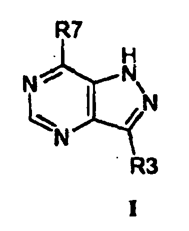 and the pharmaceutically acceptable acid salts thereof, wherein
**R3** is
selected from ethyl, isopropyl, n-butyl, isobutyl, tert-butyl, 3-pentyl, benzyl, cyclopropyl C₂₋₈ alkenyl, or C₃₋₈ alkinyl, wherein each of the groups may be optionally be subsituted by a halogen;
**R7** is
- **R7'-X,** wherein **X** is -NH-or -N(alkyl)- selected at each occurrence from the group methyl, ethyl, propyl, isopropyl, ethinyl, allyl, propargyl, and isopent-2-en-1-yl;
**R7'** is
- C₁-C₈ branched or unbranched alkyl, alkenyl or alkinyl preferentially selected from the group methyl, ethyl, isopropyl, butyl, isobutyl, allyl, propargyl, isopent-2-en-1-yl, which is substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
- cykloalkyl is C₃-C₁₅ cycloalkyl is preferentially selected from the group cyclopropyl, cyclobutyl, cyclopentyl, cyclohexyl, cycloheptyl or adamantyl;
- substituted cycloalkyl is C₃-C₁₅ cycloalkyl is preferentially selected from the group cyclopropyl, cyclobutyl, cyclopentyl, cyclohexyl, cycloheptyl or adamantyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
- cycloalkyl alkyl is R_{f}(cycloalkyl), wherein R_{f} is
   - C₁-C₆ alkyl, alkenyl or alkinyl preferentially selected from the group methyl, ethyl, isopropyl, butyl, allyl, propargyl, isopent-2-en-1-yl and 2-methylallyl, which is substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group,
   - cykloalkyl is C₃-C₁₅ cycloalkyl is preferentially selected from the group cyclopropyl, cyclobutyl, cyclopentyl, cyclohexyl, cycloheptyl or adamantyl;
   - substituted cycloalkyl is C₃-C₁₅ cycloalkyl is preferentially selected from the group cyclopropyl, cyclobutyl, cyclopentyl, cyclohexyl, cycloheptyl or adamantyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
   - aryl is preferentially selected from the group phenyl, biphenyl, naphthyl, tetrahydronaphtyl, fluorenyl, indenyl or fenanthrenyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
   - heterocycle is preferentially selected from the group thienyl, furyl, pyranyl, pyrrolyl, imidazolyl, pyrazolyl, pyridyl, pyrazinyl, pyrimidinyl, pyridazinyl, isothiazolyl, isoxazyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
- heteroalkyl is -R_{g}-Het, wherein
   - **R_{g}** is C₁-C₆ alkyl, alkenyl or alkinyl preferentially selected from the group methylen, 1,2-ethyliden, 1,3-propiliden, 1,4-butyliden, pentamethylen, hexamethylen, ethylendiyl, allyl-1,3-diyl, methylethan-1,1-diyl, methylethan-1,2-diyl, butan-1,3-diyl, which is substituted independently at each occurrence with 0 - 5 substituents selected from the group halogen, hydroxy, alkoxy, cyano and
   - Het is preferentially selected from the group thienyl, furyl, pyranyl, pyrrolyl, imidazolyl, pyrazolyl, pyridyl, pyrazinyl, pyrimidinyl, pyridazinyl, isothiazolyl, isoxazyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
- heteroaryl is -Rₕ-HetAr, wherein
   - **Rₕ is** C₁-C₆ alkyl, alkenyl or alkinyl preferentially selected from the group methylen, 1,2-ethyliden, 1,3-propiliden, 1,4-butyliden, pentamethylen, hexamethylen, ethylendiyl, allyl-1,3-diyl, methylethan-1,1-diyl, methylethan-1,2-diyl, butan-1,3-diyl, which is substituted independently at each occurrence with 0 - 5 substituents selected from the group halogen, hydroxy, alkoxy, cyano;
   - HetAr is preferentially selected from the group benzothienyl, naphthothienyl, benzofuranyl, chromenyl, indolyl, isoindolyl, indazolyl, qinolyl, isoqinolyl, ftalazinyl, qinaxalinyl, cinnolinyl, qinazolinyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
- arylalkyl is -RᵢAr, wherein
   - Rᵢ is C₁-C₆ alkyl, alkenyl or alkinyl preferentially selected from the group group methylen, 1,2-ethyliden, 1,3-propiliden, 1,4-butyliden, pentamethylen, hexamethylen, ethylendiyl, allyl-1,3-diyl methylethan-1,1-diyl, methylethan-1,2-diyl, butan-1,3-diyl, which is substituted independently at each occurrence with 0 - 5 substituents selected from the group halogen, hydroxy, alkoxy, cyano;
   - Ar is preferentially selected from the group phenyl, biphenyl, naphthyl, tetrahydronaphtyl, fluorenyl, indenyl or fenanthrenyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
- cycloheteroalkyl is preferentially selected from the group piperidinyl, piperazinyl, morfolinyl, pyrrolidinyl, imidazolidinyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
- cycloheteroalkyl alkyl, -Rⱼ(cycloheteroalkyl), wherein
   - Rⱼ is arylalkyl -RᵢAr, wherein
      - Rᵢ is C₁-C₆ alkyl, alkenyl or alkinyl preferentially selected from the group group methylen, 1,2-ethyliden, 1,3-propiliden, 1,4-butyliden, pentamethylen, hexamethylen, ethylendiyl, allyl-1,3-diyl, methylethan-1,1-diyl, methylethan-1,2-diyl, butan-1,3-diyl, which is substituted independently at each occurrence with 0 - 5 substituents selected from the group halogen, hydroxy, alkoxy, cyano, and
      - Ar is preferentially selected from the group phenyl, biphenyl, naphthyl, tetrahydronaphtyl, fluorenyl, indenyl or fenanthrenyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group, and
      - cycloheteroalkyl is preferentially selected from the group piperidinyl, piperazinyl, morfolinyl, pyrrolidinyl, imidazolidinyl substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group;
   - heteroarylalkyl is -Rₖ-HetAr, wherein
      - Rₖ is C₁-C₆ alkyl, alkenyl or alkinyl preferentially selected from the group group methylen, 1,2-ethyliden, 1,3-propiliden, 1,4-butyliden, pentamethylen, hexamethylen, ethylendiyl, allyl-1,3-diyl methylethan-1,1-diyl, methylethan-1,2-diyl, butan-1,3-diyl, which is substituted independently at each occurrence with 0 - 5 substituents selected from the group halogen, hydroxy, alkoxy, cyano, and
   HetAr is preferentially selected from the group benzothienyl, benzofuranyl, chromenyl, indolyl, isoindolyl, indazolyl, quinolinyl, phthalazinyl, quinoxalinyl, quinazolinyl, carbazolyl, acridinyl, indolinyl, and isoindolinyl, which is substituted independently at each occurrence with 0 - 5 substituents selected from the group halogen, hydroxy, alkoxy, amino, hydrazo, mercapto, carboxyl, cyano, nitro, amido, sulfo, sulfamido, acylamino, acyloxy, alkylamino, dialkylamino, alkylthio and carbamoyl group.

The following derivatives are particularly preferred, in particular with respect to their pharmaceutical use as described herein, namely: 7-(2-hydroxy-3-chlorobenzyl)amino-3-(methyl, ethyl, isopropyl, cyclopropyl, 3-pentyl, benzyl)pyrazolo[4,3-d]pyrimidine, 7-(2-hydroxy-4-chlorobenzyl)amino-3-(methyl,ethyl,isopropyl, cyclopropyl, 3-pentyl, benzyl)pyrazolo[4,3-d]pyrimidine, 7-(2-hydroxy-5-chlorobenzyl)amino-3-(methyl, ethyl, isopropyl, cyclopropyl, 3-pentyl, benzyl)pyrazolo[4,3-d]pyrimidine, 7-(2-hydroxy-6-chlorobenzyl)amino-3-(methyl, ethyl, isopropyl, 3-pentyl, benzyl)pyrazolo[4,3-d]pyrimidine, 7-(2-hydroxy-3-iodobenzyl)amino-3-(methyl, ethyl, isopropyl, 3-pentyl, benzyl)pyrazolo[4,3-d]pyrimidine, 7-(2-hydroxy-4-iodobenzyl)amino-3-(methyl, ethyl, isopropyl, 3-pentyl, benzyl)pyrazolo[4,3-d]pyrimidine, 7-(2-hydroxy-5-iodobenzyl)amino-3-(methyl, ethyl, isopropyl, 3-pentyl, benzyl)pyrazolo[4,3-d]pyrimidine, 7-(2-hydroxy-6-iodobenzyl)amino-3-(methyl, ethyl, isopropyl, 3-pentyl, benzyl)pyrazolo[4,3-d]pyrimidine, 7-(2-hydroxy-3-bromobenzyl)amino-3-(methyl, ethyl, isopropyl, 3-pentyl, benzyl)pyrazolo[4,3-d]pyrimidine, 3-pentyl, 7-(2-hydroxy-4-bromobenzyl)amino-3-(methyl, ethyl, isopropyl, 3-pentyl, benzyl)pyrazolo[4,3-d]pyrimidine, 7-(2-hydroxy-5-bromobenzyl)amino-3-(methyl, ethyl, isopropyl, 3-pentyl, benzyl)pyrazolo[4,3-d]pyrimidine, 7-(2-hydroxy-6-bromobenzyl)amino-3-(methyl, ethyl, isopropyl, 3-pentyl, benzyl)pyrazolo[4,3-d]pyrimidine, 7-(2-hydroxy-3-fluorobenzyl)amino-3-(methyl, ethyl, isopropyl, 3-pentyl, benzyl)pyrazolo[4,3-d]pyrimidine, 7-(2-hydroxy-4-fluorobenzyl)amino-3-(methyl, ethyl, isopropyl, 3-pentyl, benzyl)pyrazolo[4,3-d]pyrimidine, 7-(2-hydroxy-5-fluorobenzyl)amino-3-(methyl, ethyl, isopropyl, 3-pentyl, benzyl)pyrazolo[4,3-d]pyrimidine, 6-(2-hydroxy-6-fluorobenzyl)amino-3-(methyl, ethyl, isopropyl, 3-pentyl, benzyl)pyrazolo[4,3-d]pyrimidine, 7-(2,3-dihydroxy-4-methoxybenzyl)amino-3-(methyl, ethyl, isopropyl, 3-pentyl, benzyl)pyrazolo[4,3-d]pyrimidine, 7-(2,5-dihydroxy-4-methoxybenzyl)amino-3-(methyl, ethyl, isopropyl, 3-pentyl, benzyl)pyrazolo[4,3-d]pyrimidine, 7-(2,6-dihydroxy-3-methoxybenzyl)amino-3-(methyl, ethyl, isopropyl, 3-pentyl, benzyl)pyrazolo[4,3-d]pyrimidine, 7-(2,3-dihydroxy-3-methoxybenzyl)amino-3-(methyl, ethyl, isopropyl, 3-pentyl, benzyl)pyrazolo[4,3-d]pyrimidine, 7-(2,5-dihydroxy-3-methoxybenzyl)amino-3-(methyl, ethyl, isopropyl, 3-pentyl, benzyl)pyrazolo[4,3-d]pyrimidine, 7-(2,6-dihydroxy-4-methoxybenzyl)amino-3-(methyl, ethyl, isopropyl, 3-pentyl, benzyl)pyrazolo[4,3-d]pyrimidine, 7-(2,3-dihydroxy-4-chlorobenzyl)amino-3-(methyl, ethyl, isopropyl, 3-pentyl, benzyl)pyrazolo[4,3-d]pyrimidine, 7-(2,3-dihydroxy-4-chlorobenzyl)amino-3-(methyl, ethyl, isopropyl, 3-pentyl, benzyl)pyrazolo[4,3-d]pyrimidine, 7-(2,5-dihydroxy-4-chlorobenzyl)amino-3-(methyl, ethyl, isopropyl, 3-pentyl, benzyl)pyrazolo[4,3-d]pyrimidine, 7-(2,6-dihydroxy-4-chlorobenzyl)amino-3-(methyl, ethyl, isopropyl, 3-pentyl, benzyl)pyrazolo[4,3-d]pyrimidine, 7-(2,6-dihydroxy-4-bromoxybenzyl)amino-3-(methyl, ethyl, isopropyl, 3-pentyl, benzyl)pyrazolo[4,3-d]pyrimidine, 7-(2,6-dihydroxy-4-iodobenzyl)amino-3-(methyl, ethyl, isopropyl, 3-pentyl, benzyl)pyrazolo[4,3-d]pyrimidine, 7-(2,6-dihydroxy-3-chlorobenzyl)amino-3-(methyl, ethyl, isopropyl, 3-pentyl, benzyl)pyrazolo[4,3-d]pyrimidine, 7-(2,6-dihydroxy-3-bromobenzyl)amino-3-(methyl, ethyl, isopropyl, 3-pentyl, benzyl)pyrazolo[4,3-d]pyrimidine, 7-(2,6-dihydroxy-3-iodobenzyl)amino-3-(methyl, ethyl, isopropyl, 3-pentyl, benzyl)pyrazolo[4,3-d]pyrimidine, 7-(2,6-dihydroxy-3-fluorobenzyl)amino-3-(methyl, ethyl, isopropyl, 3-pentyl, benzyl)pyrazolo[4,3-d]pyrimidine, 7-(2,6-dihydroxy-3,5-dichlorobenzyl)amino-3-(methyl, ethyl, isopropyl, 3-pentyl, benzyl)pyrazolo[4,3-d]pyrimidine, 7-(2,6-dihydroxy-3,5-dibromobenzyl)amino-3-(methyl, ethyl, isopropyl, 3-pentyl, benzyl)pyrazolo[4,3-d]pyrimidine, 7-(2,6-dihydroxy-3,5-diiodobenzyl)amino-3-(methyl, ethyl, isopropyl, 3-pentyl, benzyl)pyrazolo[4,3-d]pyrimidine, 7-(2,6-dihydroxy-3,5-difluorobenzyl)amino-3-(methyl, ethyl, isopropyl, 3-pentyl, benzyl)pyrazolo[4,3-d]pyrimidine, 7-(2-fluorobenzyl)amino-3-(methyl, ethyl, isopropyl, 3-pentyl, benzyl)pyrazolo[4,3-d]pyrimidine, 7-(3-fluorobenzyl)amino-3-(methyl, ethyl, isopropyl, 3-pentyl, benzyl)pyrazolo[4,3-d]pyrimidine, 7-(4-fluorobenzyl)amino-3-(methyl, ethyl, isopropyl, 3-pentyl, benzyl)pyrazolo[4,3-d]pyrimidine, 7-(2-bromobenzyl)amino-3-(methyl, ethyl, isopropyl, 3-pentyl, benzyl)pyrazolo[4,3-d]pyrimidine, 7-(3-bromobenzyl)amino-3-(methyl, ethyl, isopropyl, 3-pentyl, benzyl)pyrazolo[4,3-d]pyrimidine, 7-(4-bromobenzyl)amino-3-(methyl, ethyl, isopropyl, 3-pentyl, benzyl)pyrazolo[4,3-d]pyrimidine, 7-(2-iodobenzyl)amino-3-(methyl, ethyl, isopropyl, 3-pentyl, benzyl)pyrazolo[4,3-d]pyrimidine, 7-(3-iodobenzyl)amino-3-(methyl, ethyl, isopropyl, 3-pentyl, benzyl)pyrazolo[4,3-d]pyrimidine, 7-(4-iodobenzyl)amino-3-(methyl, ethyl, isopropyl, 3-pentyl, benzyl)pyrazolo[4,3-d]pyrimidine, 7-(2-chlorobenzyl)amino-3-(methyl, ethyl, isopropyl, 3-pentyl, benzyl)pyrazolo[4,3-d]pyrimidine, 7-(2-chlorobenzyl)amino-3-(methyl, ethyl, isopropyl, 3-pentyl, benzyl)pyrazolo[4,3-d]pyrimidine, 7-(3-chlorobenzylamino)-3-(methyl, ethyl, isopropyl, 3-pentyl, benzyl)pyrazolo[4,3-d]pyrimidine 7-(4-chlorobenzyl)amino-3-(methyl, ethyl, isopropyl, 3-pentyl, benzyl)pyrazolo[4,3-d]pyrimidine, 7-(2-acetylbenzyl)amino-3-(methyl, ethyl, isopropyl, 3-pentyl, benzyl)pyrazolo[4,3-d]pyrimidine, 7-(3-acetylbenzyl)amino-3-(methyl, ethyl, isopropyl, 3-pentyl, benzyl)pyrazolo[4,3-d]pyrimidine, 7-(4-acetylbenzyl)amino-3-(methyl, ethyl, isopropyl, 3-pentyl, benzyl)pyrazolo[4,3-d]pyrimidine, 7-(3-karboxybenzyl)amino-3-(methyl, ethyl, isopropyl, 3-pentyl, benzyl)pyrazolo[4,3-d]pyrimidine, 7-(4-karboxybenzyl)amino-3-(methyl, ethyl, isopropyl, 3-pentyl, benzyl)pyrazolo[4,3-d]pyrimidine, 7-(2-acetoxybenzyl)amino-3-(methyl, ethyl, isopropyl, 3-pentyl, benzyl)pyrazolo[4,3-d]pyrimidine, 7-(3-acetoxybenzyl)amino-3-(methyl, ethyl, isopropyl, 3-pentyl, benzyl)pyrazolo[4,3-d]pyrimidine, 7-(4-acetoxybenzyl)amino-3-(methyl, ethyl, isopropyl, 3-pentyl, benzyl)pyrazolo[4,3-d]pyrimidine, 7-(2-nitrobenzyl)amino-3-(methyl, ethyl, isopropyl, 3-pentyl, benzyl)pyrazolo[4,3-d]pyrimidine, 7-(3-nitrobenzyl)amino-3-(methyl, ethyl, isopropyl, 3-pentyl, benzyl)pyrazolo[4,3-d]pyrimidine, 7-(4-nitrobenzyl)amino-3-(methyl, ethyl, isopropyl, 3-pentyl, benzyl)pyrazolo[4,3-d]pyrimidine, 7-(2-cyanobenzyl)amino-3-(methyl, ethyl, isopropyl, 3-pentyl, benzyl)pyrazolo[4,3-d]pyrimidine, 7-(3-cyanobenzyl)amino-3-(methyl, ethyl, isopropyl, 3-pentyl, benzyl)pyrazolo[4,3-d]pyrimidine, 7-(4-cyanobenzyl)amino-3-(methyl, ethyl, isopropyl, 3-pentyl, benzyl)pyrazolo[4,3-d]pyrimidine, 7-(5-nitro-2-methylbenzyl)amino-3-(methyl, ethyl, isopropyl, benzyl)pyrazolo[4,3-d]pyrimidine, 7-(2-methylbenzyl)amino-3-(methyl, ethyl, isopropyl, benzyl)pyrazolo[4,3-d]pyrimidine, 7-(3-methylbenzyl)amino-3-(methyl, ethyl, isopropyl, benzyl)pyrazolo[4,3-d]pyrimidine, 7-(4-methylbenzyl)amino-3-(methyl, ethyl, isopropyl, 3-pentyl, benzyl)pyrazolo[4,3-d]pyrimidine, 7-(4-methylaminobenzyl)amino-3-(methyl, ethyl, isopropyl, 3-pentyl, benzyl)pyrazolo[4,3-d]pyrimidine, 7-(2-methoxybenzyl)amino-3-(methyl, ethyl, isopropyl, 3-pentyl, benzyl)pyrazolo[4,3-d]pyrimidine, 7-(3-methoxybenzyl)amino-3-(methyl, ethyl, isopropyl, 3-pentyl, benzyl)pyrazolo[4,3-d]pyrimidine, 7-(4-methoxybenzyl)amino-3-(methyl, ethyl, isopropyl, 3-pentyl, benzyl)pyrazolo[4,3-d]pyrimidine, 7-(2-hydroxybenzyl)amino-3-(methyl, ethyl, isopropyl, 3-pentyl, benzyl)pyrazolo[4,3-d]pyrimidine, 7-(3-hydroxybenzyl)amino-3-(methyl, ethyl, isopropyl, 3-pentyl, benzyl)pyrazolo[4,3-d]pyrimidine, 7-(4-hydroxybenzyl)amino-3-(methyl, ethyl, isopropyl, 3-pentyl, benzyl)pyrazolo[4,3-d]pyrimidine, 7-(4-hexylbenzyl)amino-3-(methyl, ethyl, isopropyl, 3-pentyl, benzyl)pyrazolo[4,3-d]pyrimidine, 7-(4-hexyloxybenzyl)amino-3-(methyl, ethyl, isopropyl, 3-pentyl, benzyl)pyrazolo[4,3-d]pyrimidine, 7-(2-ethoxybenzyl)amino)-3-(methyl, ethyl, isopropyl, 3-pentyl, benzyl)pyrazolo[4,3-d]pyrimidine, 7-(3-ethoxybenzyl)amino-3-(methyl, ethyl, isopropyl, 3-pentyl, benzyl)pyrazolo[4,3-d]pyrimidine, 7-(4-ethoxybenzyl)amino-3-(methyl, ethyl, isopropyl, 3-pentyl, benzyl)pyrazolo[4,3-d]pyrimidine, 7-(4-ethylbenzyl)amino-3-(methyl, ethyl, isopropyl, 3-pentyl, benzyl)pyrazolo[4,3-d]pyrimidine, 7-(4-penthylbenzyl)amino-3-(methyl, ethyl, isopropyl, 3-pentyl, benzyl)pyrazolo[4,3-d]pyrimidine, 7-(4-penthyloxybenzyl)amino-3-(methyl, ethyl, isopropyl, 3-pentyl, benzyl)pyrazolo[4,3-d]pyrimidine, 7-(4-phenoxybenzyl)amino-3-(methyl, ethyl, isopropyl, 3-pentyl, benzyl)pyrazolo[4,3-d]pyrimidine, 7-(4-fenylbenzyl)amino-3-(methyl, ethyl, isopropyl, 3-pentyl, benzyl)pyrazolo[4,3-d]pyrimidine, 7-(4-propylbenzyl)amino-3-(methyl, ethyl, isopropyl, 3-pentyl, benzyl)pyrazolo[4,3-d]pyrimidine, 7-(4-oktylbenzylamino)-3-(methyl, ethyl, isopropyl, 3-pentyl, benzyl)pyrazolo[4,3-d]pyrimidine, 7-(4-octyloxybenzyl)amino-3-(methyl, ethyl, isopropyl, 3-pentyl, benzyl)pyrazolo[4,3-d]pyrimidine, 7-(4-ethyloxybenzyl)amino-3-(methyl, ethyl, isopropyl, 3-pentyl, benzyl)pyrazolo[4,3-d]pyrimidine, 7-(3,4-diacetoxybenzyl)amino-3-(methyl, ethyl, isopropyl, 3-pentyl, benzyl)pyrazolo[4,3-d]pyrimidine, 7-(3,5-diacetoxybenzyl)amino-3-(methyl, ethyl, isopropyl, 3-pentyl, benzyl)pyrazolo[4,3-d]pyrimidine, 67-(2,5-diaminobenzyl)amino-3-(methyl, ethyl, isopropyl, 3-pentyl, benzyl)pyrazolo[4,3-d]pyrimidine, 7-(3,5-dibromobenzyl)amino-3-(methyl, ethyl, isopropyl, 3-pentyl, benzyl)pyrazolo[4,3-d]pyrimidine, 7-(3,5-dibromo-4-methoxybenzyl)amino-3-(methyl, ethyl, isopropyl, 3-pentyl, benzyl)pyrazolo[4,3-d]pyrimidine, 7-(2,3-dichlorobenzyl)amino-3-(methyl, ethyl, isopropyl, 3-pentyl, benzyl)pyrazolo[4,3-d]pyrimidine, 7-(2,4-dichlorobenzyl)amino-3-(methyl, ethyl, isopropyl, 3-pentyl, benzyl)pyrazolo[4,3-d]pyrimidine, 7-(2,5-dichlorobenzyl)amino-3-(methyl, ethyl, isopropyl, 3-pentyl, benzyl)pyrazolo[4,3-d]pyrimidine, 7-(2,6-dichlorobenzyl)amino-3-(methyl, ethyl, isopropyl, 3-pentyl, benzyl)pyrazolo[4,3-d]pyrimidine, 7-(3,4-dichlorobenzyl)amino 3-(methyl, ethyl, isopropyl, 3-pentyl, benzyl)pyrazolo[4,3-d]pyrimidine, 7-(3,5-dichlorobenzyl)amino-3-(methyl, ethyl, isopropyl, 3-pentyl, benzyl)pyrazolo[4,3-d]pyrimidine, 7-(2,3,4,5-tetrafluorobenzyl)amino-3-(methyl, ethyl, isopropyl, 3-pentyl, benzyl)pyrazolo[4,3-d]pyrimidine, 7-(2-chloro-3,6-difluorobenzyl)amino-3-(methyl, ethyl, isopropyl, 3-pentyl, benzyl)pyrazolo[4,3-d]pyrimidine, 7-(5-chloro-2-fluorobenzyl)amino-3-(methyl, ethyl, isopropyl, 3-pentyl, benzyl)pyrazolo[4,3-d]pyrimidine, 7-(2,3,4-trifluorobenzyl)amino-3-(methyl, ethyl, isopropyl, 3-pentyl, benzyl)pyrazolo[4,3-d]pyrimidine, 7-(2,3,5-trifluorobenzyl)amino-3-(methyl, ethyl, isopropyl, 3-pentyl, benzyl)pyrazolo[4,3-d]pyrimidine, 7-(2,4,5-trifluorobenzyl)amino-3-(methyl, ethyl, isopropyl, 3-pentyl, benzyl)pyrazolo[4,3-d]pyrimidine, 7-(3,4,5-trifluorobenzyl)amino-3-(methyl, ethyl, isopropyl, 3-pentyl, benzyl)pyrazolo[4,3-d]pyrimidine, 7-(2,3,6-trifluorobenzyl)amino-3-(methyl, ethyl, isopropyl, 3-pentyl, benzyl)pyrazolo[4,3-d]pyrimidine, 7-(3-chloro-2,6-difluorobenzyl)amino-3-(methyl, ethyl, isopropyl, 3-pentyl, benzyl)pyrazolo[4,3-d]pyrimidine, 7-(2-chloro-6-fluorobenzyl)amino-3-(methyl, ethyl, isopropyl, 3-pentyl, benzyl)pyrazolo[4,3-d]pyrimidine, 7-(2,6-difluorobenzyl)amino-3-(methyl, ethyl, isopropyl, 3-pentyl, benzyl)pyrazolo[4,3-d]pyrimidine, 7-(2,4-difluorobenzyl)amino-3-(methyl, ethyl, isopropyl, 3-pentyl, benzyl)pyrazolo[4,3-d]pyrimidine, 7-(3,4-difluorobenzyl)amino-3-(methyl, ethyl, isopropyl, 3-pentyl, benzyl)pyrazolo[4,3-d]pyrimidine, 7-(2,5-difluorobenzyl)amino-3-(methyl, ethyl, isopropyl, 3-pentyl, benzyl)pyrazolo[4,3-d]pyrimidine, 7-(3,5-difluorobenzyl)amino-3-(methyl, ethyl, isopropyl, 3-pentyl, benzyl)pyrazolo[4,3-d]pyrimidine, 7-[5-fluoro-2-(trifluoromethyl)benzyl]amino-3-(methyl, ethyl, isopropyl, 3-pentyl, benzyl)pyrazolo[4,3-d]pyrimidine, 7-[4-fluoro-2-(trifluoromethyl)benzyl]amino-3-(methyl, ethyl, isopropyl, 3-pentyl, benzyl)pyrazolo[4,3-d]pyrimidine, 7-(2-chloro-5-(trifluoromethyl)benzyl)amino-3-(methyl, ethyl, isopropyl, 3-pentyl, benzyl)pyrazolo[4,3-d]pyrimidine, 7-(2-(difluoromethoxy)benzyl)amino-3-(methyl, ethyl, isopropyl, 3-pentyl, benzyl)pyrazolo[4,3-d]pyrimidine, 7-(3-(difluoromethoxy)benzyl)amino-3-(methyl, ethyl, isopropyl, 3-pentyl, benzyl)pyrazolo[4,3-d]pyrimidine, 7-(4-(difluoromethoxy)benzyl)amino-3-(methyl, ethyl, isopropyl, 3-pentyl, benzyl)pyrazolo[4,3-d]pyrimidine, 7-[2-fluoro-5-(trifluoromethyl)benzyl]amino-3-(methyl, ethyl, isopropyl, 3-pentyl, benzyl)pyrazolo[4,3-d]pyrimidine, 7-[3-fluoro-4-(trifluoromethyl)benzyl]amino-3-(methyl, ethyl, isopropyl, 3-pentyl, benzyl)pyrazolo[4,3-d]pyrimidine, 7-[2-fluoro-4-(trifluoromethyl)benzyl]amino-3-(methyl, ethyl, isopropyl, 3-pentyl, benzyl)pyrazolo[4,3-d]pyrimidine, 7-[2-fluoro-3-(trifluoromethyl)benzy]lamino-3-(methyl, ethyl, isopropyl, 3-pentyl, benzyl)pyrazolo[4,3-d]pyrimidine, 7-(2-chloro-6-fluoro-3-methylbenzylamino)-3-(methyl, ethyl, isopropyl, 3-pentyl, benzyl)pyrazolo[4,3-d]pyrimidine, 7-(6-chloro-2-fluoro-3-methylbenzylamino)-3-(methyl, ethyl, isopropyl, 3-pentyl, benzyl)pyrazolo[4,3-d]pyrimidine, 7-[3-chloro-2-fluoro-5-(trifluoromethyl)benzyl]amino-3-(methyl, ethyl, isopropyl, 3-pentyl, benzyl)pyrazolo[4,3-d]pyrimidine, 7-[3-chloro-2-fluoro-6-(trifluoromethyl)benzyl]amino-3-(methyl, ethyl, isopropyl, 3-pentyl, benzyl)pyrazolo[4,3-d]pyrimidine, 7-(2,3-difluoro-4-methylbenzyl)amino-3-(methyl, ethyl, isopropyl, 3-pentyl, benzyl)pyrazolo[4,3-d]pyrimidine, 7-(2,6-difluoro-3-methylbenzyl)amino-3-(methyl, ethyl, isopropyl, 3-pentyl, benzyl)pyrazolo[4,3-d]pyrimidine, 7-(2-fluoro-6-(trifluoromethyl)benzyl)amino-3-(methyl, ethyl, isopropyl, 3-pentyl, benzyl)pyrazolo[4,3-d]pyrimidine, 7-(3-chloro-2,6-difluorobenzyl)amino-3-(methyl, ethyl, isopropyl, 3-pentyl, benzyl)pyrazolo[4,3-d]pyrimidine, 7-[3-(trifluoromethylthio)benzy]lamino)-3-(methyl, ethyl, isopropyl, 3-pentyl, benzyl)pyrazolo[4,3-d]pyrimidine, 7-(3-fluoro-4-methylbenzyl)amino-3-(methyl, ethyl, isopropyl, 3-pentyl, benzyl)pyrazolo[4,3-d]pyrimidine, 7-[4-fluoro-3-methylbenzyl]amino-3-(methyl, ethyl, isopropyl, 3-pentyl, benzyl)pyrazolo[4,3-d]pyrimidine, 7-(5-fluoro-2-methylbenzylamino)-3-(methyl, ethyl, isopropyl, 3-pentyl, 3-pentyl, benzyl)pyrazolo[4,3-d]pyrimidine, 7-(2-chloro-3,6-difluorobenzyl)amino-3-(methyl, ethyl, isopropyl, 3-pentyl, benzyl)pyrazolo[4,3-d]pyrimidine, 7- [4-(trifluoromethylthio)benzyl] amino-3-(methyl, ethyl, isopropyl, 3-pentyl, benzyl)pyrazolo[4,3-d]pyrimidine, 7-(3-fluoro-5-(trifluoromethyl)benzyl)amino-3-(methyl, ethyl, isopropyl, 3-pentyl, benzyl)pyrazolo[4,3-d]pyrimidine, 7-(2-chloro-4-fluorobenzyl)amino-3-(methyl, ethyl, isopropyl, 3-pentyl, benzyl)pyrazolo[4,3-d]pyrimidine, 7-(2-(trifluoromethoxy)benzyl)amino-3-(methyl, ethyl, isopropyl, 3-pentyl, benzyl)pyrazolo[4,3-d]pyrimidine, 7-(3-(trifluoromethyl)benzyl)amino-3-(methyl, ethyl, isopropyl, 3-pentyl, benzyl)pyrazolo[4,3-d]pyrimidine, 7-[2-(trifluoromethyl)benzyl]amino-3-(methyl, ethyl, isopropyl, 3-pentyl, benzyl)pyrazolo[4,3-d]pyrimidine, 7-(4-(trifluoromethyl)benzyl)amino-3-(methyl, ethyl, isopropyl, 3-pentyl, benzyl)pyrazolo[4,3-d]pyrimidine, 7-[4-chloro-3-(trifluoromethyl)benzyl]amino)-3-(methyl, ethyl, isopropyl, 3-pentyl, benzyl)pyrazolo[4,3-d]pyrimidine, 7-(4-fluoro-3-(trifluoromethyl)benzyl)amino-3-(methyl, ethyl, isopropyl, 3-pentyl, benzyl)pyrazolo[4,3-d]pyrimidine, 7-[3,5-di(trifluoromethyl)benzyl]amino-3-(methyl, ethyl, isopropyl, 3-pentyl, benzyl)pyrazolo[4,3-d]pyrimidine, 7-[3-(trifluoromethoxy)benzyl]amino-3-(methyl, ethyl, isopropyl, 3-pentyl, benzyl)pyrazolo[4,3-d]pyrimidine, 7-[4-(trifluoromethoxy)benzyl]amino-3-(methyl, ethyl, isopropyl, 3-pentyl, benzyl)pyrazolo[4,3-d]pyrimidine, 7-(4-(trifluoromethyl)benzyl)amino-3-(methyl, ethyl, isopropyl, 3-pentyl, benzyl)pyrazolo[4,3-d]pyrimidine, 7-[4-(diethylamino)benzyl]amino-3-(methyl, ethyl, isopropyl, 3-pentyl, benzyl)pyrazolo[4,3-d]pyrimidine, 7-(3,4-dihydroxybenzyl]amino-3-(methyl, ethyl, isopropyl, 3-pentyl, benzyl)pyrazolo[4,3-d]pyrimidine, 7-(3,5-dihydroxybenzyl]amino-3-(methyl, ethyl, isopropyl, 3-pentyl, benzyl)pyrazolo[4,3-d]pyrimidine, 7-(3,4-dihydroxybenzyl]amino-3-(methyl, ethyl, isopropyl, 3-pentyl, benzyl)pyrazolo[4,3-d]pyrimidine, 7-(2,3-ethylenedioxybenzyl)amino-3-(methyl, ethyl, isopropyl, 3-pentyl, benzyl)pyrazolo[4,3-d]pyrimidine, 7-(2,4-dihydroxybenzyl)amino-3-(methyl, ethyl, isopropyl, 3-pentyl, benzyl)pyrazolo[4,3-d]pyrimidine, 7-(2,5-dihydroxybenzyl)amino-3-(methyl, ethyl, isopropyl, 3-pentyl, isopropyl, benzyl)pyrazolo[4,3-d]pyrimidine, 7-(2,6-dihydroxybenzyl)amino-3-(methyl, ethyl, isopropyl, 3-pentyl, benzyl)pyrazolo[4,3-d]pyrimidine, 7-(3,4-dimethoxybenzyl)amino-3-(methyl, ethyl, isopropyl, 3-pentyl, benzyl)pyrazolo[4,3-d]pyrimidine, 7-(3,4-dimethoxybenzyl)amino-3-(methyl, ethyl, isopropyl, 3-pentyl, benzyl)pyrazolo[4,3-d]pyrimidine, 7-(3,5-dimethoxybenzyl)amino-3-(methyl, ethyl, isopropyl, 3-pentyl, benzyl)pyrazolo[4,3-d]pyrimidine, 7-(2,3-dimethoxybenzyl)amino-3-(methyl, ethyl, isopropyl, 3-pentyl, benzyl)pyrazolo[4,3-d]pyrimidine, 7-(2,4-dimethoxybenzyl)amino-3-(methyl, ethyl, isopropyl, 3-pentyl, benzyl)pyrazolo[4,3-d]pyrimidine, 7-(2,5-dimethoxybenzyl)amino-3-(methyl, ethyl, isopropyl, 3-pentyl, benzyl)pyrazolo[4,3-d]pyrimidine, 7-(2,6-dimethoxybenzyl)amino-3-(methyl, ethyl, isopropyl, 3-pentyl, benzyl)pyrazolo[4,3-d]pyrimidine, 7-(3-hydroxy-4-methoxybenzyl)amino-3-(methyl, ethyl, isopropyl, 3-pentyl, benzyl)pyrazolo[4,3-d]pyrimidine, 7-(2-hydroxy-3-methoxybenzyl)amino-3-(methyl, ethyl, isopropyl, 3-pentyl, benzyl)pyrazolo[4,3-d]pyrimidine, 7-(4-hydroxy-3-methoxybenzyl)amino-3-(methyl, ethyl, isopropyl, 3-pentyl, benzyl)pyrazolo[4,3-d]pyrimidine, 7-(2-hydroxy-4-methoxybenzyl)amino-3-(methyl, ethyl, isopropyl, 3-pentyl, benzyl)pyrazolo[4,3-d]pyrimidine, 7-(4-hydroxy-2-methoxybenzyl)amino-3-(methyl, ethyl, isopropyl, 3-pentyl, benzyl)pyrazolo[4,3-d]pyrimidine, 7-(2-hydroxy-5-methoxybenzyl)amino-3-(methyl, ethyl, isopropyl, 3-pentyl, benzyl)pyrazolo[4,3-d]pyrimidine, 7-(3-hydroxy-4-methoxybenzyl)amino-3-(methyl, ethyl, isopropyl, 3-pentyl, benzyl)pyrazolo[4,3-d]pyrimidine, 7-(4-hydroxy-3-methoxybenzylamino)-3-(methyl, ethyl, isopropyl, 3-pentyl, benzyl)pyrazolo[4,3-d]pyrimidine, 7-(2-hydroxy-6-methoxybenzyl)amino-3-(methyl, ethyl, isopropyl, 3-pentyl, benzyl)pyrazolo[4,3-d]pyrimidine, 7-(3-hydroxy-5-methoxybenzyl)amino-3-(methyl, ethyl, isopropyl, 3-pentyl, benzyl)pyrazolo[4,3-d]pyrimidine, 7-(4,5-dimethoxy-2-nitrobenzyl)amino-3-(methyl, ethyl, isopropyl, 3-pentyl, benzyl)pyrazolo[4,3-d]pyrimidine, 7-(3,4-dimethylbenzyl)amino-3-(methyl, ethyl, isopropyl, 3-pentyl, benzyl)pyrazolo[4,3-d]pyrimidine, 7-(2,3-dimethylbenzyl)amino-3-(methyl, ethyl, isopropyl, 3-pentyl, benzyl)pyrazolo[4,3-d]pyrimidine, 7-(2,4-dimethylbenzyl)amino-3-(methyl, ethyl, isopropyl, 3-pentyl, benzyl)pyrazolo[4,3-d]pyrimidine, 7-(2,6-dimethylbenzyl)amino-3-(methyl, ethyl, isopropyl, 3-pentyl, benzyl)pyrazolo[4,3-d]pyrimidine, 7-(2,6-dimethyl-4-hydroxybenzyl)amino-3-(methyl, ethyl, isopropyl, 3-pentyl, benzyl)pyrazolo[4,3-d]pyrimidine, 7-(3,5-dimethyl-4-hydroxybenzyl)amino-3-(methyl, ethyl, isopropyl, 3-pentyl, benzyl)pyrazolo[4,3-d]pyrimidine, 7-(2-fluoro-4-hydroxybenzyl)amino-3-(methyl, ethyl, isopropyl, 3-pentyl, benzyl)pyrazolo[4,3-d]pyrimidine, 7-(3-fluoro-4-methylbenzyl)amino-3-(methyl, ethyl, isopropyl, 3-pentyl, benzyl)pyrazolo[4,3-d]pyrimidine, 7-(3,4-dinitrobenzyl)amino-3-(methyl, ethyl, isopropyl, 3-pentyl, benzyl)pyrazolo[4,3-d]pyrimidine, 7-(3,5-dinitrobenzyl)amino-3-(methyl, ethyl, isopropyl, 3-pentyl, benzyl)pyrazolo[4,3-d]pyrimidine, 7-(2-methyl-5-nitrobenzyl)amino-3-(methyl, ethyl, isopropyl, 3-pentyl, benzyl)pyrazolo[4,3-d]pyrimidine, 7-(3-methyl-4-nitrobenzyl)amino-3-(methyl, ethyl, isopropyl, 3-pentyl, benzyl)pyrazolo[4,3-d]pyrimidine, 7-(3,4-diiodo-4-hydroxybenzyl)amino-3-(methyl, ethyl, isopropyl, 3-pentyl, benzyl)pyrazolo[4,3-d]pyrimidine, 7-(2-chloro-3,4-dimethoxybenzyl)amino-3-(methyl, ethyl, isopropyl, 3-pentyl, benzyl)pyrazolo[4,3-d]pyrimidine, 7-(4-chloro-3,5-dinitrobenzyl)amino-3-(methyl, ethyl, isopropyl, 3-pentyl, benzyl)pyrazolo[4,3-d]pyrimidine, 7-(2-chloro-4-fluorobenzyl)amino-3-(methyl, ethyl, isopropyl, 3-pentyl, benzyl)pyrazolo[4,3-d]pyrimidine, 7-(3-chloro-4-fluorobenzyl)amino-3-(methyl, ethyl, isopropyl, 3-pentyl, benzyl)pyrazolo[4,3-d]pyrimidine, 7-(2-chloro-6-methylbenzyl)amino-3-(methyl, ethyl, isopropyl, benzyl)pyrazolo[4,3-d]pyrimidine, 7-(3-chloro-2-methylbenzyl)amino-3-(methyl, ethyl, isopropyl, 3-pentyl, benzyl)pyrazolo[4,3-d]pyrimidine, 7-(3-chloro-4-methylbenzyl)amino-3-(methyl, ethyl, isopropyl, 3-pentyl, benzyl)pyrazolo[4,3-d]pyrimidine, 7-(5-chloro-2-methoxybenzyl)amino-3-(methyl, ethyl, isopropyl, 3-pentyl, benzyl)pyrazolo[4,3-d]pyrimidine, 7-(2-chloro-4-fluorobenzyl)amino-3-(methyl, ethyl, isopropyl, 3-pentyl, benzyl)pyrazolo[4,3-d]pyrimidine, 7-(4-chloromethylbenzyl)amino-3-(methyl, ethyl, isopropyl, 3-pentyl, benzyl)pyrazolo[4,3-d]pyrimidine, 7-(2-chloro-5-nitrobenzyl)amino-3-(methyl, ethyl, isopropyl, 3-pentyl, benzyl)pyrazolo[4,3-d]pyrimidine, 7-(2-chloro-6-nitrobenzyl)amino-3-(methyl, ethyl, isopropyl, 3-pentyl, benzyl)pyrazolo[4,3-d]pyrimidine, 7-(4-chloro-3-nitrobenzyl)amino-3-(methyl, ethyl, isopropyl, 3-pentyl, benzyl)pyrazolo[4,3-d]pyrimidine, 7-(5-chloro-2-nitrobenzyl)amino-3-(methyl, ethyl, isopropyl, 3-pentyl, benzyl)pyrazolo[4,3-d]pyrimidine, 7-(3-bromo-4-hydroxybenzyl)amino-3-(methyl, ethyl, isopropyl, 3-pentyl, benzyl)pyrazolo[4,3-d]pyrimidine, 7-(3,5-dibromo-4-hydroxybenzyl)amino-3-(methyl, ethyl, isopropyl, 3-pentyl, benzyl)pyrazolo[4,3-d]pyrimidine, 7-(3-bromo-4-methoxybenzyl)amino-3-(methyl, ethyl, isopropyl, 3-pentyl, benzyl)pyrazolo[4,3-d]pyrimidine, 7-(4-butoxybenzyl)amino-3-(methyl, ethyl, isopropyl, 3-pentyl, benzyl)pyrazolo[4,3-d]pyrimidine, 7-(4-butoxybenzyl)amino-3-(methyl, ethyl, isopropyl, 3-pentyl, benzyl)pyrazolo[4,3-d]pyrimidine, 7-(4-/t-butyl/benzyl)amino-3-(methyl, ethyl, isopropyl, 3-pentyl, benzyl)pyrazolo[4,3-d]pyrimidine, 7-(4-t-butyl-2,6-dimethylbenzyl)amino-3-(methyl, ethyl, isopropyl, 3-pentyl, benzyl)pyrazolo[4,3-d]pyrimidine, 7-(2-aminobenzyl)amino-3-(methyl, ethyl, isopropyl, 3-pentyl, benzyl)pyrazolo[4,3-d]pyrimidine, 7-(3-aminobenzyl)amino-3-(methyl, ethyl, isopropyl, 3-pentyl, benzyl)pyrazolo[4,3-d]pyrimidine, 7-(4-aminobenzyl)amino-3-(methyl, ethyl, isopropyl, 3-pentyl, benzyl)pyrazolo[4,3-d]pyrimidine, 7-(2-amino-6-chlorobenzyl)amino-3-(methyl, ethyl, isopropyl, 3-pentyl, benzyl)pyrazolo[4,3-d]pyrimidine, 7-(3-amino-4-chlorobenzyl)amino-3-(methyl, ethyl, isopropyl, 3-pentyl, benzyl)pyrazolo[4,3-d]pyrimidine, 7-(2-amino-3-chlorobenzyl)amino-3-(methyl, ethyl, isopropyl, 3-pentyl, benzyl)pyrazolo[4,3-d]pyrimidine, 7-(2-amino-4-chlorobenzyl)amino-3-(methyl, ethyl, isopropyl, 3-pentyl, benzyl)pyrazolo[4,3-d]pyrimidine, 7-(2-amino-6-chlorobenzyl)amino-3-(methyl, ethyl, isopropyl, 3-pentyl, benzyl)pyrazolo[4,3-d]pyrimidine, 7-(2,6-diamino-3-chlorobenzyl)amino-3-(methyl, ethyl, isopropyl, 3-pentyl, benzyl)pyrazolo[4,3-d]pyrimidine, 7-(2,6-diamino-4-chlorobenzyl)amino-3-(methyl, ethyl, isopropyl, 3-pentyl, benzyl)pyrazolo[4,3-d]pyrimidine, 7-(4-amino-3-chlorobenzyl)amino-3-(methyl, ethyl, isopropyl, benzyl)pyrazolo[4,3-d]pyrimidine, 7-(4-amino-5-dichlorobenzyl)amino-3-(methyl, ethyl, isopropyl, 3-pentyl, benzyl)pyrazolo[4,3-d]pyrimidine, 7-(5-amino-2-methylbenzyl)amino-3-(methyl, ethyl, isopropyl, 3-pentyl, benzyl)pyrazolo[4,3-d]pyrimidine, 7-(2-amino-3-nitrobenzyl)amino-3-(methyl, ethyl, isopropyl, 3-pentyl, benzyl)pyrazolo[4,3-d]pyrimidine, 7-(4-amino-3-nitrobenzyl)amino-3-(methyl, ethyl, isopropyl, 3-pentyl, benzyl)pyrazolo[4,3-d]pyrimidine, 7-(4-benzyloxybenzyl)amino-3-(methyl, ethyl, isopropyl, 3-pentyl, benzyl)pyrazolo[4,3-d]pyrimidine, 7-(3-acetylbenzyl)amino-3-(methyl, ethyl, isopropyl, 3-pentyl, benzyl)pyrazolo[4,3-d]pyrimidine, 7-(2-acetylbenzyl)amino-3-(methyl, ethyl, isopropyl, 3-pentyl, benzyl)pyrazolo[4,3-d]pyrimidine, 7-(2,3,4-trimethoxybenzyl)amino-3-(methyl, ethyl, isopropyl, 3-pentyl, benzyl)pyrazolo[4,3-d]pyrimidine, 7-(2,4,5-trimethoxybenzyl)amino-3-(methyl, ethyl, isopropyl, 3-pentyl, benzyl)pyrazolo[4,3-d]pyrimidine, 7-(2,4,6-trimethoxybenzyl)amino-3-(methyl, ethyl, isopropyl, 3-pentyl, benzyl)pyrazolo[4,3-d]pyrimidine, 7-(3,4,5-trimethoxybenzyl)amino-3-(methyl, ethyl, isopropyl, 3-pentyl, benzyl)pyrazolo[4,3-d]pyrimidine, 7-(2,4,6-trimethoxybenzyl)amino-3-(methyl, ethyl, isopropyl, 3-pentyl, benzyl)pyrazolo[4,3-d]pyrimidine, 7-(2,3,4-trihydroxybenzy)amino-3-(methyl, ethyl, isopropyl, 3-pentyl, benzyl)pyrazolo[4,3-d]pyrimidine, 7-(2,4,6-trihydroxybenzy)lamino-3-(methyl, ethyl, isopropyl, 3-pentyl, benzyl)pyrazolo[4,3-d]pyrimidine, 7-(2,3,4-trihydroxybenzyl)amino-3-(methyl, ethyl, isopropyl, 3-pentyl, benzyl)pyrazolo[4,3-d]pyrimidine, 7-(3,4,5-trihydroxybenzyl)amino-3-(methyl, ethyl, isopropyl, 3-pentyl, benzyl)pyrazolo[4,3-d]pyrimidine, 7-(2,4,6-trihydroxybenzyl)amino-3-(methyl, ethyl, isopropyl, 3-pentyl, benzyl)pyrazolo[4,3-d]pyrimidine, 7-(2,4,5-trichlorobenzyl)amino-3-(methyl, ethyl, isopropyl, 3-pentyl, benzyl)pyrazolo[4,3-d]pyrimidine, 7-(2,4,5-trichlorobenzyl)amino-3-(methyl, ethyl, isopropyl, 3-pentyl, benzyl)pyrazolo[4,3-d]pyrimidine, 7-(2,4,6-trichlorobenzyl)amino-3-(methyl, ethyl, isopropyl, 3-pentyl, benzyl)pyrazolo[4,3-d]pyrimidine, 7-(2,3,4-trichlorobenzyl)amino-3-(methyl, ethyl, isopropyl, 3-pentyl, benzyl)pyrazolo[4,3-d]pyrimidine, 7-(2,3,5-trichlorobenzyl)amino-3-(methyl, ethyl, isopropyl, 3-pentyl, benzyl)pyrazolo[4,3-d]pyrimidine, 7-(2,3,6-trichlorobenzyl)amino-3-(methyl, ethyl, isopropyl, 3-pentyl, benzyl)pyrazolo[4,3-d]pyrimidine, 7-(2,5,6-trichlorobenzyl)amino-3-(methyl, ethyl, isopropyl, 3-pentyl, benzyl)pyrazolo[4,3-d]pyrimidine, 7-anilino-3-(methyl, ethyl, isopropyl, 3-pentyl, benzyl)pyrazolo[4,3-d]pyrimidine, 7-[2,4-bis(trifluoromethyl)anilino]-3-(methyl, ethyl, isopropyl, 3-pentyl, benzyl)pyrazolo[4,3-d]pyrimidine, 7-[2,5-bis(trifluoromethyl)anilino]-3-(methyl, ethyl, isopropyl, 3-pentyl, benzyl)pyrazolo[4,3-d]pyrimidine, 7-[2,4-bis(trifluoromethyl)anilino]-3-(methyl, ethyl, isopropyl, benzyl)pyrazolo[4,3-d]pyrimidine, 7-(3,5-bis(trifluoromethyl)anilino)-3-(methyl, ethyl, isopropyl, 3-pentyl, benzyl)pyrazolo[4,3-d]pyrimidine, 7-(2-bromoanilino)-3-(methyl, ethyl, isopropyl, 3-pentyl, benzyl)pyrazolo[4,3-d]pyrimidine, 7-(3-bromoanilino)-3-(methyl, ethyl, isopropyl, benzyl)pyrazolo[4,3-d]pyrimidine, 7-(4-bromoanilino)-3-(methyl, ethyl, isopropyl, 3-pentyl, benzyl)pyrazolo[4,3-d]pyrimidine, 7-(4-bromo-2-chloroanilino)-3-(methyl, ethyl, isopropyl, 3-pentyl, benzyl)pyrazolo[4,3-d]pyrimidine, 7-(4-bromo-3-chloroanilino)-3-(methyl, ethyl, isopropyl, 3-pentyl, benzyl)pyrazolo[4,3-d]pyrimidine, 7-[2-bromo-6-chloro-4-(trifluoromethyl)anilino]-3-(methyl, ethyl, isopropyl, 3-pentyl, benzyl)pyrazolo[4,3-d]pyrimidine, 7-(4-bromo-5,6-difluoroanilino)-3-(methyl, ethyl, isopropyl, 3-pentyl, benzyl)pyrazolo[4,3-d]pyrimidine, 7-(2-bromo-4,6-difluoroanilino)-3-(methyl, ethyl, isopropyl, 3-pentyl, benzyl)pyrazolo[4,3-d]pyrimidine, 7-(4-bromo-2,6-difluoroanilino)-3-(methyl, ethyl, isopropyl, 3-pentyl, benzyl)pyrazolo[4,3-d]pyrimidine, 7-(4-bromo-2-fluoroanilino)-3-(methyl, ethyl, isopropyl, 3-pentyl, benzyl)pyrazolo[4,3-d]pyrimidine, 7-(2-bromo-4-fluoroanilino)-3-(methyl, ethyl, isopropyl, 3-pentyl, benzyl)pyrazolo[4,3-d]pyrimidine, 7-(2-bromo-4-methylanilino)-3-(methyl, ethyl, isopropyl, 3-pentyl, benzyl)pyrazolo[4,3-d]pyrimidine, 7-(3-bromo-2-methylanilino)-3-(methyl, ethyl, isopropyl, 3-pentyl, benzyl)pyrazolo[4,3-d]pyrimidine, 6-(4-bromo-3-methylanilino))-3-(methyl, ethyl, isopropyl, 3-pentyl, benzyl)pyrazolo[4,3-d]pyrimidine, 7-[2-bromo-4-(trifluoromethoxy)anilino]-3-(methyl, ethyl, isopropyl, 3-pentyl, benzyl)pyrazolo[4,3-d]pyrimidine, 7-[3-bromo-4-(trifluoromethoxy)anilino]-3-(methyl, ethyl, isopropyl, 3-pentyl, benzyl)pyrazolo[4,3-d]pyrimidine, 7-[4-bromo-2-(trifluoromethoxy)anilino]-3-(methyl, ethyl, isopropyl, 3-pentyl, benzyl)pyrazolo[4,3-d]pyrimidine, 7-(2-bromo-4,5,6-trifluoroanilino)-3-(methyl, ethyl, isopropyl, 3-pentyl, benzyl)pyrazolo[4,3-d]pyrimidine, 7-(2,4-dibromoanilino)-3-(methyl, ethyl, isopropyl, 3-pentyl, benzyl)pyrazolo[4,3-d]pyrimidine, 7-(2,5-dibromoanilino)-3-(methyl, ethyl, isopropyl, 3-pentyl, benzyl)pyrazolo[4,3-d]pyrimidine, 7-(2,4-dibromo-3,6-dichloroanilino)-3-(methyl, ethyl, isopropyl, 3-pentyl, benzyl)pyrazolo[4,3-d]pyrimidine, 7-(2,6-dibromo-4-fluoroanilino)-3-(methyl, ethyl, isopropyl, 3-pentyl, benzyl)pyrazolo[4,3-d]pyrimidine, 7-[2,6-dibromo-4-(trifluoromethoxy)anilino]-3-(methyl, ethyl, isopropyl, 3-pentyl, benzyl)pyrazolo[4,3-d]pyrimidine, 7-[2,4-dibromo-6-(trifluoromethyl)anilino]-3-(methyl, ethyl, isopropyl, 3-pentyl, benzyl)pyrazolo[4,3-d]pyrimidine, 7-[2,6-dibromo-4-(trifluoromethyl)anilino]-3-(methyl, ethyl, isopropyl, 3-pentyl, benzyl)pyrazolo[4,3-d]pyrimidine, 7-(2,3-dichloroanilino)-3-(methyl, ethyl, isopropyl, 3-pentyl, benzyl)pyrazolo[4,3-d]pyrimidine, 7-(2,4-dichloroanilino)-3-(methyl, ethyl, isopropyl, 3-pentyl, benzyl)pyrazolo[4,3-d]pyrimidine, 7-(2,5-dichloroanilino)-3-(methyl, ethyl, isopropyl, 3-pentyl, benzyl)pyrazolo[4,3-d]pyrimidine, 7-(2,6-dichlorooanilino)-3-(methyl, ethyl, isopropyl, 3-pentyl, benzyl)pyrazolo[4,3-d]pyrimidine, 7-(3,4-dichloroanilino)-3-(methyl, ethyl, isopropyl, 3-pentyl, benzyl)pyrazolo[4,3-d]pyrimidine, 7-(3,5-dichloroanilino)-3-(methyl, ethyl, isopropyl, 3-pentyl, benzyl)pyrazolo[4,3-d]pyrimidine, 7-[2,6-dichloro-4-(trifluoromethoxy)anilino]-3-(methyl, ethyl, isopropyl, 3-pentyl, benzyl)pyrazolo[4,3-d]pyrimidine, 7-[2,4-dichloro-6-(trifluoromethyl)anilino]-3-(methyl, ethyl, isopropyl, 3-pentyl, benzyl)pyrazolo[4,3-d]pyrimidine, 7-[2,6-dichloro-4-(trifluoromethyl)anilino]-3-(methyl, ethyl, isopropyl, 3-pentyl, benzyl)pyrazolo[4,3-d]pyrimidine, 7-(2,3-difluoroanilino)-3-(methyl, ethyl, isopropyl, 3-pentyl, benzyl)pyrazolo[4,3-d]pyrimidine, 7-(2,4-difluoroanilino)-3-(methyl, ethyl, isopropyl, 3-pentyl, benzyl)pyrazolo[4,3-d]pyrimidine, 7-(2,5-difluoroanilino)-3-(methyl, ethyl, isopropyl, 3-pentyl, benzyl)pyrazolo[4,3-d]pyrimidine, 7-(2,6-difluoroanilino)-3-(methyl, ethyl, isopropyl, 3-pentyl, benzyl)pyrazolo[4,3-d]pyrimidine, 7-(3,4-difluoroanilino)-3-(methyl, ethyl, isopropyl, 3-pentyl, benzyl)pyrazolo[4,3-d]pyrimidine, 7-(3,5-difluoroanilino)-3-(methyl, ethyl, isopropyl, 3-pentyl, benzyl)pyrazolo[4,3-d]pyrimidine, 7-(2-difluoromethoxyanilino)-3-(methyl, ethyl, isopropyl, 3-pentyl, benzyl)pyrazolo[4,3-d]pyrimidine, 7-(2-difluoromethoxy-5-nitroanilino)-3-(methyl, ethyl, isopropyl, 3-pentyl, benzyl)pyrazolo[4,3-d]pyrimidine, 7-(2,3-difluoro-6-nitroanilino)-3-(methyl, ethyl, isopropyl, 3-pentyl, benzyl)pyrazolo[4,3-d]pyrimidine, 7-(2,4-difluoro-6-nitroanilino)-3-(methyl, ethyl, isopropyl, 3-pentyl, benzyl)pyrazolo[4,3-d]pyrimidine, 7-(2,4-diiodoanilino)-3-(methyl, ethyl, isopropyl, 3-pentyl, benzyl)pyrazolo[4,3-d]pyrimidine, 7-(2,3-dimethylanilino)-3-(methyl, ethyl, isopropyl, 3-pentyl, benzyl)pyrazolo[4,3-d]pyrimidine, 7-(2,4-dimethylanilino)-3-(methyl, ethyl, isopropyl, 3-pentyl, benzyl)pyrazolo[4,3-d]pyrimidine, 7-(2,3-dimethyl-6-nitroanilino)-3-(methyl, ethyl, isopropyl, 3-pentyl, benzyl)pyrazolo[4,3-d]pyrimidine, 7-(2,4-dimethoxyanilino)-3-(methyl, ethyl, isopropyl, 3-pentyl, benzyl)pyrazolo[4,3-d]pyrimidine, 7-(2,3-dimethoxyanilino)-3-(methyl, ethyl, isopropyl, 3-pentyl, benzyl)pyrazolo[4,3-d]pyrimidine, 7-(2,3-dinitro-6-methylanilino)-3-(methyl, ethyl, isopropyl, 3-pentyl, benzyl)pyrazolo[4,3-d]pyrimidine, 7-(4-hydroxy-2-methylanilino)-3-(methyl, ethyl, isopropyl, 3-pentyl, benzyl)pyrazolo[4,3-d]pyrimidine, 7-(2-chloroanilino)-3-(methyl, ethyl, isopropyl, 3-pentyl, benzyl)pyrazolo[4,3-d]pyrimidine, 7-(3-chloroanilino)-3-(methyl, ethyl, isopropyl, 3-pentyl, benzyl)pyrazolo[4,3-d]pyrimidine, 7-(4-chloroanilino)-3-(methyl, ethyl, isopropyl, 3-pentyl, benzyl)pyrazolo[4,3-d]pyrimidine, (7-chloro-2,6-dibromo-4-fluoroanilino)-3-(methyl, ethyl, isopropyl, 3-pentyl, benzyl)pyrazolo[4,3-d]pyrimidine, 7-(2-chloro-4-fluoroanilino)-3-(methyl, ethyl, isopropyl, 3-pentyl, benzyl)pyrazolo[4,3-d]pyrimidine, 7-(2-chloro-5-fluoroanilino)-3-(methyl, ethyl, isopropyl, 3-pentyl, benzyl)pyrazolo[4,3-d]pyrimidine, 7-(2-chloro-6-fluoroanilino)-3-(methyl, ethyl, isopropyl, 3-pentyl, benzyl)pyrazolo[4,3-d]pyrimidine, 7-(3-chloro-2-fluoroanilino)-3-(methyl, ethyl, isopropyl, 3-pentyl, benzyl)pyrazolo[4,3-d]pyrimidine, 7-(3-chloro-4-fluoroanilino)-3-(methyl, ethyl, isopropyl, 3-pentyl, benzyl)pyrazolo[4,3-d]pyrimidine, 7-(4-chloro-2-fluoroanilino)-3-(methyl, ethyl, isopropyl, 3-pentyl, benzyl)pyrazolo[4,3-d]pyrimidine, 7-(5-chloro-2-fluoroanilino)-3-(methyl, ethyl, isopropyl, 3-pentyl, benzyl)pyrazolo[4,3-d]pyrimidine, 7-(2-chloro-4-fluoro-5-methylanilino)-3-(methyl, ethyl, isopropyl, 3-pentyl, benzyl)pyrazolo[4,3-d]pyrimidine, 7-(5-chloro-4-fluoro-2-nitroanilino)-3-(methyl, ethyl, isopropyl, 3-pentyl, benzyl)pyrazolo[4,3-d]pyrimidine, 7-(5-chloro-2-hydroxyanilino)-3-(methyl, ethyl, isopropyl, 3-pentyl, benzyl)pyrazolo[4,3-d]pyrimidine, 7-(4-chloro-2-iodoanilino)-3-(methyl, ethyl, isopropyl, 3-pentyl,benzyl)pyrazolo[4,3-d]pyrimidine, 7-(2-chloro-4-iodoanilino)-3-(methyl, ethyl, isopropyl, 3-pentyl, benzyl)pyrazolo[4,3-d]pyrimidine, 7-(2-chloro-6-methylanilino)-3-(methyl, ethyl, isopropyl, 3-pentyl, benzyl)pyrazolo[4,3-d]pyrimidine, 7-(3-chloro-2-methylanilino)-3-(methyl, ethyl, isopropyl, 3-pentyl, benzyl)pyrazolo[4,3-d]pyrimidine, 7-[3-chloro-4-(trifluoromethoxy)anilino]-3-(methyl, ethyl, isopropyl, 3-pentyl, cyclopropyl, benzyl)pyrazolo[4,3-d]pyrimidine, 7-[4-chloro-2-(trifluoromethoxy)anilino]-3-(methyl, ethyl, isopropyl, 3-pentyl, cyclopropyl, benzyl)pyrazolo[4,3-d]pyrimidine, 7-(2-fluoroanilino)-3-(methyl, ethyl, isopropyl, 3-pentyl, cyclopropyl, benzyl)pyrazolo[4,3-d]pyrimidine, 7-(3-fluoroanilino)-3-(methyl, ethyl, isopropyl, 3-pentyl, cyclopropyl, benzyl)pyrazolo[4,3-d]pyrimidine, 7-(4-fluoroanilino)-3-(methyl, ethyl, isopropyl, 3-pentyl, cycopropyl, benzyl)pyrazolo[4,3-d]pyrimidine, 7-(2-fluoro-4-iodoanilino)-3-(methyl, ethyl, isopropyl, 3-pentyl, benzyl)pyrazolo[4,3-d]pyrimidine, 7-(2-fluoro-5-nitroanilino)-3-(methyl, ethyl, isopropyl, 3-pentyl, cyclopropyl, benzyl)pyrazolo[4,3-d]pyrimidine, 7-(2-fluoro-4-methylanilino)-3-(methyl, ethyl, isopropyl, 3-pentyl, cyclopropyl, benzyl)pyrazolo[4,3-d]pyrimidine, 7-(3-fluoro-2-methylanilino)-3-(methyl, ethyl, isopropyl, 3-cyclopropyl, 3-pentyl, cyclopropyl, benzyl)pyrazolo[4,3-d]pyrimidine, 7-(3-fluoro-4-methylanilino)-3-(methyl, ethyl, isopropyl, 3-pentyl, cyclopropyl, benzyl)pyrazolo[4,3-d]pyrimidine, 7-(4-fluoro-2-methylanilino)-3-(methyl, ethyl, isopropyl, 3-pentyl, cyclopropyl, benzyl)pyrazolo[4,3-d]pyrimidine, 7-(3-fluoro-4-methylanilino)-3-(methyl, ethyl, isopropyl, 3-pentyl, cyclopropyl, benzyl)pyrazolo[4,3-d]pyrimidine, 7-(5-fluoro-2-methylanilino)-3-(methyl, ethyl, isopropyl, 3-pentyl, cyclopropyl, benzyl)pyrazolo[4,3-d]pyrimidine, 7-(4-fluoro-2-nitroanilino)-3-(methyl, ethyl, isopropyl, 3-pentyl, cyclopropyl, benzyl)pyrazolo[4,3-d]pyrimidine, 7-(4-fluoro-3-nitroanilino)-3-(methyl, ethyl, isopropyl, 3-pentyl, cyclopropyl, benzyl)pyrazolo[4,3-d]pyrimidine, 7-(2-jodoanilino)-3-(methyl, ethyl, isopropyl, 3-pentyl, cyclopropyl, benzyl)pyrazolo[4,3-d]pyrimidine, 7-[2-fluoro-4-(trifluoromethyl)anilino]-3-(methyl, ethyl, isopropyl, 3-pentyl, cyclopropyl, benzyl)pyrazolo[4,3-d]pyrimidine, 7-(4-iodo-2-methylanilino)-3-(methyl, ethyl, isopropyl, 3-pentyl, cyclopropyl, benzyl)pyrazolo[4,3-d]pyrimidine, 7-(2-methoxyanilino)-3-(methyl, ethyl, isopropyl, 3-pentyl, cyclopropyl, benzyl)pyrazolo[4,3-d]pyrimidine, 7-(3-methoxyanilino)-3-(methyl, ethyl, isopropyl, 3-pentyl, cyclopropyl, benzyl)pyrazolo[4,3-d]pyrimidine, 7-(4-methoxyanilino)-3-(methyl, ethyl, isopropyl, 3-pentyl, cyclopropyl, benzyl)pyrazolo[4,3-d]pyrimidine, 7-(2-methoxy-5-methylanilino)-3-(methyl, ethyl, isopropyl, 3-pentyl, cyclopropyl, benzyl)pyrazolo[4,3-d]pyrimidine, 7-(2-methoxy-6-methylanilino)-3-(methyl, ethyl, isopropyl, 3-pentyl, cyclopropyl, benzyl)pyrazolo[4,3-d]pyrimidine, 7-(4-methoxy-2-methylanilino)-3-(methyl, ethyl, isopropyl, 3-pentyl, cyclopropyl, benzyl)pyrazolo[4,3-d]pyrimidine, 7-(5-methoxy-2-methylanilino)-3-(methyl, ethyl, isopropyl, 3-pentyl, cyclopropyl, benzyl)pyrazolo[4,3-d]pyrimidine, 6-[4-methoxy-3-(trifluoromethyl)anilino]-3-(methyl, ethyl, isopropyl, 3-pentyl, cyclopropyl, benzyl)pyrazolo[4,3-d]pyrimidine, 7-(2-methylanilino)-3-(methyl, ethyl, isopropyl, 3-pentyl, cyklopropyl, benzyl)pyrazolo[4,3-d]pyrimidine, 7-(4-methylanilino)-3-(methyl, ethyl, isopropyl, 3-pentyl, cyclopropyl, benzyl)pyrazolo[4,3-d]pyrimidine, 7-(3-methylanilino)-3-(methyl, ethyl, isopropyl, 3-pentyl, cyclopropyl, benzyl)pyrazolo[4,3-d]pyrimidine, 7-[2-methyl-3-(trifluoromethoxy)anilino]-3-(methyl, ethyl, isopropyl, 3-pentyl, cyclopropyl, benzyl)pyrazolo[4,3-d]pyrimidine, 7-[2-methyl-4-(trifluoromethoxy)anilino]-3-(methyl, ethyl, isopropyl, 3-pentyl, cyclopropyl, benzyl)pyrazolo[4,3-d]pyrimidine, 7-[2-(methylthio)anilino]-3-(methyl, ethyl, isopropyl, 3-pentyl, cyclopropyl, benzyl)pyrazolo[4,3-d]pyrimidine, 7-[4-(methylthio)anilino]-3-(methyl, ethyl, isopropyl, 3-pentyl, cyclopropyl, benzyl)pyrazolo[4,3-d]pyrimidine, 7-(2-nitroanilino)-3-(methyl, ethyl, isopropyl, 3-pentyl, cyclopropyl, benzyl)pyrazolo[4,3-d]pyrimidine, 7-(3-nitroanilino)-3-(methyl, ethyl, isopropyl, 3-pentyl, cyclopropyl, benzyl)pyrazolo[4,3-d]pyrimidine, 7-(4-nitroanilino)-3-(methyl, ethyl, isopropyl, 3-pentyl, cyclopropyl, benzyl)pyrazolo[4,3-d]pyrimidine, 7-(2-nitro-4,5,6-trifluoroanilino)-3-(methyl, ethyl, isopropyl, 3-pentyl, cyclopropyl, benzyl)pyrazolo[4,3-d]pyrimidine, 7-[2-nitro-4-(trifluoromethoxy)anilino]-3-(methyl, ethyl, isopropyl, 3-pentyl, cyclopropyl, benzyl)pyrazolo[4,3-d]pyrimidine, 7-(2-nitrotetrafluoroanilino)-3-(methyl, ethyl, isopropyl, 3-pentyl, cyclopropyl, benzyl)pyrazolo[4,3-d]pyrimidine, 7-(2,3,4,5,6-pentabromoanilino)-3-(methyl, ethyl, isopropyl, 3-pentyl, cyclopropyl, benzyl)pyrazolo[4,3-d]pyrimidine, 6-(2,3,4,5,6-pentafluoroanilino)-3-(methyl, ethyl, isopropyl, 3-pentyl, cyclopropyl, benzyl)pyrazolo[4,3-d]pyrimidine, 7-(2,3,4,5-tetrachloroanilino)-3-(methyl, ethyl, isopropyl, 3-pentyl, cyclopropyl, benzyl)pyrazolo[4,3-d]pyrimidine, 7-(2,3,5,6-tetrachloroanilino)-3-(methyl, ethyl, isopropyl, 3-pentyl, cyclopropyl, benzyl)pyrazolo[4,3-d]pyrimidine, 7-(4-(1,1,2,2-tetrafluoroethoxy)anilino)-3-(methyl, ethyl, isopropyl, 3-pentyl, cyclopropyl, benzyl)pyrazolo[4,3-d]pyrimidine, 7-(2,3,4,5,-tetrafluoroanilino)-3-(methyl, ethyl, isopropyl, 3-pentyl, cyclopropyl, benzyl)pyrazolo[4,3-d]pyrimidine, 7-(2,3,4,6,-tetrafluoroanilino)-3-(methyl, ethyl, isopropyl, 3-pentyl, cyclopropyl, benzyl)pyrazolo[4,3-d]pyrimidine, 7-(2,3,5,6,-tetrafluoroanilino)-3-(methyl, ethyl, isopropyl, 3-pentyl, cyclopropyl, benzyl)pyrazolo[4,3-d]pyrimidine, 7-[2,3,5,6-tetrafluoro-4-(trifluoromethyl)anilino]-3-(methyl, ethyl, isopropyl, 3-pentyl, cyclopropyl, benzyl)pyrazolo[4,3-d]pyrimidine, 7-(2,4,6-tribromoanilino)-3-(methyl, ethyl, isopropyl, 3-pentyl, cyclopropyl, benzyl)pyrazolo[4,3-d]pyrimidine, 7-(2,4,6-tribromo-3,5-dijodoanilino)-3-(methyl, ethyl, isopropyl, 3-pentyl, cyclopropyl, benzyl)pyrazolo[4,3-d]pyrimidine, 7-(2,3,4-trichloroanilino)-3-(methyl, ethyl, isopropyl, 3-pentyl, cyclopropyl, benzyl)pyrazolo[4,3-d]pyrimidine, 7-(2,4,5-trichloroanilino)-3-(methyl, ethyl, isopropyl, 3-pentyl, cyclopropyl, benzyl)pyrazolo[4,3-d]pyrimidine, 7-(2,4,6-trichloroanilino)-3-(methyl, ethyl, isopropyl, 3-pentyl, cyclopropyl, benzyl)pyrazolo[4,3-d]pyrimidine, 7-(2,4,5-trifluoroanilino)-3-(methyl, ethyl, isopropyl, 3-pentyl, cyclopropyl, benzyl)pyrazolo[4,3-d]pyrimidine, 7-(2,3,5-trifluoroanilino)-3-(methyl, ethyl, isopropyl, 3-pentyl, cyclopropyl, benzyl)pyrazolo[4,3-d]pyrimidine, 7-(2,3,6-trifluoroanilino)-3-(methyl, ethyl, isopropyl, 3-pentyl, cyclopropyl, benzyl)pyrazolo[4,3-d]pyrimidine, 7-(2,3,4-trifluoroanilino)-3-(methyl, ethyl, isopropyl, 3-pentyl, cyclopropyl, benzyl)pyrazolo[4,3-d]pyrimidine, 7-(2-trifluoromethoxyanilino)-3-(methyl, ethyl, isopropyl, 3-pentyl, cyclopropyl, benzyl)pyrazolo[4,3-d]pyrimidine, 7-(3-trifluoromethoxyanilino)-3-(methyl, ethyl, isopropyl, 3-pentyl, cyclopropyl, benzyl)pyrazolo[4,3-d]pyrimidine, 7-(4-trifluoromethoxyanilino)-3-(methyl, ethyl, isopropyl, 3-pentyl, cyclopropyl, benzyl)pyrazolo[4,3-d]pyrimidine, 7-(2,3,4-trifluoro-6-nitroanilino)-3-(methyl, ethyl, isopropyl, 3-pentyl, cyclopropyl, benzyl)pyrazolo[4,3-d]pyrimidine, 7-(2,4,5-trimethylanilino)-3-(methyl, ethyl, isopropyl, 3-pentyl, cyclopropyl, benzyl)pyrazolo[4,3-d]pyrimidine, 7-(2,4,6-trimethylanilino)-3-(methyl, ethyl, isopropyl, 3-pentyl, cyclopropyl, benzyl)pyrazolo[4,3-d]pyrimidine, 7-(3-chloro-4-carboxyanilino)-3-(methyl, ethyl, isopropyl, 3-pentyl, cyclopropyl, benzyl)pyrazolo[4,3-d]pyrimidine, 7-(3-carboxy-4-hydroxyanilino)-3-(methyl, ethyl, isopropyl, 3-pentyl, cyclopropyl, benzyl)pyrazolo[4,3-d]pyrimidine, 7-cyclohexylamino-3-(methyl, ethyl, isopropyl, 3-pentyl, cyclopropyl, benzyl)pyrazolo[4,3-d]pyrimidine, 7-cyclopentylamino-3-(methyl, ethyl, isopropyl, 3-pentyl, cyclopropyl, benzyl)pyrazolo[4,3-d]pyrimidine, 7-cyclobutylamino- 3-(methyl, ethyl, isopropyl, 3-pentyl, cyclopropyl, benzyl)pyrazolo[4,3-d]pyrimidine, 7-allylamino-3-(methyl, ethyl, isopropyl, 3-pentyl, cyclopropyl, benzyl)pyrazolo[4,3-d]pyrimidine, 7-diallylamino-3-(methyl, ethyl, isopropyl, 3-pentyl, cyclopropyl, benzyl)pyrazolo[4,3-d]pyrimidine 7-isopentylamino-3-(methyl, ethyl, isopropyl, 3-pentyl, cyclopropyl, benzyl)pyrazolo[4,3-d]pyrimidine, 7-(3,3-dimethylallylamino)-3-(methyl, ethyl, isopropyl, 3-pentyl, cyclopropyl, benzyl)pyrazolo[4,3-d]pyrimidine, 7-(3-hydroxymethyl-3-methylallyl)amino-3-(methyl, ethyl, isopropyl, 3-pentyl, cyclopropyl, benzyl)pyrazolo[4,3-d]pyrimidine, 7-propargylamino-3-(methyl, ethyl, isopropyl, 3-pentyl, cyclopropyl, benzyl)pyrazolo[4,3-d]pyrimidine, 7-furfurylamino-3-(methyl, ethyl, isopropyl, 3-pentyl, cyclopropyl, benzyl)pyrazolo[4,3-d]pyrimidine, 7-(oxazol-4-yl)amino-3-(methyl, ethyl, isopropyl, 3-pentyl, cyclopropyl, benzyl)pyrazolo[4,3-d]pyrimidine, 7-(2-pyridylamino)-3-(methyl, ethyl, isopropyl, 3-pentyl, cyclopropyl, benzyl)pyrazolo[4,3-d]pyrimidine, 7-(3-pyridylamino)-3-(methyl, ethyl, isopropyl, 3-pentyl, cyclopropyl, benzyl)pyrazolo[4,3-d]pyrimidine, 7-(4-pyridylamino)-3-(methyl, ethyl, isopropyl, 3-pentyl, cyclopropyl, benzyl)pyrazolo[4,3-d]pyrimidine, 7-(4-morfolinyl)- 3-(methyl, ethyl, 3-pentyl, benzyl)pyrazolo[4,3-d]pyrimidine, 7-(1-chinuklidinyl)-3-(methyl, isopropyl, 3-pentyl, benzyl)pyrazolo[4,3-d]pyrimidine, 7-(1-etyleniminyl)-3-(methyl, ethyl, 3-pentyl, benzyl)pyrazolo[4,3-d]pyrimidine, 7-(1-propyleniminyl)-3-(methyl, isopropyl, 3-pentyl, benzyl)pyrazolo[4,3-d]pyrimidine, 7-(1-pyrolidinyl)-3-(methyl, ethyl, isopropyl, 3-pentyl, benzyl)pyrazolo[4,3-d]pyrimidine, 7-(1-piperidinyl)-3-(methyl, ethyl, isopropyl, 3-pentyl, cyclopropyl, benzyl)pyrazolo[4,3-d]pyrimidine, 7-(1-piperazinyl)-3-(methyl, ethyl, isopropyl, 3-pentyl, cyclopropyl, benzyl)pyrazolo[4,3-d]pyrimidine, 7-pyrazol-3-(methyl, ethyl, isopropyl, 3-pentyl, cyclopropyl, benzyl)pyrazolo[4,3-d]pyrimidine, 7-imidazol-3-(methyl, ethyl, isopropyl, 3-pentyl, benzyl)pyrazolo[4,3-d]pyrimidine, 7-(1-imidazolinyl)-3-(methyl, ethyl, 3-pentyl, cyclopropyl, benzyl)pyrazolo[4,3-d]pyrimidine, 7-(1-pyrazolinyl)-3-(methyl, ethyl, isopropyl, 3-pentyl, cyclopropyl, benzyl)pyrazolo[4,3-d]pyrimidine.

The novel compounds of this invention per se or as intermediates in the preparation of novel compound having a wide variety of industrial utilities.

The compounds of the formula I and their pharmaceutically acceptable salts inhibit selectively the enzyme p34^{cdc2}/cyclin B kinase and related cdks (cdk1, cdk2, cdk5, cdk7, MAP kinases).

In another embodiment, this invention relates to the use of the compounds as defined in the claims for inhibiting cdks and cell proliferation and/or for inducing apoptosis in plants comprising administering an effective amount to the plant.

In still another embodiment, this invention relates to the use of the compounds as defined in the claims for treating fungal infections (fungi) in humans, animals and plants.

Diubstituted pyrazolo[4,3-d]pyrimidine derivatives result in the acquisition of extremely high potency against viruses on the part of the defined compounds. An important aspect of the present invention is the use of the compounds as defined in the claims for inhibiting proliferation of a DNA virus dependent upon events associated with cell proliferation for replication. The DNA virus includes any of the retrovirus family. The efective amount is that sufficient to inhibit cellular CDK activity to extent impending viral replication.

In addition to other CDK1-related kinases, this kinase controls certain steps of cell division cycles, in particular the transition from G₁ phase into the S phase and in particular the transition from the G₂ phase into the M-phase. Out the basis of this findings, it can be expected that the compounds of the formula I and their acceptable salts can be used as antimitotic compounds and for treatment of proliferative diseases.

In addition to therapeutic applications it will be apparent the subject compounds can be used as a cell culture additive for controlling proliferative and/or differentiation states of cells *in vitro,* for instance, by controlling the level of activation of a CDK. By preventing the activation of a Go/G₁ CDK, the subject inhibitors can prevent mitotic progression and hence provide a means for ensuring an adequately restrictive environment in order to maintain cells at various stages of differentiations, and can be employed, for instance, in cell cultures designed to test the specific activities of trophic factors. Other tissue culture systems which require maintenance of differentiation will be readily apparent to those skilled in the art

It is likely that inhibition by the compounds, of the invention of the catalytic activity of cyclin-dependent kinases in mediated by interaction of the compounds at the ATP-binding site of the enzyme. Such compounds are particularly desirable for reducing excessive cell growth, since they allow inhibition of the kinase activity regardless of the cause underlying the excessive kinase activity leading to excessive cell proliferation. Thus, the compounds of the invention are active in situations in which the excessive kinase activity results from the kinase being a mutated hyperactive, form of the kinase and situations in which the kinase is present at excessive levels. Such compounds can also block excessive kinase activity in situations in which the cyclin regulating the kinase is present at excessive levels or its binding to the kinase is enhanced. Furthermore, compounds which block kinase activity by interacting with the ATP binding site of the enzyme are also useful for inhibiting kinase activity in situations in which a natural inhibitor of cyclin-kinase complexes is mutated.

It will also be aparent that differential screening assays can be used to select for those compounds of the present invention with specificity for CDK enzymes. Thus, compounds, which act specifically on eukaryotic pathogens, e.g., are anti-fungal or anti-parasitic agents, can be selected from the subject of the inhibitors.

By way of illustration, the assays described in the art can be used to screen for agents which may ultimately be useful for inhibiting at leas one fungus implicated in such mycosis as aspergillosis, blastomycosis, chromoblastomycosis, coccidiomycosis, conidiosporosis, actinomycosis, penicilliosis, monoliasis, or sporotrichosis. For example, if the mycotic infection to which treatment is desired is aspergillosis, an assay as described above or in the appended examples can comprise comparing the relative effectiveness of a test compound on inhibiting a plant CDK enzyme with its effectiveness towards a CDK enzyme from yeast. Likewise, the differential screening assays can be used to identify anti-fungal agents which may have value in the treatment of aspergillosis by making use of the CDK genes cloned from yeast such as *Aspergillus fumigatus, Aspergillus flavus, Aspergillus niger, Aspergillus nidulans,* or *Apergillus terreus.*

In yer another embodiment, certain of the subject CDK inhibitors can be selected on the basis of inhibitory specificity for plant CDK^{..}s relative to the mammalian enzyme. For example, a plant CDK can be identified in a differential screen with one or more of the human enzymes to select those compounds of greatest selectivity for inhibiting the plant enzyme. Thus, the present invention specifically contemplates formulations of the subject CDK inhibitors for agricultural applications, such as in the form of a defoliant or the like.

### PROCESSES FOR PREPARATION

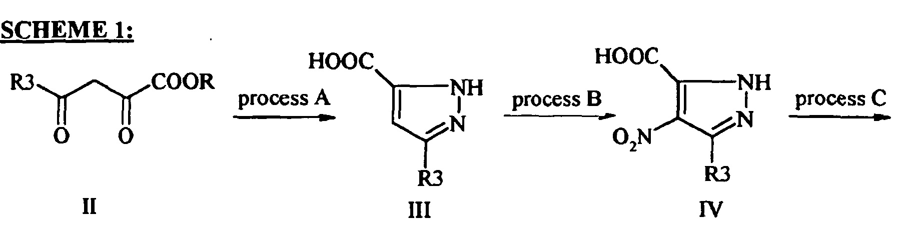

process A: N₂H₄·H₂O/T=96°C
process B: H₂SO₄/HNO₃
process C: ROH /HCl
process D: RaNi + H₂ / CH₃OH+H₂O; Pd or Pt + H₂/ CH₃OH+CH₃COOH, SnCl₂; S₂O₄²⁻

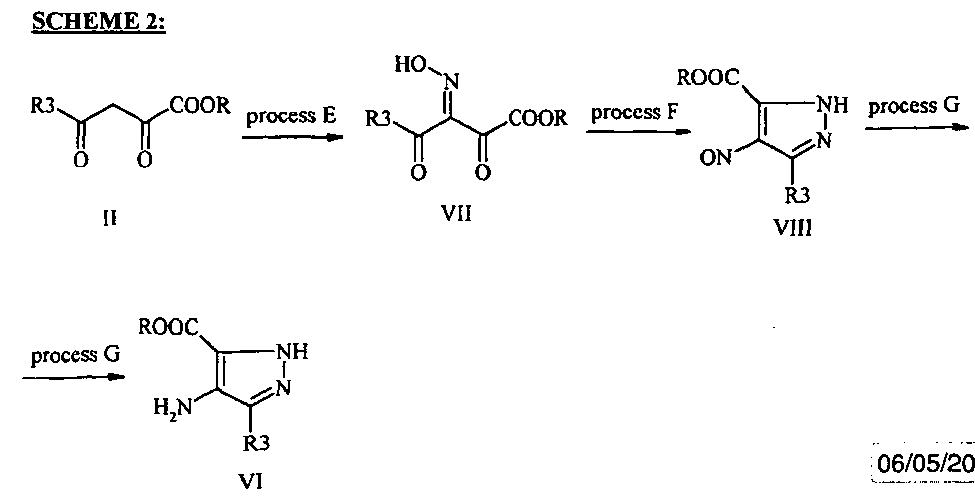

process E: NaNO₂ / HCl in C₂H₅OH or N₂O₃ (g)
process F: N₂H₄ H₂O
process G: Na₂S₂O₄ / EtOAc+H₂O

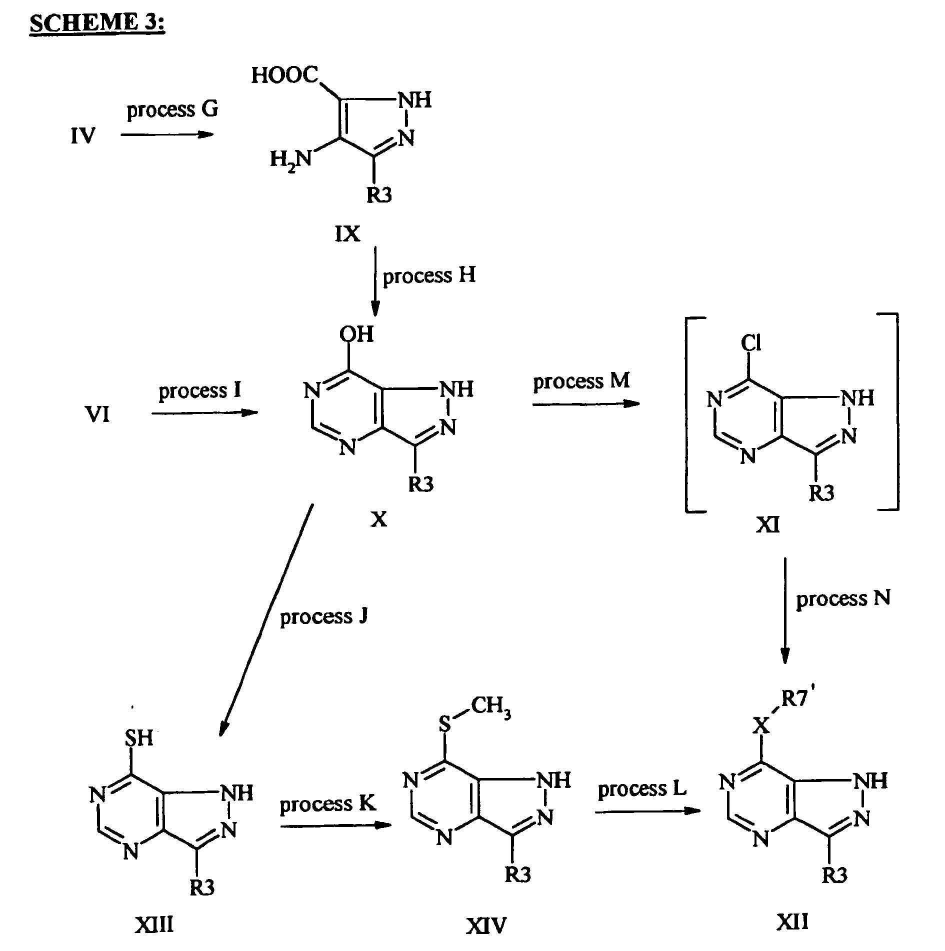

process G: Na₂S₂O₄ / EtOAc+H₂O
process H: formamide/ reflux
process I: formamidin acetate/ Et₃N/ 2-ethoxyethanol/ 90°C
process J: P₂S₅/ pyridine/ reflux
process K: CH₃I/ 1 M KOH
process L: R7'NH₂ or R7'ONa (K,Li) or R7'SNa (K,Li))
process M: SOCl₂/CHCl₃/ DMF /T=80°C or POCl₃ or POCl₃ / PCl₅
process N: R7'NH₂ or R7'ONa (K,Li) or R7'SNa (K,Li))

In one approach the 3-isopropyl-7-substituted pyrazolo[4,3-d]pyrimidine of the formula **I**, wherein R7 substituents are defined above for a compound of the formula **I**, are prepared by reaction of 7-chloro-3-isopropylpyrazolo[4,3-d]pyrimidine with appropriate nucleophile, such as 1)amine (ammonium hydroxide, hydrazine, hydroxylamine, benzylamine; 2-, 3-, 4-hydroxybenzylamine; dihydroxybenzylamine; 3-chloroaniline, etc.) or 2) lithium(sodium, potassium) salt of alcohol or mercaptane. Preferably, the appropriate nucleophil reactant may be R7'-X-Y, wherein R7'-X- is as defined in the claims and Y is H. A nucleophil is a reagent which is able to attack a position in a molecule with absence of electrons. An appropriate alkylating agent is a reagent which is source of carbo cations which attack a position in a molecule with excess of electrons - preferentially free electron couples, usually oxygen, nitrogen and sulfur, such as R7'-Z, wherein R7' is as defined in the claims and Z is selected from halogen, toluenesulfonate, and mesylate. 7-Chloro-3-isopropylpyrazolo[4,3-d]pyrimidine is dissolved in chloroform and appropriate R^{7'}-NH₂ or R^{7'}-O(Li, Na,K), or R7'-S(Li, Na,K) (5 - 20 eq.) was added. After heating for several hours, the reaction mixture is cooled and the 7-substituted-3-isopropylpyrazolo[4,3-d]pyrimidine is obtained.

In another approach the 3-ethyl substituted pyrazolo[4,3-d]pyrimidine of the formula **I**, wherein R7 substituents are defined above for a compound of the formula **I**, are prepared by reaction of 7-chloro-3-ethylpyrazolo[4,3-d]pyrimidine with appropriate nucleophiles such as 1)amine (ammonium hydroxide, hydrazine, hydroxylamine, benzylamine; 2-, 3-, 4-hydroxybenzylamine; dihydroxybenzylamine; 3-chloroaniline, etc.) or 2) lithium(sodium, potassium) salt of alcohol or mercaptane. 7-Chloro-3-isopropylpyrazolo[4,3-d]pyrimidine is dissolved in chloroform and appropriate R^{7'}-NH₂ or R⁷'-O(Li, Na,K), or R^{7'}-S(Li, Na,K) (5 - 20 eq.) was added. After heating for several hours, the reaction mixture is cooled and the 7-substituted-3-ethylpyrazolo[4,3-d]pyrimidine is obtained.

In yet another approach the 3-methyl substituted pyrazolo[4,3-d]pyrimidine of the formula **I**, wherein R7 substituents are defined above for a compound of the formula **I**, are prepared by reaction of 7-chloro-3-methylpyrazolo[4,3-d]pyrimidine with appropriate nucleophiles such as 1)amine (ammonium hydroxide, hydrazine, hydroxylamine, benzylamine; 2-, 3-, 4-hydroxybenzylamine; dihydroxybenzylamine; 3-chloroaniline, etc.) or 2) lithium(sodium, potassium) salt of alkohole or mercaptane. 7-Chloro-3-isopropylpyrazolo[4,3-d]pyrimidine is dissolved in chloroform and appropriate R^{7'}-NH₂ or R^{7'}-O(Li, Na,K), or R^{7'}-S(Li, Na,K) (5 - 20 eq.) was added. After heating for several hours, the reaction mixture is cooled and the7-substituted-3-methylpyrazolo[4,3-d]pyrimidine is obtained.

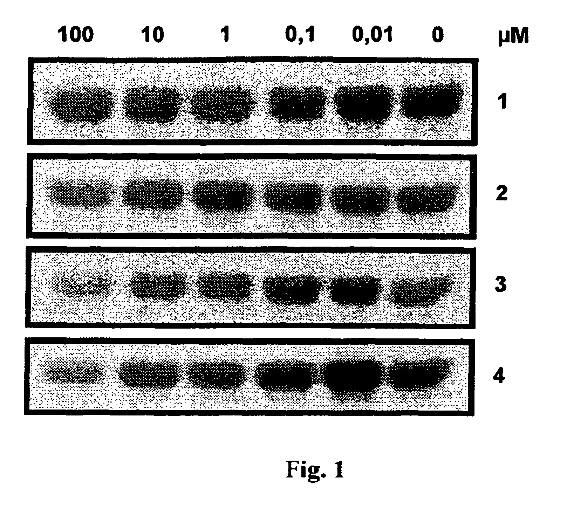

Further, Fig. 1 shows a diagram displaying the specific inhibition of plant cdc2 kinase activity in plant cells. Enzyme activity bound to p13^{suc1}-agarose was measured by phosphorylation of histone H1 substrate protein in the presence of various concentrations of (1) 7-furfurylamino-3-methylpyrazolo[4,3-d]pyrimidine, (2) 7-benzylamino-3-isopropylpyrazolo[4,3-d]pyrimidine, (3) 7-(3-chloroanilino)-3-isopropylpyrazolo[4,3-d]pyrimidine, (4) 7-(3-hydroxybenzyl)amino-3-isopropylpyrazolo[4,3-d]pyrimidine.

Fig. 2 shows pictures of the induction of aberrant mitosis in root meristem cells of *V*. *faba* after the treatment with 200 mM A2.16.32, wherein
pictures a-e - control cells
pictures a'-e' - cells treated with 200 mM A2.16.32 for 12 hr.
picturre a - lower magnification showing frequency of mitosis (a), and aberrant mitosis (a'). b,b' - prophase, c,c' - metaphase, d,d' - anaphase, e,e' - telophase.

Fig. 3 shows immunofluorescence visualization of microtubules in control (A) and treated (B) root meristem cells of *V.faba.* Green - FITC immunolabelling for alfa-tubulin (left column), red - immunolabelling for gamma-tubulin (middle column), blue- immunolabelling for DNA labelling with DAPI (right column).

Fig. 4 shows pictures of the electrophoretic detection of double strand DNA breaks (marker of apoptosis) after the treatment of root meristem cells of *Vicia faba* with bohemine (200 µM) wherein the slots are
1. molecular weight markers
2. control I - DNA after 10 h incubation without the drug
3. DNA after 10 h incubation with bohemine
4. control II - DNA after 44 hot incubation without the drug and
5. DNA after 44 hot incubation with bohemine

Fig 5 shows immunofluorescence detection of DNA double strand breaks in root meristem cells of *V.faba* treated with bohemine.
A. Root meristem cells of *V.faba,* treated with bohemine (200 mM). 1^{st} line
B. Control cells. 2nd. line

1. column - FITC labelling of DNA breaks.
2. column - DAPI labelling for chromatin
3. column - merged images of FITC and DAPI

### The following EXAMPLES serve to illustrate the invention without limiting the scope thereof.

The starting material for the compounds of the formula **I** is available from commercial sources (Sigma, Aldrich, Fluka, etc.). Melting points were determined on a Koffler block and are uncorrected. Evaporations were carried out on a rotary evaporator under vacuum at temperatures below 80°C. The ¹H NMR spectra (σ, ppm; J, Hz) were measured on Varian VXR-400 (400MHz) or on Varian Unity 300 (400MHz) instruments. All spectra were obtained at 25°C using tetramethylsilane as on a internal standard. Electron impact mass spectra m/z (rel.%, composition, deviation) were measured on a VG 7070E spectrometer (70eV, 200°C, direct inlet) Quadrupole mass spectra were measured on a Micromass ZMD detector with electrospray ionization. Merck silica gel Kieselgel 60 (230-400 mesh) was used for column chromatography. All compounds gave satisfactory elemental analyses (±0,4%).

### Reference EXAMPLE 1:

### methyl 2,4-dioxo-5-methylhexenoate (II): Prepared according to :

E Royals. J. Am. Chem. Soc. 67, 1508 (1945)

### EXAMPLE 2:

### 5-isopropylpyrazol-3-carbozylic acid (III)

A solution of methyl 2,4-dioxo-5-methylhexenoate II (31g; 180 mmol ) and N₂H₄·H₂O (13mL, 180 mmol ) in ethanol (120 mL ) was refluxed for 2 hours. The mixture was poured into H₂O and extracted with chloroform. The chloroform extract was concentraded under reduced pressure to give a yellow liquid The liquid and aq. 3 M NaOH (120 mL) was stirred overnight at room temperature. The acidification of the yellow solution to pH=2 with conc. HCl affords crystals. Crystals were filtered off and washed with ice-water. Yield 68%, mp=136-140°C.
¹H NMR(300MHz, MeOD): 1.14d(6H, 7.1Hz), 3.02sept.(1H, 7.1Hz), 6.59s(1H); CHN required C=54.54%; H=6.54%; N=18.17%; found: C=54.58%; H=6.38%, N=18.12%

### EXAMPLE 3:

### 5-isopropyl-4-nitropyrazole-3-carboxylic acid (IV):

To an ice-cooled and stirred solution of 2.9g (18.8mmol) 5-isopropylpyrazol-3-carboxylic acid III in fuming sulphuric acid 1 mL (65%,), sulphuric acid 7.6 mL (100%) and the nitric acid 3 mL (65%) was added portionwise. The stirring was continued for 1h at room temperature and then another 3h at 104°C temperature and then poured into ice-water. The white precipitate of product was filtered and crystallized from water; (yield 76%), mp=139-142°C; ¹H NMR(300MHz, DMSO): 1.22d(6H, 7.1Hz), 2.94sept(1H, 7.1Hz), 3.33s(1H), 6.45bs(1H); CHN required. C=42.02%; H=4.56%; N=21.09%; found: C=42.41%, H=4.49%; N=21 01%.

### EXAMPLE 4:

### Methyl 5-isopropyl-4-nitropyrazol-3-carboxylate (V)

5-Isopropyl-4-nitropyrazole-3-carboxylic acid was added to a 4 5M solution of HCl in absolute methanol. The reaction mixture was heated at 60°C for 7 hours and then was evaporated to dryness The title compound was crystallised from ethyl acetate; yield 91%; mp=78-80°C. ¹H NMR (300MHz, CDCl₃): 1.39d(6H, J=7.1Hz); 3.64sept(1H, J=7 1Hz), 3.98s(3H). CHN required. C=45.07%, H=5.20%; N=19.70% ; found: C=45.21%; H=5.23%; N=19 65%.

### EXAMPLE 5:

### Methyl 4-amino-5-isopropylpyrazol-3-carboxylate (VI)

### mode A

To a solution of methyl 5-isopropyl-4-nitropyrazol-3-carboxylate (4.34g, 24 mmol) in 20 mL ethanol and 5 mL water was added 1g RaNi (an activity W5). The mixture was stirred under hydrogen atmosphere (760 Torr) for 12 hours. The RaNi was filtered off and the filtrate was concenrated in vacuo. The residue crystals were washed with cooled ethylacetate; yield 95%; mp=122-123°C. MS(EI, 70eV, direct inlet): 183(88; C₈H₁₃N₃O₂^{.+.}; -1.0), 168(59), 152(3), 136(100), 108(8), 80(16), 68(20). ¹H NMR(400MHz, CDCl₃): 1.31d(6H; J=6.9Hz), 2.93sept(1H; J=6.9Hz), 3.9s(3H). IR (KBr, cm⁻¹): 3399, 3296, 1710, 1626, 1584,1302.

### mode B

To a solution of methyl 4-nitropyrazol-3-carboxylate V (7.34g, 34.4mmol) in 36 mL n-propanole, 6 mL water and 5.6 mL 10 M HCl was added 0.55g PtO₂. The mixture was stirred under hydrogen atmosphere (760 Torr) for 9 hours. The reaction mixture was filtered and the filtrate was concentrated to dryness in vacuo. The desired amine was liberated by treatment of aq. ammonia during extraction into chloroform. The product crystallised after evaporation; yield 95%; mp=122-123°C.

### EXAMPLE 6:

### 7-hydroxy-3-isopropylpyrazolo[4,3-d]pyrimidine (X)

A mixture of aminoester VI ( 1.5 g, 8.42 mmol ) , formamidine acetate ( 2.47 g, 24 mmol ) and triethylamine ( 5,25 mL ) in 32 mL of 2-ethoxyetanol was heated for 2 hours at 90°C under argon atmosphere. The excess of triethylamine was evaporated from cellosolve solution in vacuo, the crystallised product was filtered off and washed with chloroform. An analytical sample was obtained by recrystallisation from ethanol. Yield 96%; mp=302-304°C. ¹H-NMR ( 300MHz, CD₃OD ): 1.41d(6H, J=7.15Hz), 3.40sept(1H, J=7.15Hz), 7.82s(1H). ¹³C-NMR ( 400MHz, DMSO-d₆+AcOD ): 21.912, 25.985, 141.85, 172.17. CHN required: C=53.92%; H=5.66%; N=31.44; found: C=53.20%; H=5.58%; N=31.39%. MS (EI): 178(35; C₈H₁₀N₄O.⁺); 177(8) 163(25;C₇H₇N₄O.⁺); 150(18;C₆h₆N₄O.⁺); 54(18); 53(10); 41(11), 28(38); 27(11).

### EXAMPLE 7:

### [7-chloro-3-isopropylpyrazolo[4,3-d]pyrimidine (XI) ]

7-Hydroxy-3-isopropylpyrazolo[4,3-d]pyrimidine X (200 mg, 1.122 mmol) was dissolved in the mixture of 0.81 mL (11 mmol) of thionyl chloride, 0.12 mL (1.56 mmol) of dimethylformamide and 5 mL of chloroform. This mixture was heated under reflux for 3 hours. The solution was evaporated to dryness in vacuo and the residue was dissolved in chloroform. This solution was extracted twice with a small portions of water and combined chloroform extract was dried over Na₂SO₄. This compound VIII was not isolated and was used immediately as chloroform solution in following reaction step.

### EXAMPLE 8:

### 7-benzylamino-3-isopropylpyrazolo[4,3-d]pyrimidine XIIa

To chloroform solution of XI(prepared in the subsequent step from 200 mg X) was added 3 mL of benzylamine. This mixture was stirred at room temperature 10 minutes and then evaporated to dryness in vacuo. The crude product was purified by columm chromatography on silica gel, the mixture of chloroform/methanol (98.5 / 1.5) was used as mobile phase. Yield 82%; mp=153-154°C. ¹H-NMR (400MHz, CDCl₃): 1.40d(6H, J=7.02Hz); 3.41sept(1H, J=7.02Hz); 4.80s(2H), 7.25m(5H), 6.57s (1H), 8.4s (1H). CHN required: C=67.39%; H=6.41%; N=26.20; found: C=67.33%; H=6.43%; N=26.24%. MS (EI): 267(62;C₈H₁₀N₄O.⁺); 266(18) 252(44); 239(6); 106(49); 91(100); 65(21); 43(14); 41(16).

### EXAMPLE 9:

### 7-(2-hydrozybenzyl)amino-3-isopropylpyrazolo[4,3-d]pyrimidine XIIb

To chloroform solution of XI (prepared in the subsequent step from 200 mg X) was added 1 31 mL (7.7 mmol) *N*-ethyldiisopropylamine, 3 mL EtOH and 500 mg (4.06 mmol) 2-hydroxybenzylamine This mixture was heated one hour at 60°C and then was evaporated to dryness in vacuo The crude product was purified by chromatography on silica gel in the mixture of chloroform/methanol/AcOH (20.0.4.0.1). Yield 40%; mp=214-217°C. ¹H-NMR (400MHz, DMSO-d₆): 1.36d(6H, J=6.96Hz), 3.28sept(1H, J=6.96Hz); 4.65s(2H), 6.78-7 26m(4H), 8.21s (1H). CHN required: C=63.59%; H=6.05%, N=24.72; found : C=63 39%; H=6.07%; N=24.62%. MS (ES). [M+H]⁺=274.3 (100).

### EXAMPLE 10:

### 7-(3-hydroxybenzylamino)-3-isopropylpyrazolo[4,3-d]pyrimidine XIIc

To chloroform solution of XI (prepared in the subsequent step from 200 mg X) was added 1.5 mL (8.8 mmol) *N*-ethyldiisopropylamine, 5 mL EtOH and 500 mg (4.06 mmol) 3-hydroxybenzylamine. This mixture was heated one hour at 60°C and then was evaporated to dryness in vacuo. The crude product was purified by column chromatography on silica gel in the mixture of chloroform/methanol/AcOH (20:0.6:0.1). Yield 48%; mp=220-221°C. ¹H -NMR (300MHz, DMSO-d₆): 1.38d(6H, J=7.14Hz); 3.26sept(1H, J=7.14Hz); 4.68s(2H), 6.62-7.17m(3H), 8.22s (1H); 9.31s(1H). MS (ES): [M+H]⁺=274 3 (100).

### EXAMPLE 11:

### 7-(4-hydroxybenzyl)amino-3-isopropylpyrazolo[4,3-d]pyrimidine XIId

To chloroform solution of XI (prepared in the subsequent step from 200 mg X) was added 1 5 mL (8.8 mmol) *N*-ethyldiisopropylamine, 5 mL EtOH and 500 mg (4.06 mmol) 4-hydroxybenzylamine. This mixture was heated one hour at 60°C and then was evaporated to dryness in vacuo. The crude product was purified by column chromatography on silica gel in the mixture of chlorofom-/methanol/AcOH (20:1:0.1). Yield 49%; mp=234-236°C. ¹H-NMR (300MHz, DMSO-d₆): 1.28d(6H, J=6.59Hz); 3.58sept(1H, J=6.59Hz); 4 60s(2H), 6.73-7.21m(4H), 8.20s (1H) MS (ES): [M+H]⁺=274.3 (100)

### EXAMPLE 12:

### 7-(3-chloroanilino)-3-isopropylpyrazolo[4,3-d]pyrimidine XIIe

To chloroform solution of XI (prepared in the subsequent step from 200 mg X) was added 1.20 ml (11.2 mmol) 3-chloroaniline. This mixture was heated one hour at 60°C, then was cooled at room temperature and crystals were precipitated These colourless crystals were washed with ether, the analytical sample was obtained by recrystallisation from mixture ethanol-ether. Yield 58%, mp=213-216°C ¹H-NMR (400MHz, DMSO-d₆): 1.40d(6H, J=6.94Hz); 3.48sept(1H, J=6.94Hz); 7.30dd(1H), 7.49dd(1H), 786dd(1H), 8.20s(1H), 8.78s (1H). CHN (C₁₄H₁₄N₅Cl·HCl·H₂O) required: C=49.20%; H=5.01%; N=20.48%; Cl=20.48%; found: C=49.43%; H=5.09%; N=20 13%; Cl=20.58%. MS (ES): [M+H]⁺=288.5 (100), 290.5 (33).

### EXAMPLE 13:

### 7-(isopent-2-en-(1-yl)amino)-3-isopropylpyrazolo[4,3-d]pyrimidine XIIf

To chloroform solution of XI (prepared in the subsequent step from 200 mg X) was added 3 5 mL (20 mmol) *N*-ethyldiisopropylamine, 3 mL EtOH and 620 mg (4 7 mmol) isopent-2-en-1-(yl)amino hydrochloride. This mixture was stirred 12 hours at room temperature and then was evaporated to dryness in vacuo. The crude product was purified by column chromatography on silica gel in the mixture of chloroform/methanol/aq. NH₄OH (98:2:1). Yield 48%; syrupy. ¹H-NMR (400MHz, CDCl₃): 145d(6H, J=6.96Hz); 1.65d(1H, J=1.28Hz), 3.47sept(1H, J=6.96Hz); 4.18d(2H, J=1.28Hz), 5.25m(1H, J=1.28Hz), 6.25s(1H), 8.22s (1H). COSY[1.45d(6H, J=6.96Hz); 3.47sept(1H, J=6.96Hz)], COSY[1.65d(6H, J=1.28Hz); 4.18d(2H, J=1.28Hz); 525m(1H, J=1.28Hz)], COSY[4.18d(2H), 6.25s(1H)]. MS (ES): [M+H]⁺=246.5 (100).

### EXAMPLE 14:

### 7-furfurylamino-3-isopropylpyrazolo[4,3-d]pyrimidine XIIg

To the chloroform solution of XI (prepared in the subsequent step from 150 mg X) was added 2 mL ( 0 205 mol) furfurylamine. This mixture was stirred 1 hour at 50°C and then was evaporated to dryness in vacuo. The crude product was purified by column chromatography on silica gel in the mixture of chloroform/methanol/aq. NH₄OH (98:2:0.2). Yield 43%; mp=179-182°C ¹H-NMR (500MHz, MeOD): 1.422d (6H, J=7.0Hz); 3 455sept (1H, J=7 0Hz); 4 802s (2H), 6 373s (2H); 7 468s (1H); 8.273s (1H). MS (ES): [M+H]⁺=258.3 (100).

### EXAMPLE 15:

### 7-pentylamino-3-isopropylpyrazolo[4,3-d]pyrimidine XIIh

To the chloroform solution of XI (prepared in the subsequent step from 150 mg X) was added 0.29 mL ( 2.53 mmol) 3-pentylamine. This mixture was stirred 1 hour at 50°C and then was evaporated to dryness in vacuo The crude product was purified by column chromatography on silica gel in the mixture of chloroform/methanol (99:1). Yield 25%, mp=73-75°C. ¹H-NMR (500MHz, MeOD): 0.933t (3H, J=7.0Hz); 1.374bs (2H); 1.388bs (2H); 1.418d (6H, J=6.9Hz); 1.715pent (2H, J=7.0Hz) 3.447hept (1H, J=6 9Hz), 3.583t (2H, J=7.0Hz); 8.207s (1H). MS (ES): [M+H]⁺=248.2 (100)

### EXAMPLE 16:

### 7-(2-bromobenzyl)amino-3-isopropylpyrazolo[4,3-d]pyrimidine XIIk

To chloroform solution of XI (prepared in the subsequent step from 152 mg X, 0.855 mmol) was added 0.44 mL (2.60 mmol) *N*-ethyldiisopropylamine, 2 ml methanol and 343 mg (1.54 mmol) 2-bromobenzylamine hydrochloride. This mixture was heated two hours at 60°C and then was evaporated to dryness in vacuo. The crude product was purified by column chromatography on silica gel in the mixture of chloroform/methanol (98.5:1.5) crystallization from Et₂O Yield 42%, mp=194-196°C. ¹H-NMR (300MHz, CH₃OD): 1 44d(6H, J=6.9Hz); 3.43hept(1H, J=6.9Hz); 4.89s(2H); 7.20t(1H, J=7.1Hz); 7.32t(1H, J=7.1Hz); 7.45bs(1H), 7.62d(1H, J=7 1Hz), 8.24s (1H). MS (ES): [M+H]⁺=246.2 (95), 248.2 (100).

### EXAMPLE 17:

### 7-(4-methoxybenzyl)amino-3-isopropylpyrazolo[4,3-d]pyrimidine XIII

To chloroform solution of XI (prepared in the subsequent step from 152 mg X, 0.855 mmol) was added 0.34 mL (2.60 mmol) 4-methoxybenzylamine. This mixture was heated one hour at 52°C and then was evaporated to dryness in vacuo The crude product was purified by column chromatography on silica gel in the mixture of chloroform/methanol (98:2), crystallization from Et₂O Yield 42%; mp=143-144°C. ¹H-NMR (300MHz, CDCl₃): 1 36d(6H, J=6.9Hz); 3.46sept. (1H, J=6.9Hz), 3.69s (3H), 4.86bs (2H); 6.72d (2H, J=8.8Hz); 7.30d (2H, J=8.8Hz); 8.34s (1H). MS (ES): [M+H]⁺=298.3 (100).

### EXAMPLE 18:

### 7-(3-hydroxy-4-methoxybenzyl)amino-3-isopropylpyrazolo[4,3-d]pyrimidine XIIm

To chloroform solution of XI (prepared in the subsequent step from 152 mg X, 0.855 mmol) was added 0.44 mL (2.60 mmol) *N*-ethyldiisopropylamine, 7 mL methanol and 236 mg (1.54 mmol) 3-hydroxy-4-methoxybenzylamine. This mixture was heated one hour at 52°C and then was evaporated to dryness in vacuo. The crude product was purified by column chromatography on silica gel in the mixture of chloroform/methanol/NH₄OH (93.5:6.5:0.1) crystallization from chloroform/Et₂O. Yield 62%; mp=197-199°C. ¹H-NMR (300MHz, CH₃OD): 1.43d(6H, J=7.15Hz); 4.45hept(1H, J=7.15Hz); 3.83s(3H); 4.67s(2H), 6.82-6.90m (3H), 8.24s (1H). MS (ES): [M+H]⁺=314.3 (100).

**Table 1: Compounds Prepared by the Method of Examples 8-18**

| PYRAZOLO[4,3-d]PYRIMIDINE SUBSTITUENT | | | CHN ANALYSES | MS ANALYSES-ZMD | |
|---|---|---|---|---|---|
| | C7 | ^{C3} | [%] | [M-H]⁻a) | [M+H]⁺ b) |
| 1 * | 3-chloroanilino | methyl | C=55.50;H=3.88 | | 260.1 |
| | | | N=26.97;Cl=13.65 | | 262. 1 |
| 2 * | anilino | methyl | C=63.99;H=4.92 N=31.09 | | 226.1 |
| 3 * | 4-bromoanilino | methyl | C=47 39;H=3.31 | 303.0 | 304.0 |
| | | | N=23.03, Br=26.27 | 305.0 | 306 0 |
| 4 * | 4-chloroanilino | methyl | C=55.50;H=3.88 | | 260.1 |
| | | | N=26 97,Cl=13.65 | | 262.1 |
| 5 * | 2-hydroxybenzylamino | methyl | C=61 17,H=5 13 N=27 43 | 254.1 | 256 .1 |
| 6 * | 3-hydroxybenzylamimo | methyl | C=61.17;H=5.13 N=27.43 | 254.1 | 256.1 |
| 7 * | 4- hydroxybenzylamino | methyl | C=61. 17,H=5 13 N=27 43 | 254.1 | 256.1 |
| 8 * | 2-methylbenzylamino | methyl | C=66.38,H=5 79 N=27.65 | | 254 .1 |
| 9 * | 3-methylbenzylamino | methyl | C=66.38;H=5.79 N=27.65 | | 254.1 |
| 10 * | 4-methoxybenzylamino | methyl | C=62.44;H=5.61 N=26.00 | | 270.0 |
| 11 * | 2-chlorobenzylamino | methyl | C=57.04;H=4.42 | | 274.1 |
| | | | N=25.59; Cl=12.95 | | 276.1 |
| 12 * | 2-bromobenzylamino | methyl | C=49.08;H=3.80 | | 319.0 |
| | | | N=22.01; Br=25.11 | | 321.0 |
| 13 * | 3-chlorobenzylamino | methyl | C=57.04;H=4.42 | | 274.1 |
| | | | N=25.59; Cl=12.95 | | 276.1 |
| 14 * | 3-hydroxy-4-methoxybenzylamino | methyl | C=58.94;H=5.30 N=24.55 | | 286.0 |
| 15 * | furfurylamino | methyl | C=57.63;H=4.84 N=30.55 | | 230.1 |
| 16 * | allylamino | methyl | C=57.13;H=5.86 N=37.01 | | 190.1 |
| 17 * | cyclohexylamino | methyl | C=62.31;H=7.41 N=30.28 | | 232.2 |
| 18 * | *1,4- (trans)* -cyclohexyldiamino | methyl | C=58.52;H=7.37 N=34.12 | | 247.0 |
| 19 * | *1,2*-*(cis)*-cyclohexyldiamino | methyl | C=58.52;H=7.37 N=34.12 | | 247.0 |
| 20 * | cyclopentylamino | methyl | C=60.81;H=6.96 N=32.23 | | 218.1 |
| 21 * | cyclobutylamino | methyl | C=59.10;H=6.45 N=34.46 | | 204.1 |
| 22 * | (isopent-2-en-1-yl)amino | methyl | C=60.81;H=6.96 N=32.23 | | 204.1 |
| 23 * | pentylamino | methyl | C=60.25;H=7.81 N=31.94 | | 220.0 |
| 24 * | 4-chlorobenzylamino | methyl | C=57.04;H=4.42 | | 274.1 |
| | | | N=25.59; Cl=12.95 | | 276.1 |

| | | | | | |
|---|---|---|---|---|---|
| a) solution: MeOH p.a. + HCOOH b) solution: MeOH p.a. + H₂O + NH₃ * Compounds 1-24 do not relate to claimed subject matter. | | | | | |

**Table 2: Compounds Prepared by the Method of Examples 8-18**

| PYRAZOLO[4,3-d]PYRIMIDINE SUBSTITUENT | | | CHN ANALYSES | MS ANALYSES-ZMD | |
|---|---|---|---|---|---|
| | C7 | C3 | [%] | [M-H]⁻ a) | [M+H]⁺b) |
| 25 | 3-chloroanilino | ethyl | C=57.04;H=4.42 | | 274.1 |
| | | | N=25.59;Cl=12.95 | | 276.1 |
| 26 | anilino | ethyl | C=65.26;H=5.48 N=29.27 | | 240.1 |
| 27 | 4-bromoanilino | ethyl | C=49.07;H=3.80 | 317.0 | 318.0 |
| | | | N=22.01; Br--25.11 | 319.0 | 320.0 |
| 28 | 4-chloroanilino | ethyl | C=57.04;H=4.42 | | 274.1 |
| | | | N=25.59;Cl=12.95 | | 276.1 |
| 29 | 2-hydroxybenzylamino | ethyl | C=62.44;H-5.61 N=26.00 | 268.1 | 270.1 |
| 30 | 3-hydrorrybenzylamino | ethyl | C=62.44;H=5.61 N=26.00 | 268.1 | 270.1 |
| 31 | 4- hydroxybenzylamino | ethyl | C=62.44;H=5.61 N=26.00 | 268.1 | 270.1 |
| 32 | 2-methylbenzylamino | ethyl | C=67.39;H=6.41 N=26.20 | | 268.2 |
| 33 | 3-methylbenzylamino | ethyl | C=67.39;H=6.41 N=26.20 | | 268.2 |
| 34 | 4- methoxybenzylamino | ethyl | C=63.59;H=6.05 N=24.72 | | 284.0 |
| 35 | 2-chlorobenzylamino | ethyl | C=58.44;H=4.90 | | 288.1 |
| | | | N=24.34; Cl=12.32 | | 290.1 |
| 36 | 2-bromobenzylamino | ethyl | C=50.62;H=4.25 | | 333.0 |
| | | | N=21.08; Br=24.05 | | 335.0 |
| 37 | 3-chlorobenzylamino | ethyl | C=58.44;H=4.90 | | 288.1 |
| | | | N=24.34; Cl=12.32 | | 290.1 |
| 38 | 3-hydroxy-4-methoxybenzylamino | ethyl | C=60.19;H=5.72 N=23.40 | | 300.0 |
| 39 | furfurylamino | ethyl | C=59.25;H=5.39 N=28.79 | | 244.1 |
| 40 | allylamino | ethyl | C=59.10;H=6.45 N=34.46 | | 204.1 |
| 41 | cyclohexylamino | ethyl | C=63.65;H=7.81 N=28.55 | | 246.2 |
| 42 | 1,4-*(trans)*-cyclohexyldiamino | ethyl | C=59.98;H=7.74 N=32.28 | | 261.0 |
| 43 | 1,2-*(cis)*-cyclohexyldiamino | ethyl | C=59.98;H=7.74 N=32.28 | | 261.0 |
| 44 | cyclopentylamino | ethyl | C=62.31;H=7.41 N=30.28 | | 232.2 |
| 45 | cyclobutylamino | ethyl | C=60.81;H=6.96 N=32.23 | | 218.1 |
| 46 | (isopent-2-en-1-yl)amino | ethyl | C=62.31;H=7.41 N=30.28 | | 232.2 |
| 47 | pentylamino | ethyl | C=61.78;H=8.21 N=30.02 | | 234.0 |
| 48 | 4-chlorobenzylamino | ethyl | C=58.44;H=4.90 | | 288.1 |
| | | | N=24.34; Cl=12.32 | | 290.1 |

| | | | | | |
|---|---|---|---|---|---|
| a) solution: MeOH p.a. + HCOOH b) solution: MeOH p.a. + H₂O + NH₃ | | | | | |

**Table 3: Compounds Prepared by the Method of Examples 8-18**

| PYRAZOLO[4,3-d]PYRIMIDINE SUBSTITUENT | | | CHN ANALYSES | MS ANALYSES-ZMD | |
|---|---|---|---|---|---|
| | C7 | C3 | [%] | [M-H]⁻a) | [M+H]⁺b) |
| 49 | 3-chloroanilino | isopropyl | C=58,44;H=4,90 | | 288.5 |
| | | | N=24,34;Cl=12,32 | | 290.5 |
| 50 | anilino | isopropyl | C=66,38;H=5,97 N=27,65 | | 254.1 |
| 51 | 4-bromoanilino | isopropyl | C=50.62;H=4.25 | 331.1 | 332.1 |
| | | | N=21.08; Br=24.05 | 333.1 | 334.1 |
| 52 | 4-chloroanilino | isopropyl | C=58,44;H=4,90 | 286.1 | 288.1 |
| | | | N=24,34;Cl=12,32 | 288.1 | 290.1 |
| 53 | 2-hydroxybenzylamino | isopropyl | C=63.59;H=6.05 N=24.72 | 272.3 | 274.3 |
| 54 | 3-hydroxybenzylamino | isopropyl | C=63.59;H=6.05 N=24.72 | 272.3 | 274.3 |
| 55 | 4- hydroxybenzylamino | isopropyl | C=63.59;H=6.05 N=24.72 | 272.3 | 274.3 |
| 56 | 2-methylbenzylamino | isopropyl | C=68.30;H=6.81 N=24.89 | | 282.2 |
| 57 | 3-methylbenzylamino | isopropyl | C=68.30;H=6.81 N=24.89 | | 282.2 |
| 58 | 4-methoxybenzylamino | isopropyl | C=64.63;H=6.44 N=23.55 | 296.3 | 298.3 |
| 59 | 2-chlorobenzylamino | isopropyl | C=59.70;H=5.34 | | 302.1 |
| | | | N=23.21; Cl=11.75 | | 304.1 |
| 60 | 2-bromobenzylamino | isopropyl | C=52.04;H=4.66 | 344.1 | 346.1 |
| | | | N=20.23; Cl=23.08 | 346.1 | 348.1 |
| 61 | 3-chlorobenzylamino | isopropyl | C=59.70;H=5.34 | | 302.1 |
| | | | N=23.21; Cl=11.75 | | 304.1 |
| 62 | 3-hydroxy-4-methoxybenzylamino | isopropyl | C=61.33;H=6.11 N=22.35 | 312.3 | 314.3 |
| 63 | furfurylamino | isopropyl | C=60.69;H=5.88 N=27.22 | | 258.3 |
| 64 | allylamino | isopropyl | C=60.81;H=6.96 N=32.23 | | 218.1 |
| 65 | cyclohexylamino | isopropyl | C=64.84;H=8.16 N=27.00 | | 260.2 |
| 66 | *1,4- (trans)* -cyclohexyldiamino | isopropyl | C=61.29;H=8.08 N=30.63 | 273.3 | 275.3 |
| 67 | 1,2-*(cis)*-cyclohexyldiamino | isopropyl | C=61.29;H=8.08 N=30.63 | 273.3 | 275.3 |
| 68 | cyclopentylamino | isopropyl | C=63.65;H=7.81 N=28 55 | | 246.2 |
| 69 | cyclobutylamino | isopropyl | C=62.31,H=7.41 N=30.28 | | 232.2 |
| 70 | isopent-(2-en)-1-ylamimo | isopropyl | C=63.65;H=7.81 N=28.55 | | 246.5 |
| 71 | pentylamino | isopropyl | C=63 13;H=8.56 N=28.31 | | 248.2 |
| 72 | 4-chlorobenzylamino | isopropyl | C=59.70;H=5.34 | | 302.1 |
| | | | N=23.21; Cl=11.75 | | 304.1 |

| | | | | | |
|---|---|---|---|---|---|
| a) solution: MeOH p.a. + HCOOH b) solution: MeOH p.a. + H₂O + NH₃ | | | | | |

### EXAMPLE 19: CDK Inhibition Assays

### Proteins

Cyclin-dependent kinases (P34^{cdc2} , p33^{cdk2}) and cyclins (cyclin B, E) are produced in Sf9 insect cells coinfected with appropriate baculoviral constructs. The cells are harvested 68-72 hrs post infection in lysis buffer for 30 min on ice and the soluble fraction is recovered by centrifugation at 14.000 g for 10 min. The protein extract is stored at -80 °C.

Lysis buffer: 50mM Tris pH 7.4, 150mM NaCl, 5mM EDTA, 20mM NaF, 1% Tween, 1mM DTT, 0.1mM PMSF, leupeptine, aprotonine.

### Enzyme inhibition assays

To carry out experiments on kinetics under linear conditions, the final point test system for kinase activity measurement is used. The kinase is added to reaction mixture in such a way as to obtain linear activity with respect to the concentration of enzyme and with respect to time.

The p34^{cdc2} and p33^{cdk2} kinase inhibition determination involves the use of 1mg/ml histone H1 (Sigma, type III-S) in the presence of 15 µM [γ-³³P]ATP (500-100 cpm/pmol) (ICN) in a final volume of 10 µl. Kinase activity is determined at 30 °C in the kinase buffer.

Tested compounds are usually dissolved to 100 mM solutions in DMSO, final concentration of DMSO in reaction mixture never exceeds 1%. The controls contain suitable dilutions of DMSO.

After 10 min, addition 3x SDS sample buffer stops the incubations. Phosphorylated proteins are separated electrophoretically using 10 % SDS polyacrylamide gel. The measurement of kinase activity is done using digital image analysis.

The kinase activity is expressed as a percentage of maximum activity, the apparent inhibition constants are determined by graphic analysis.

Kinase buffer: 50 mM Hepes pH 7.4, 10 mM MgCl₂, 5 mM EGTA, 10 mM
2-glycerolphosphate, 1 mM NaF, 1 mM DTT

**Table 4: Kinase Inhibitory Activity of Selected 3,7-Disubstituted Pyrazolo[4,3-d]pyrimidine Derivatives**

| SUBSTITUENT | | CDK1 | CDK2 |
|---|---|---|---|
| C7 | C3 | IC₅₀ (µM) | IC₅₀ (µM) |
| **Olomoucine** | | 7 | 7 |
| 3-chloroanilino * | methyl | 14 | 16 |
| anilino * | methyl | 22 | 21 |
| 3-chloro-5-aminoanilino * | methyl | 19 | 24 |
| 3-chloro-4-carboxyanilino * | methyl | 25 | 28 |
| 3-carboxy-4-chloroanilino * | methyl | 24 | 29 |
| 3-carboxy-4-hydroxyanilino * | methyl | 34 | 40 |
| 4-bromoanilino * | methyl | 16 | 18 |
| 4-chloroanilino * | methyl | 26 | 28 |
| 3-amino-4-chloroanilino * | methyl | 27 | 28 |
| 3-chloro-4-aminoanilino * | methyl | 26 | 28 |
| 2-hydroxybenzylamino * | methyl | 4 | 5.2 |
| 3-hydroxybenzylamino * | methyl | 5.5 | 7.2 |
| 2-methylbenzylamino * | methyl | 18 | 17 |
| 3-methylbenzylamino * | methyl | 28 | 31 |
| 2-chlorobenzylamino * | methyl | 25 | 24 |
| 3-chlorobenzylamino * | methyl | 8.8 | 9.4 |
| furfurylamino * | methyl | 18 | 16 |
| allylamino * | methyl | 42 | 48 |
| cyclohexylamino * | methyl | 34 | 32 |
| cyclopentylamino * | methyl | 29 | 46 |
| cyclobutylamino * | methyl | 35 | 28 |
| isopentenylamino * | methyl | 45 | 44 |
| 4-chlorobenzylamino * | methyl | 20 | 18 |
| benzylamino | ethyl | 12 | 11 |
| 3-chloroanilino | ethyl | 24 | 18 |
| anilino | ethyl | 14 | 20 |
| 3-chloro-5-aminoanilino | ethyl | 20 | 15.5 |
| 3-chloro-4-carboxyanilino | ethyl | 36 | 32 |
| 3-carboxy-4-chloroanilino | ethyl | 38 | 40 |
| 3-carboxy-4-hydroxyanilino | ethyl | 24 | 22 |
| 4-bromoanilino | ethyl | 22 | 18 |
| 4-chloroanilino | ethyl | 14 | 16 |
| 3-amino-4-chloroanilino | ethyl | 23 | 22 |
| 3-chloro-4-aminoanilino | ethyl | 35 | 28 |
| 2-hydroxybenzylamino | ethyl | 6.2 | 7 |
| 3-hydroxybenzylamino | ethyl | 6 | 3.2 |
| 2-methylbenzylamino | ethyl | 15 | 14 |
| 3-methylbenzylamino | ethyl | 18 | 17 |
| 2-chlorobenzylamino | ethyl | 12 | 12 |
| 3-chlorobenzylamino | ethyl | 9.7 | 10.2 |
| furfurylamino | ethyl | 8.2 | 5.3 |
| allylamino | ethyl | 25 | 29 |
| cyclohexylamino | ethyl | 39 | 42 |
| cyclopentylamino | ethyl | 32 | 32 |
| cyclobutylamino | ethyl | 27 | 25 |
| isopentenylamino | ethyl | 45 | 41 |
| 4-chlorobenzylamino | ethyl | 26 | 19 |
| benzylamino | isopropyl | 1.3 | 0.5 |
| 3-chloroanilino | isopropyl | 2.0 | 0.8 |
| anilino | isopropyl | 4.4 | 5,2 |
| 3-chloro-5-aminoanilino | isopropyl | 6.1 | 5.8 |
| 3-chloro-4-carboxyanilino | isopropyl | 2.5 | 2.8 |
| 3-carboxy-4-chloroanilino | isopropyl | 2.8 | 2.9 |
| 3-carboxy-4-hydroxyanilino | isopropyl | 3.1 | 4.2 |
| 4-bromoanilino | isopropyl | 1.7 | 1.9 |
| 4-chloroanilino | isopropyl | 2.1 | 3.1 |
| 3-amino-4-chloroanilino | isopropyl | 2.9 | 3.0 |
| 3-chloro-4-aminoanilino | isopropyl | 2.9 | 3.0 |
| 2-hydroxybenzylamino | isopropyl | 0,4 | 0,27 |
| 3-hydroxybenzylamino | isopropyl | 1,1 | 0.9 |
| 4-hydroxybenzylamino | isopropyl | 1.8 | 0.2 |
| 4-methoxybenzylamino | isopropyl | 2.3 | 1.0 |
| 2-methylbenzylamino | isopropyl | 2 | 1.4 |
| 3-methylbenzylamino | isopropyl | 2.2 | 3.1 |
| 2-chlorobenzylamino | isopropyl | 2.1 | 2.0 |
| 3-chlorobenzylamino | isopropyl | 8.8 | 9.4 |
| 3-hydroxy-4-methoxy | isopropyl | 0,9 | 0,2 |
| 2-bromobenzylamino | isopropyl | 7 | 9 |
| furfurylamino | isopropyl | 1,8 | 0,8 |
| allylamino | isopropyl | 16 | 14 |
| cyclohexylamino | isopropyl | 80 | 95 |
| cyclopentylamino | isopropyl | 18 | 20 |
| (2-aminocyclohexyl)amino | isopropyl | >100 | >100 |
| (4-aminocyclohexyl)amino | isopropyl | 80 | 70 |
| pentylamino | isopropyl | 1,7 | 1.4 |
| isopentenylamino | isopropyl | 4 5 | 1.3 |
| 4-chlorobenzylamino | isopropyl | 1.8 | 1.1 |

| | | | |
|---|---|---|---|
| These compounds do not relate to claimed subject-matter | | | |

Table 4 shows the results of inhibitory activity of novel compounds against CDK1 and CDK2 in comparison with the data on a prototype compound (trisubstituted purine olomoucine). Most of the 3,7-disubstituted pyrazolo[4,3-d]pyrimidine derivatives showed marked inhibitory activity in *in vitro* kinase assays

### EXAMPLE 20: CDK Inhibitory Activity on Plant Kinases and Antimitotic Effects

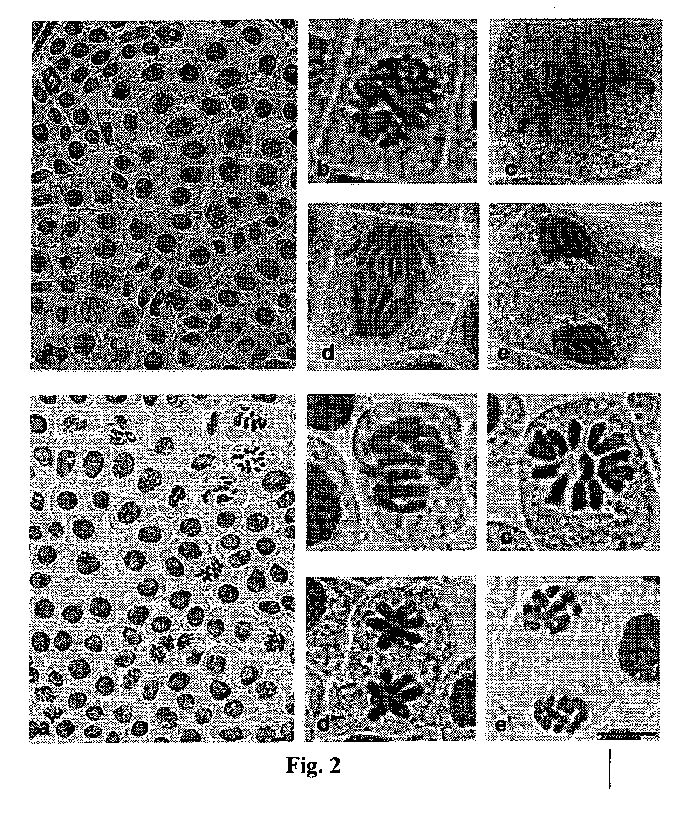

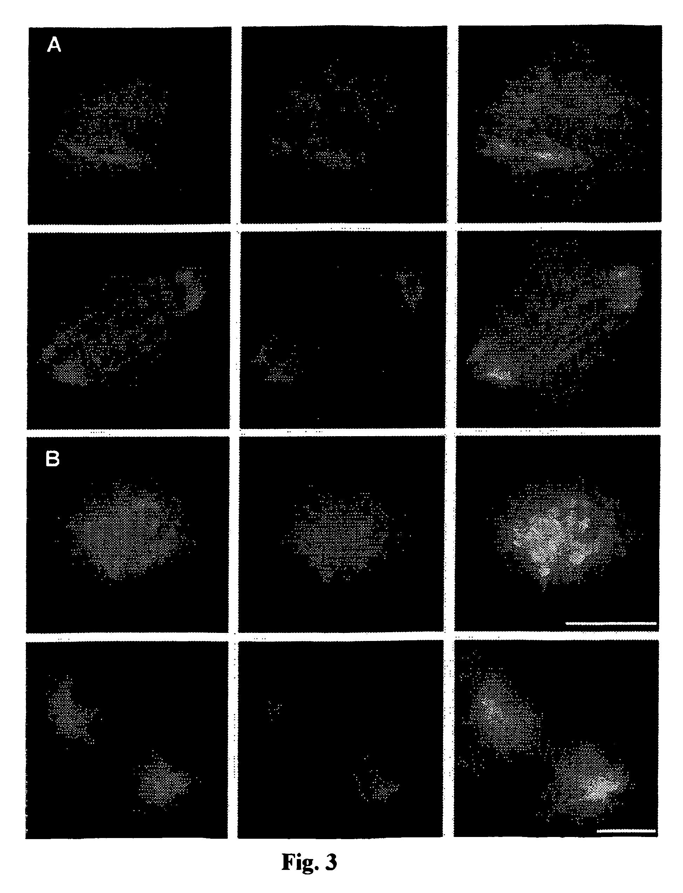

Protein extraction and purification of pant CDK by binding to p13^{suc1}-beads or immunopurification with an antibody specific to the cdc2a-MS protein was carried out as described previously (Bögre et al. 1997, Plant Physiol. 113, 1997, 841-852). The MMK1 protein kinase was purified with a specific antibody from *Vicia faba* extracts as described by Bögre te al. 1997a, Plant Cell 9, 75-83). Protein kinase activity was measured as described above in Example 19. The quantification of radioactivity incorporated into histone H1 or myelin basic protein was undertaken using Phosphoimager (original gel images shown on Fig. 1). IC₅₀ were calculated from dose-response curves. The drugs inhibited the activity of immunopurified *Vicia faba* and alfalfa Cdc2-kinase. An observed transient arrest at the G1/S and G2/M indicated that inhibition of the Cdc2-kinase had an effect on both transitions. In contrast to the regular bipolar spindle in untreated cell, in drug-treated metaphase cells abnormally short and dense kinetochore microtubule fibres were observed These microtubules were randomly arranged in the vicinity of the kinetochores and connected the chromosomes. Thus the chromosomes were not aligned on the metaphase plate but were arranged in a circle, with kinetochores pointing inwards and chromosome arms pointing outwards. γ-tubulin, which plays a role in microtubule nucleation, also localised to the centre of the monopolar spindle. The observed abnormalities in mitosis, after inhibition of Cdc2-kinase by specific drugs suggest a role for this enzyme in regulating some of the steps leading to a bipolar spindle structure (Fig. 2 and 3).

**Table 5: Kinase Inhibitory Activity of Selected 3,7-Disubstituted Pyrazolo[4,3-d]pyrimidine Derivatives**

| SUBSTITUENT | | CDC2a | MMK1 |
|---|---|---|---|
| C7 | C3 | IC₅₀ (µM) | IC₅₀ (µM) |
| **Olomoucine** | | **8** | **15.4** |
| 3-chloroanilino * | methyl | 10 | 9.6 |
| anilino * | methyl | 16 | 14 |
| 3-chloro-5-aminoanilino * | methyl | 21 | 24 |
| 3-chloro-4-carboxyanilino * | methyl | 27 | 29 |
| 3-carboxy-4-chloroanilino * | methyl | 25 | 30 |
| 3-carboxy-4-hydroxyanilino * | methyl | 32 | 44 |
| 4-bromoanilino * | methyl | 28 | 28 |
| 4-chloroanilino * | methyl | 26 | 26 |
| 3-amino-4-chloroanilino * | methyl | 35 | 34 |
| 3-chloro-4-aminoanilino * | methyl | 30 | 32 |
| 2-hydroxybenzylamino * | methyl | 4.2 | 4.8 |
| 3-hydroxybenzylamino * | methyl | 5.8 | 8.3 |
| 2-methylbenzylamino * | methyl | 20 | 20 |
| 3-methylbenzylamino * | methyl | 30 | 31 |
| 2-chlorobenzylamino * | methyl | 25 | 25 |
| 3-chlorobenzylamino * | methyl | 8.4 | 10 |
| furfurylamino * | methyl | 18 | 16 |
| allylamino * | methyl | 45 | 50 |
| Cyclohexylamino * | methyl | 35 | 30 |
| Cyclopentylamino * | methyl | 30 | 45 |
| Cyclobutylamino * | methyl | 35 | 28 |
| Isopentenylamino * | methyl | 45 | 44 |
| 4-chlorobenzylamino * | methyl | 20 | 18 |
| benzylamino | ethyl | 14 | 16 |
| 3-chloroanilino | ethyl | 22 | 21 |
| anilino | ethyl | 19 | 24 |
| 3-chloro-5-aminoanilino | ethyl | 25 | 28 |
| 3-chloro-4-carboxyanilino | ethyl | 24 | 29 |
| 3-carboxy-4-chloroanilino | ethyl | 34 | 40 |
| 3-carboxy-4-hydroxyanilino | ethyl | 16 | 18 |
| 4-bromoanilino | ethyl | 26 | 28 |
| 4-chloroanilino | ethyl | 27 | 28 |
| 3-amino-4-chloroanilino | ethyl | 26 | 28 |
| 3-chloro-4-aminoanilino | ethyl | 4 | 5.2 |
| 2-hydroxybenzylamino | ethyl | 5.5 | 7.2 |
| 3-hydroxybenzylamino | ethyl | 18 | 17 |
| 2-methylbenzylamino | ethyl | 28 | 31 |
| 3-methylbenzylamino | ethyl | 25 | 24 |
| 2-chlorobenzylamino | ethyl | 8.8 | 9.4 |
| 3-chlorobenzylamino | ethyl | 18 | 16 |
| furfurylamino | ethyl | 42 | 48 |
| allylamino | ethyl | 34 | 32 |
| cyclohexylamino | ethyl | 29 | 46 |
| cyclopentylamino | ethyl | 35 | 28 |
| cyclobutylamino | ethyl | 45 | 44 |
| isopentenylamino | ethyl | 20 | 18 |
| 4-chlorobenzylamino | ethyl | 8 | 5.3 |
| benzylamino | isopropyl | 1.1 | 1.2 |
| 3-chloroanilino | isopropyl | 2.0 | 0.8 |
| anilino | isopropyl | 4.4 | 5.2 |
| 3-chloro-5-aminoanilino | isopropyl | 2.8 | 2.9 |
| 3-chloro-4-carboxyanilino | isopropyl | 3.2 | 4.1 |
| 3-carboxy-4-chloroanilino | isopropyl | 1.6 | 1.7 |
| 3-carboxy-4-hydroxyanilino | isopropyl | 2.2 | 3.6 |
| 4-bromoanilino | isopropyl | 3.2 | 3.7 |
| 4-chloroanilino | isopropyl | 3.1 | 3.0 |
| 3-amino-4-chloroanilino | isopropyl | 4.1 | 1.8 |
| 3-chloro-4-aminoanilino | isopropyl | 0.6 | 0.4 |
| 2-hydroxybenzylamino | isopropyl | 1.7 | 1.3 |
| 3-hydroxybenzylamino | isopropyl | 2.1 | 2.7 |
| 4-hydroxybenzylamino | isopropyl | 2.2 | 1.8 |
| 4-methoxybenzylamino | isopropyl | 2.4 | 3.1 |
| 2-methylbenzylamino | isopropyl | 1.9 | 2.2 |
| 3-methylbenzylamino | isopropyl | 7.4 | 8.4 |
| 2-chlorobenzylamino | isopropyl | 1.7 | 0.7 |
| 3-chlorobenzylamino | isopropyl | 2.0 | 1.4 |
| 2-bromobenzylamino | isopropyl | 5.5 | 12 |
| furfurylamino | isopropyl | 4.3 | 5.3 |
| allylamino | isopropyl | 13.1 | 15 |
| cyclohexylamino | isopropyl | 30 | 34 |
| cyclopentylamino | isopropyl | 20 | 22 |
| isopentenylamino | isopropyl | 14 | 15 |
| (2-aminocyclohexyl)amino | isopropyl | >100 | >100 |
| pentylamino | isopropyl | 3.2 | 4.2 |
| 4-chlorobenzylamino | isopropyl | 1.7 | 1.6 |

| | | | |
|---|---|---|---|
| * These compounds do not relate to claimed subject-matter | | | |

Table 5 shows the results of inhibitory activity of novel compounds against plant in comparison with the data on the prototype compounds (disubstituted purines olomoucine, roscovitine and purvalanol A). Most of the 3,7-disubstituted pyrazolo[4,3-d]pyrimidine derivatives showed marked inhibitory activity in *in vitro* plant kinase assays.

### EXAMPLE 21: In vitro Cytotoxic Activity of Novel Compounds

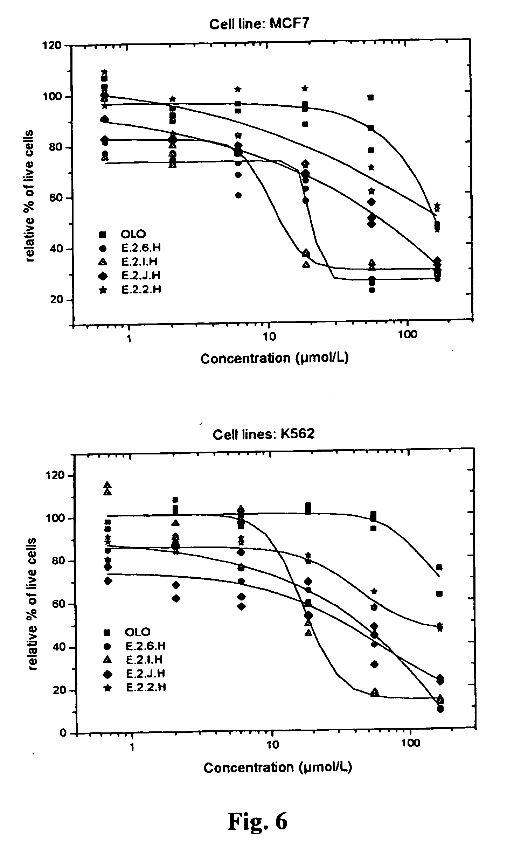

We have been using the following cell lines: *HELA* (human cervical carcinoma), *MCF7* (human breast adenocarcinoma), *NIH 3T3* (mouse fibroblasts), *HOS* (human osteogenic sarcoma), *HL 60* (human promyelocytic leukemia), *G 361* (human malignant melanoma), *K562* (human chronic myeloblastic leukemia), *CEM* (human lymphoblastoid leukaemia). Tested drugs were added to the cell cultures in six different concentration and kept at 37 °C and 5% CO₂ for three days. All cell lines were grown in DMEM medium (Gibco BRL) supplemented with 10% (v/v) fetal bovine serum and L-glutamine and maintained at 37 °C in a humidified atmosphere with 5% CO₂. 10⁴ cells were seeded into each well of 96 well plate, allowed to stabilize for at least 2 h and then tested compounds were added at various concentrations ranging from 200 to 0,2 µM in triplicates. Three days after drug addition Calcein AM solution (Molecular Probes) was added and let to enter the cells for 1 hour. Fluorescence of viable cells was quantified employing Fluoroskan Ascent (Microsystems). The IC₅₀ value, the drug concentration lethal to 50% of the tumour cells, was calculated from the obtained dose response curves (Fig. 6).

Cytoxicity of novel compounds was tested on panel of cell lines of different histogenetic and species origin (Tab. 6). Higher activities were found in all tumour cell lines tested. Notably, the higher effectiveness of novel derivatives was also found in cell lines bearing various mutations or deletions in cell cycle associated proteins, e.g. HL-60, BT549, Hela, U2OS, MDA-MB231, and Saos2. It indicates that these substances should be equally effective in tumours with various alterations of tumour suppressor genes, namely p53, Rb, etc. Importantly, this observation distinguishes the novel compounds from flavopiridol and related compounds, as their biological activity is dependent on p53 status.

**Table 6: Cytotoxicity of Novel Compounds for Different Cancer Cell Lines**

| SUBSTITUENT | | MCF7 | K-562 |
|---|---|---|---|
| C7 | C3 | IC₅₀ (µM) | IC₅₀ (µM) |
| **Olomoucine** | | 131,8 | >167 |
| benzylamino * | methyl | 67 | 89 |
| 3-chloroanilino * | methyl | 24 | 35 |
| 2-hydroxybenzylamino * | methyl | 67 | 55 |
| 3-hydroxybenzylamino * | methyl | 119 | 143 |
| 4-hydroxybenzylamino * | methyl | >167 | >167 |
| 3-hydroxy-4-methoxybenzylamino * | methyl | 58 | 79 |
| 4-methoxybenzylamino * | methyl | 35 | 53 |
| furfurylamino * | methyl | 142 | >167 |
| pentylamino * | methyl | 45 | 67 |
| cyclobutylamino * | methyl | 89 | 101 |
| 4-aminocyclohexylamino * | methyl | 75 | 115 |
| 2-bromobenzylamino * | methyl | 56 | 68 |
| benzylamino | ethyl | 62 | 81 |
| 3-chloroanilino | ethyl | 19 | 28 |
| 2-hydroxybenzylamino | ethyl | 63 | 51 |
| 3-hydroxybenzylamino | ethyl | 111 | 132 |
| 3-hydroxy-4-methoxybenzylamino | ethyl | 52 | 72 |
| 4-methoxybenzylamino | ethyl | 31 | 48 |
| furfurylamino | ethyl | 135 | >167 |
| pentylamino | ethyl | 41 | 62 |
| cyclobutylamino | ethyl | 82 | 94 |
| 4-aminocyclohexylamino | ethyl | 71 | 110 |
| 2-bromobenzylamino | ethyl | 54 | 60 |
| benzylamino | isopropyl | 55 | 72 |
| 3-chloroanilino | isopropyl | 9 | 12 |
| anilino | isopropyl | 15 | 21 |
| 3-chloro-5-aminoanilino | isopropyl | 29 | 35 |
| 3-chloro-4-carboxyanilino | isopropyl | 46 | 69 |
| 3-carboxy-4-chloroanilino | isopropyl | 0.4 | 1 |
| 3-carboxy-4-hydroxyanilino | isopropyl | 12 | 25 |
| 4-bromoanilino | isopropyl | 5 | 7 |
| 4-chloroanilino | isopropyl | 3 | 4 |
| 3-amino-4-chloroanilino | isopropyl | 0..2 | 0.3 |
| 3-chloro-4-aminoanilino | isopropyl | 12 | 13 |
| 2-hydroxybenzylamino | isopropyl | 63 | 50 |
| 3-hydroxybenzylamino | isopropyl | 105 | 132 |
| 4-hydroxybenzylamino | isopropyl | 152 | >167 |
| 3-hydroxy-4-methoxybenzylamino | isopropyl | 45 | 68 |
| 4-methoxybenzylamino | isopropyl | 28 | 41 |
| 2-methylbenzylamino | isopropyl | 63 | 75 |
| 3-methylbenyzylamino | isopropyl | 76 | 94 |
| 2-chlorobenzylamino | isopropyl | 15 | 21 |
| 3-chlorobenzylamino | isopropyl | 24 | 26 |
| furfurylamino | isopropyl | 130 | >167 |
| allylamino | isopropyl | 64 | 77 |
| cyclohexylamino | isopropyl | 95 | 98 |
| pentylamino | isopropyl | 32 | 61 |
| cyclobutylamino | isopropyl | 45 | 60 |
| isopentenylamino | isopropyl | >167 | >167 |
| 2-aminocyclohexylamino | isopropyl | > 167 | > 167 |
| 4-aminocyclohexylamino | isopropyl | 68 | 107 |
| 2-bromobenzylamino | isopropyl | 42 | 57 |
| 2-hydroxy-3 -methoxybenzylamino | isopropyl | 0.6 | 0 8 |
| 2-hydroxy-4-methoxybenzylamino | isopropyl | 0 2 | 0.5 |
| 2-hydroxy-5-methoxybenzylamino | isopropyl | 0.9 | 1.2 |
| 2-aminobenzylamino | isopropyl | 0.8 | 2.1 |

| | | | |
|---|---|---|---|
| * These compounds do not relate to claimed subject-matter. | | | |

### EXAMPLE 9: Novel Compounds Have Cytotoxic Effects for Plant Cells and Induce their Apoptosis

The novel compounds have also been tested in tobacco callus bioassay for cytotoxicity (herbicidal activity) and induction of cell death. The compounds to be tested were dissolved in dimethylsulfoxide (DMSO) and the solution brought up to 10⁻³ M with distilled water. This tock solution was further diluted in the respective media used for the tobacco bioassay to concentration ranging from 10⁻⁸ M to 10⁻⁴ M. The final concentration of DMSO in the media did not exceed 0.2%, and therefore did not affect biological activity in the assay system used. Cytokinin-dependent tobacco callus *Nicotiana tabacum* L. cv. Wisconsins 38 Murashige-Skoog medium, containing per 1 liter: 4µmol nicotinic acid, 2.4 µmol pyridoxine hydrochloride, 1.2 µmol thiamine, 26.6 µmol glycine, 1.37 µmol glutamine, 1.8 µmol myoinositol, 30 g of sucrose, 8 g of agar, 5.37 µmol α-naphtylacetic acid and 0.5 µmol 6-benzylaminopurine. Subcultivation was carried out every three weeks. Fourteen days before the bioassay, the callus tissue was transferred to the media without 6-benzylaminopurine. Compounds were tested with two different concentrations of 6-benzylaminopurine (10⁻⁵ M and 10⁻⁶ M). Inhibitory growth activity was determined from the increase in fresh callus weight after four weeks of cultivation. Five replicates were prepared for each concentration tested and the entire test was repeated at least twice. Inhibitory activity was compared with growth response curve of 6-benzylaminopurine in the range from 10⁻⁸ M to 10⁻⁴ M and IC50 was calculated for each compound for 10⁻⁵ M and 10⁻⁶ M of 6-benzylaminopurine.

**Table 7: Cytotoxicity of Novel Compounds for Tobacco Plant Cells Cultivated in vitro**

| SUBSTITUENT | | 10⁻⁵ M BAP | 10⁻⁶ M BAP |
|---|---|---|---|
| C7 | C3 | IC₅₀ (µM) | IC₅₀ (µM) |
| **OLOMOUCIN** | | **>50** | **>50** |
| benzylamino | isopropyl | 24 | 6.5 |
| 3-chloroanilino | isopropyl | 34 | 8.2 |
| anilino | isopropyl | 41 | 5.3 |
| 2-methylbenzylamino | isopropyl | 14 | 4.2 |
| 3-methylbenzylamino | isopropyl | 26 | 8 5 |
| 2-chlorobenzylamino | isopropyl | 31 | 4.6 |
| 3-chlorobenzylamino | isopropyl | 20 | 1.7 |
| furfurylamino | isopropyl | 25 | 9.4 |
| allylamino | isopropyl | 18 | 3.7 |
| cyclohexylamino | isopropyl | 46 | 9 4 |
| cyclopentylamino | isopropyl | 15.6 | 8.4 |
| cyclobutylamino | isopropyl | 12.1 | 4.3 |
| isopentenylamino | isopropyl | 15.3 | 1.7 |
| 4-chlorobenzylamino | isopropyl | 12.1 | 4.7 |

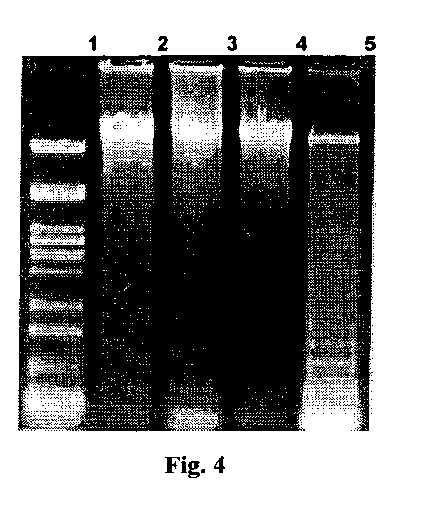

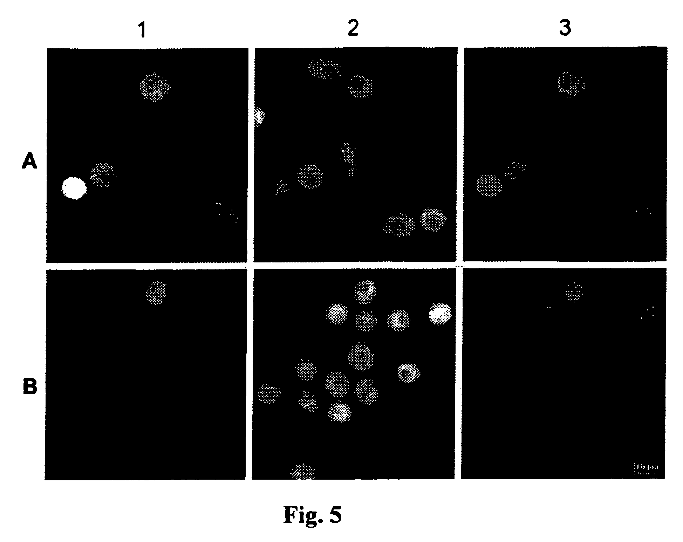

Table 7 shows the results of inhibitory activity of novel compounds on growth of tobacco cells cultivated *in vitro* in comparison with the data on the prototype compound (trisubstituted purine olomoucine). Most of the 3,7-disubstituted pyrazolo[4,3-d]pyrimidine derivatives showed marked inhibitory activity on *in vitro* growth. Furthermore, these compounds are able to induce apoptosis in plants cells (are able to kill plant cells) and induce strong antimitotic activities (see Fig.4 and 5). A dose-dependent inhibition of the cell cycle in G1/S and G2/M transition points was observed. The appearance of DNA fragmentation observed by DNA double strand breaks labelling in situ started 3h after drugs addition with highest frequency after 24-48h of treatment, when oligonucleosomal DNA ladder occurred. The high doses of roscovitine , bohemine which induced apoptosis were shown to downregulate in vivo activity of cdk; decrease of cdk protein level was shown by Western blotting and immunofluorescence labelling. Microtubule reorganization contributing to apoptotic morphology.was observed. The results presented here clearly show that the novel compounds exhibit herbicidal activity.

## Claims

1. 3,7-disubstituted pyrazolo[4,3-d]pyrimidines represented by the general formula **I** 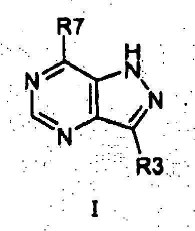 and pharmaceutically acceptable salts thereof, wherein
**R3** is ethyl, isopropyl n-butyl, isobutyl, tert.-butyl, 3-pentyl, benzyl, cyclopropyl, alkenyl, alkinyl, wherein each of the groups may optionally be substituted by halogen, hydroxyl, amino, mercapto, carboxyl, cyano, nitro, amido sulfo, sulfamido, carbamino, alkyl, alkoxy and/or an alkyl group substituted by one of these substitutents
**R7** is
**R7'-X-** wherein **X** is an -NH- or -N(alkyl)-, moiety and
**R7'** is selected from the group consisting of H, alkyl, cycloalkyl, aryl, alkylcycloalkyl, arylalkyl, heterocycle, heterocycloalkyl, substituted alkyl, substituted cycloalkyl, substituted aryl, substituted arylalkyl, substituted heterocycle, substituted heteroaryl, substituted heteroarylalkyl, substituted heteroalkyl, substituted cycloalkyl alkyl and substituted cycloheteroalkyl alkyl.

2. 3-, 7-disubstituted pyrazolo[4,3-d]pyrimidines according to claim 1, wherein the groups are substituted by more than one halogen, hydroxyl, amino, mercapto, carboxyl, cyano, nitro, amido, sulfo, sulfamido, carbamino, alkyl, alkoxy, and/or substituted alkyl group.

3. 3,7-disubstituted pyrazolo[4,3-d]pyrimidines according to claims 1 or 2, wherein
**R3** is ethyl, isopropyl, n-butyl, isobutyl, tert.-butyl, 3-pentyl, benzyl, cyclopropyl, C₂₋₈ alkenyl, C₃₋₈ alkinyl, which may be subsituted by 1-3 halogens like fluoro and chloro;
**R7** is
**R7'-X,** wherein X is -NH-, or -N(alkyl)- and
**R7'**-**X**, wherein X is preferably -N(alkyl)- selected at each occurrence from the group methyl, ethyl, propyl, isoproyl, ethinyl, ally, propargyl, isopent-2-en-1-yl.

4. 3-, 7-disubstituted pyrazolo[4,3-d]pynmidines according to any of the preceding claims, which has independently at each occurrence (R) or (S) configuration in R3 or R7 in case of their chirality.

5. A method of preparing the 3,7-disubstituted pyrazolo[4,3-d]pyrimidine of the formula 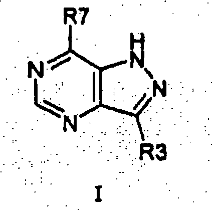 and pharmaceutically acceptable salts thereof, wherein
**R3** is selected from the group consisting of
C₁₋₈ alkyl, C₂₋₈ alkenyl, C₃₋₈ alkinyl, wherein each of the groups may optionally be substituted,
**R7** is
**R7'-X**- wherein **X** is an -NH- or -N(C₁₋₈ alkyl)-, moiety and
**R7'** is selected from the group consisting of H, alkyl, cycloalkyl, aryl, alkylcycloalkyl, aryl alkyl, heterocycle, heterocycloalkyl, substituted alkyl, substituted cycloalkyl, substituted aryl, substituted arylalkyl, substituted heterocycle, substituted heteroaryl, substituted heteroaryl alkyl, substituted heteroalkyl, substituted cycloalkyl alkyl and substituted cycloheteroalkyl alkyl, wherein 3-substituted-7-hydroxypyrazolo[4,3-d]pyrimidines are chlorinated, preferably with SOCl₂ or POCl₃ or PCl₅, or 7-hydroxy group is displaced by mercapto group, preferably by action of P₂S₅,
the thus obtained 7-chloro-3-substituted pyrazolo[4,3-d]pyrimidines are then reacted with an appropriate reactant nucleophil, or 7-mercapto-3-substituted pyrazolo[4,3-d]pyrimidines are reacted with an alkylating agent, and
the resulting 3,7-disubstituted pyrozolo[4,3-d]pyrimidine is optionally isolated.

6. The method of claim 5, wherein the appropriate nucleophil R7'-X-Y is selected from the group consisting of
1) amine, amonium hydroxide, hydrazine, hydroxylamine, benzylamine; 2-, 3-, 4-hydroxybenzylamine; dihydroxybenzylamine; and 3-chloraniline, or
2) lithium, natrium, and kalium salt of alkohole or mercaptane.

7. The method of claims 5 or 6, wherein the alkylating agent is selected from the group consisting of alkylhalogenide, alkyltoluensulfonate, and alkylmesylate.

8. The method of claims 5 to 7, wherein the resulting 3,7-disubstituted pyrazolo[4,3-d]pyrimidine is isolated by chromatography on silica gel followed by crystallization or only by crystallization.

9. Use of a 3,7-disubstituted pyrazolo[4,3-d]pyrimidine of the formula **I** 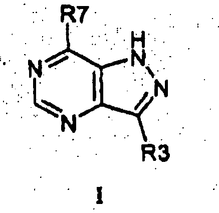 and pharmaceutically acceptable salts thereof, wherein
**R3** is selected from the group consisting of
C₁₋₈ alkyl, C₂₋₈ alkenyl, C₃₋₈ alkinyl, wherein each of the groups may optionally be substituted,
**R7** is
**R7'-X-** wherein **X** is an -NH- or, -N(C₁₋₈ alkyl)- moiety and
**R7'** is selected from the group consisting of H, alkyl, cycloalkyl, aryl, alkylcycloalkyl, aryl alkyl, heterocycle, heterocycloalkyl, substituted alkyl, substituted cycloalkyl, substituted aryl, substituted arylalkyl, substituted heterocycle; substituted heteroaryl, substituted heteroaryl alkyl, substituted heteroalkyl, substituted cycloalkyl alkyl and substituted cycloheteroalkyl alkyl for the preparation of a composition for inhibiting cell proliferation in mammals.

10. Use of a 3,7-disubstituted pyrazolo[4,3-d]pyrimidine of the formula **I** 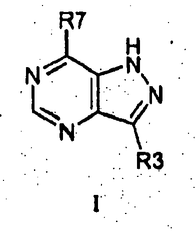 and pharmaceutically acceptable salts thereof, wherein
**R3** is selected from the group consisting of
C₁₋₈ alkyl, C₂₋₈ alkenyl, C₃₋₈ alkinyl, wherein each of the groups may optionally be substituted,
**R7** is
**R7'-X**- wherein X is an -NH-, or -N(C₁₋₈ alkyl)- moiety and
**R7'** is selected from the group consisting of H, alkyl, cycloalkyl, aryl, alkylcycloalkyl, aryl alkyl, heterocycle, heterocycloalkyl, substituted alkyl, substituted cycloalkyl, substituted aryl, substituted arylalkyl, substituted heterocycle, substituted heteroaryl, substituted heteroaryl alkyl, substituted heteroalkyl, substituted cycloalkyl alkyl and substituted cycloheteroalkyl alkyl for the preparation of a composition for treating cancer, or psoriasis, rheumatoid arthritis, lupus, type I diabetes, multiple sclerosis, restenosis, polycystic kidney disease, graft rejection, graft versus host disease and gout, parasitoses such as those caused by fungi or protists, or Alzheimer's disease, or as antineurogenerative drugs, or to suppress immunostimulation.

11. Use of a 3,7-disubstituted pyrazolo[4,3-d]pyrimidine of the formula **I** 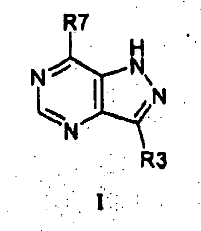 and pharmaceutically acceptable salts thereof, wherein
**R3** is selected from the group consisting of
C₁₋₈ alkyl, C₂₋₈ alkenyl, C₃₋₈ alkinyl, wherein each of the groups may optionally be substituted.
**R7** is
**R7-X-** wherein X is an -NH-, or -N(C₁₋₈ alkyl)- moiety and
**R7'** is selected from the group consisting of H, alkyl, cycloalkyl, aryl, alkylcycloalkyl, aryl alkyl, heterocycle, heterocycloalkyl, substituted alkyl, substituted cycloalkyl, substituted aryl, substituted arylalkyl, substituted heterocycle, substituted heteroaryl, substituted heteroaryl alkyl, substituted heteroalkyl, substituted cycloalkyl alkyl and substituted cycloheteroalkyl alkyl for the preparation of a composition for treating cancer comprising administering effective amount of the 3-, 7-disubstituted pylazolo[4,3-d]pyrimidines or a pharmaceutically acceptable salt thereof in combination with usually used cytostatics, such as mitoxantrone, cis-platinum, methotrexate, taxol, or doxorubicin.

12. Use of a 3,7-disubstituted pyrazolo[4,3-d]pyrimidine of the formula I 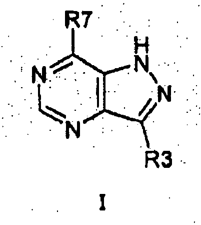 and pharmaceutically acceptable salts thereof, wherein
**R3** is selected from the group consisting of
C₁₋₈ alkyl, C₂₋₈ alkenyl, C₃₋₈ alkinyl, wherein each of the groups may optionally be substituted.
**R7** is
**R7'-X-** wherein **X** is an -NH-, or -N(C₁₋₈ alkyl)- moiety and
**R7'** is selected from the group consisting of H, alkyl, cycloalkyl, aryl, alkylcycloalkyl, aryl alkyl, heterocycle, heterocycloalkyl, substituted alkyl, substituted cycloalkyl, substituted aryl, substituted arylalkyl, substituted heterocycle, substituted heteroaryl, substituted heteroaryl alkyl, substituted heteroalkyl, substituted cycloalkyl alkyl and substituted cycloheteroalkyl alkyl for the preparation of a composition for treating an hyperproliferative skin disease in a human suffering therefrom by actinic keratosis, Bowen'sdisease, papilloma, seborrheic keratosis, toxic eczema, atopic dermatitis and ichthyosis.

13. Use of a 3,7-disubatituted pyrazolo[4,3-d]pyrimidine of the formula I 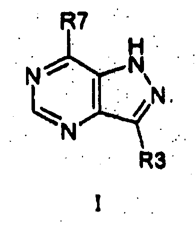 and pharmaceutically acceptable salts thereof, wherein
**R3** is selected from the group consisting of
C₁₋₈ alkyl, C₂₋₈ alkenyl, C₃₋₈ alkinyl, wherein each of the groups may optionally be substituted,
**R7** is
**R7'-X-** wherein X is an -NH-, or -N(alkyl)- moiety and
**R7'** is selected from the group consisting of H, alkyl, cycloalkyl, aryl, alkylcycloalkyl, aryl alkyl, heterocycle, heterocycloalkyl, substituted alkyl, substituted cycloalkyl, substituted aryl, substituted arylalkyl, substituted heterocycle, substituted heteroaryl, substituted heteroaryl alkyl, substituted heteroalkyl, substituted cycloalkyl alkyl and substituted cycloheteroalkyl alkyl for the preparation of a composition for treating viral infections.

14. 3,7-disubstituted pyrazolo[4,3-d]pyrimidines 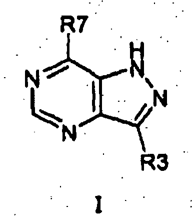 and pharmaceutically acceptable salts thereof, wherein
**R3** is selected from the group consisting of
C₁₋₈ alkyl, C₂₋₈ alkenyl, C₃₋₈ alkinyl, wherein each of the groups may optionally be substituted,
**R7** is
**R7'-X-** wherein X is an -NH-, or -N(alkyl)- moiety and
**R7'** is selected from the group consisting of H, alkyl, cycloalkyl, aryl, alkylcycloalkyl, aryl alkyl, heterocycle, heterocycloalkyl, substituted alkyl, substituted cycloalkyl, substituted aryl, substituted arylalkyl, substituted heterocycle, substituted heteroaryl, substituted heteroaryl alkyl, substituted heteroalkyl, substituted cycloalkyl alkyl and substituted cycloheteroalkyl alkyl for the preparation of a composition as a therapeutic agent.

15. Use of 3-, 7-disubstituted pyrazolo[4,3-d]pyrimidines according to claims 1 to 4 in the preparation of a medicament for the treatment of cancer, or psoriasis, rheumatoid arthritis, lupus, type I diabetes, multiple sclerosis, restenosis, polycystic kidney disease, graft rejection, graft versus host disease and gout, palasitoses such as those caused by fungi or protists, or Alzheimer's disease, asthma, actinic keratosis, Bowen's disease, papilloma, seborrheic keratosis, toxic eczema, atopic dermatitis and ichthyosis, cardiovascular, neurodegenerative, viral and inflammatory diseases.

16. 3,7 disubstituted pyrazolo[4,3-d]pyrimidines according to any one of claim 1 to 4 for use as a therapeutic agent.

## Patentansprüche

1. 3-,7-disubstituierte Pyrazolo[4,3-d]Pyrimidine nach der allgemeinen Formel I 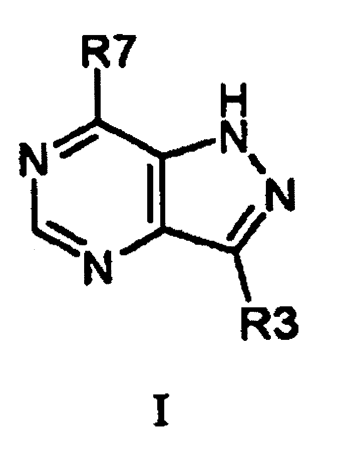 und pharmazeutisch geeignete Salze davon, wobei
R3 Ethyl, Isopropyl, n-Butyl, Isobutyl, tert.-Butyl, 3-Pentyl, Benzyl, Cyclopropyl, Alkenyl, Alkinyl ist, wobei jede der Gruppen optional mit Halogen, Hydroxyl, Amino, Mercapto, Carboxyl, Cyano, Nitro, Amido, Sulfo, Sulfamido, Carbamino, Alkyl, Alkoxy, und/oder mit einer mit einem dieser Substituenten substituierten Alkylgruppe substituiert sein kann.
R7
R7'-X ist, wobei X eine -NH-, oder- N(Alkyl)- Gruppe ist, und
R7' ausgewählt ist aus der Gruppe bestehend aus H, Alkyl, Cycloalkyl, Aryl, Alkylcycloalkyl, Arylalkyl, Heterocyklus, Hetercykloalkyl, substituiertes Alkyl, substituiertes Cycloalkyl, substituiertes Aryl, substituiertes Arylalkyl, substituierter Heterocyklus, substituiertes Heteroaryl, substituiertes Heteroarylalkyl, substituiertes Heteroalkyl, substituiertes Cycloalkylalkyl, und substituiertes Cycloheteroalkylalkyl.

2. 3-,7-disubstituierte Pyrazolo[4,3-d]Pyrimidine gemäß Anspruch 1, wobei die Gruppen mit mehr als einer Halogen-, Hydroxyl-, Amino-, Mercapto-, Carboxyl-, Cyano-, Nitro-, Amido-, Sulfo-, Sulfamido-, Carbamino-, Alkyl-, Alkoxygruppe, und/oder einer substituierten Alkylgruppe, substituiert sind.

3. 3-,7-disubstituierte Pyrazolo[4,3-d]Pyrimidine gemäß den Ansprüchen 1 oder 2, wobei
R3
Ethyl, Isopropyl, n-Butyl, Isobutyl, tert.-Butyl, 3-Pentyl, Benzyl, Cyclopropyl, C₂-C₈ Alkenyl, C₃-C₈ Alkinyl ist, welche mit 1-3 Halogenen, wie Fluor und Chlor, substituiert sein können;
R7
R7'-X ist, wobei X das -NH-, oder -N(Alkyl)- ist, und
R7'-X, wobei X bevorzugter Weise -N(Alkyl)- ist, ausgewählt bei jedem Vorkommen aus der Gruppe Methyl, Ethyl, Propyl, Isopropyl, Ethinyl, Allyl, Propargyl, Isopent-2-en-1-yl.

4. 3-,7-disubstituierte Pyrazolo[4,3-d]Pyrimidine gemäß einem der vorstehenden Ansprüche, welche, im Falle ihrer Chiralität, unabhängig voneinander bei jedem Vorkommen, die (R) oder (S) Konfiguration in R3 oder R7 haben.

5. Ein Verfahren zur Herstellung des 3-,7-disubstituierten Pyrazolo[4,3-d]Pyrimidins der Formel I, 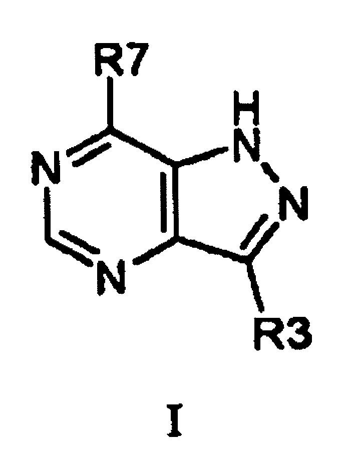 und pharmazeutisch geeigneter Salze davon, wobei
R3 ausgewählt ist aus der Gruppe bestehend aus
C₁-C₈ Alkyl, C₂-C₈ Alkenyl, C₃-C₈Alkinyl, wobei jede der Gruppen optional substituiert sein kann,
R7
R7'-X ist, wobei X eine -NH-, oder - N(C₁-C₈ Alkyl)-Gruppe ist, und
R7' ausgewählt ist aus der Gruppe bestehend aus H, Alkyl, Cycloalkyl, Aryl, Alkylcycloalkyl, Arylalkyl, Heterocyclus, Hetercycloalkyl, substituiertes Alkyl, substituiertes Cycloalkyl, substituiertes Aryl, substituiertes Arylalkyl, substituierter Heterocyclus, substituiertes Heteroaryl, substituiertes Heteroarylalkyl, substituiertes Heteroalkyl, substituiertes Cycloalkylalkyl, und substituiertes Cycloheteroalkylalkyl, wobei 3-substituierte-7-Hydroxypyrazolo[4,3-d]Pyrimidine chloriert werden, bevorzugt mit SOCl₂ oder POCl₃ oder PCl₅, oder die 7-Hydroxyguppe durch eine Mercaptogruppe ersetzt wird, bevorzugt durch die Einwirkung von P₂S₅, wonach die so gewonnenen 7-Chlor-3-substituierten Pyrazolo[4,3-d]Pyrimidine mit einem geeigneten nukleophilen Reaktionsmittel zur Reaktion versetzt werden, oder die 7-Mercapto-3-substituierten Pyrazolo[4,3-d]Pyrimidine mit einem Alkylierungsmittel zur Reaktion versetzt werden und
das resultierende 3-,7-disubstituierte Pyrazolo[4,3-d]Pyrimidin optional isoliert wird.

6. Das Verfahren von Anspruch 5, wobei das geeignete Nukleophil R7'-X-Y ausgewählt ist aus der Gruppe bestehend aus
1) Amin, Ammoniumhydroxid, Hydrazin, Hydroxylamin, Benzylamin, 2-, 3-, 4-, Hydoxybenzylamin; Dihydroxybenzylamin; und 3-Chloranilin; oder
2) Lithium, Natrium und Kaliumsalz von Alkoholen oder Mercaptan.

7. Das Verfahren der Ansprüche 5 oder 6, wobei das Alkylierungsmittel ausgewählt ist aus der Gruppe bestehend aus Alkylhalogenid, Alkyltoluensulfonat, und Alkylmesylat.

8. Das Verfahren der Ansprüche 5 bis 7, wobei das resultierende 3-,7-disubstituierte Pyrazolo[4,3-d]Pyrimidin durch Chromatographie auf Silikagel, gefolgt von Kristallisation oder nur durch Kristallisation, isoliert wird.

9. Verwendung eines 3-,7-disubstituierten Pyrazolo[4,3-d]Pyrimidines der Formel I, 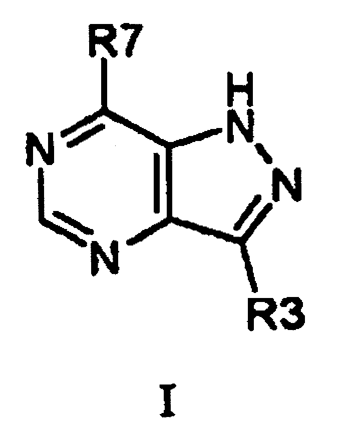 und pharmazeutisch geeigneter Salze davon, wobei
R3 ausgewählt ist aus der Gruppe bestehend aus
C₁-C₈ Alkyl, C₂-C₈ Alkenyl, C₃-C₈Alkinyl, wobei jede der Gruppen optional substituiert sein kann,
R7
R7'-X ist, wobei X eine -NH- oder -N(C₁-C₈ Alkyl)- Gruppe ist, und
R7' ausgewählt ist aus der Gruppe bestehend aus H, Alkyl, Cycloalkyl, Aryl, Alkylcycloalkyl, Arylalkyl, Heterocyclus, Hetercycloalkyl, substituiertes Alkyl, substituiertes Cycloalkyl, substituiertes Aryl, substituiertes Arylalkyl, substituierter Heterocyclus, substituiertes Heteroaryl, substituiertes Heteroarylalkyl, substituiertes Heteroalkyl, substituiertes Cycloalkylalkyl, und substituiertes Cycloheteroalkylalkyl, zur Herstellung einer Zusammensetzung für die Inhibition der Zellproliferation in Säugetieren.

10. Verwendung eines 3-,7-disubstituierten Pyrazolo[4,3-d]Pyrimidins der Formel I, 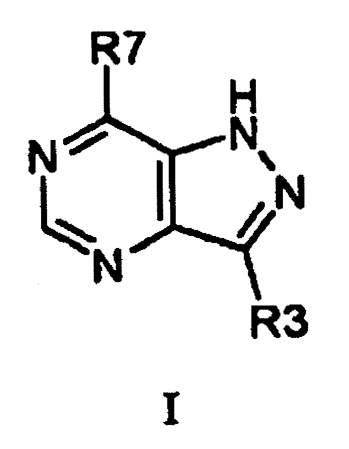 und pharmazeutisch geeigneter Salze davon, wobei
R3 ausgewählt ist aus der Gruppe bestehend aus
C₁-C₈ Alkyl, C₂-C₈ Alkenyl, C₃-C₈Alkinyl, wobei jede der Gruppen optional substituiert sein kann,
R7
R7'-X ist, wobei X eine -NH-, oder -N(C₁-C₈ Alkyl)-Gruppe ist, und
R7' ausgewählt ist aus der Gruppe bestehend aus H, Alkyl, Cycloalkyl, Aryl, Alkylcycloalkyl, Arylalkyl, Heterocyclus, Hetercycloalkyl, substituiertes Alkyl, substituiertes Cycloalkyl, substituiertes Aryl, substituiertes Arylalkyl, substituierter Heterocyclus, substituiertes Heteroaryl, substituiertes Heteroarylalkyl, substituiertes Heteroalkyl, substituiertes Cycloalkylalkyl, und substituiertes Cycloheteroalkylalkyl, zur Herstellung einer Zusammensetzung für die Behandlung von Krebs, Psoriasis, rheumatoider Artrithis, Lupus, Diabetes Typ I, multipler Sklerose, Restenose, polyzystischer Nierenkrankheit, Transplantatabstoßung, Graft-Versus-Host Krankheit und Gicht, Parasitosen, wie solche, die durch Pilze oder Protisten verursacht werden, oder M. Alzheimer, oder als antineurodegenerative Arzneien oder zur Unterdrückung der Immunstimulation.

11. Verwendung eines 3-,7-disubstituierten Pyrazolo[4,3-d]Pyrimidines der Formel I, 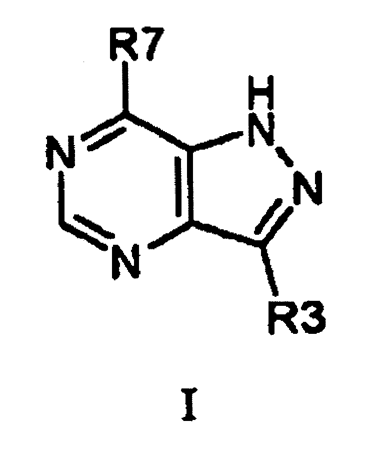 und pharmazeutisch geeigneter Salze davon, wobei
R3 ausgewählt ist aus der Gruppe bestehend aus
C₁-C₈ Alkyl, C₂-C₈ Alkenyl, C₃-C₈Alkinyl, wobei jede der Gruppen optional substituiert sein kann,
R7
R7'-X ist, wobei X eine -NH-,oder -N(C₁-C₈ Alkyl)-Gruppe ist, und
R7' ausgewählt ist aus der Gruppe bestehend aus H, Alkyl, Cycloalkyl, Aryl, Alkylcycloalkyl, Arylalkyl, Heterocyclus, Hetercycloalkyl, substituiertes Alkyl, substituiertes Cycloalkyl, substituiertes Aryl, substituiertes Arylalkyl, substituierter Heterocyclus, substituiertes Heteroaryl, substituiertes Heteroarylalkyl, substituiertes Heteroalkyl, substituiertes Cycloalkylalkyl, und substituiertes Cycloheteroalkylalkyl, für die Herstellung einer Zusammensetzung zur Behandlung von Krebs, umfassend die Verabreichung einer wirksamen Menge der 3-,7-disubstituierten Pyrazolo[4,3-d]Pyrimidine oder eines pharmazeutisch wirksamen Salzes davon in Kombination mit üblicher Weise verwendeten Zytostatika, wie Mitoxantron, Cisplatin, Methotrexat, Taxol, oder Doxorubizin.

12. Verwendung eines 3-,7-disubstituierten Pyrazolo[4,3-d]Pyrimidins der Formel I, 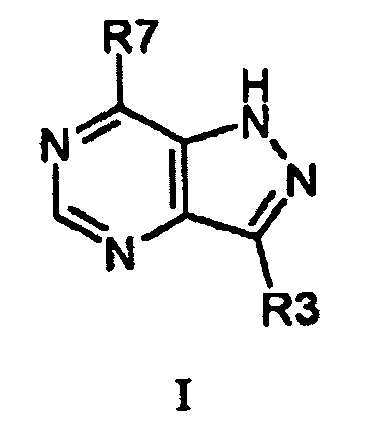 und pharmazeutisch geeigneter Salze davon, wobei
R3 ausgewählt ist aus der Gruppe bestehend aus
C₁-C₈ Alkyl, C₂-C₈ Alkenyl, C₃-C₈Alkinyle, wobei jede der Gruppen optional substituiert sein kann,
R7
R7'-X ist, wobei X eine -NH-, oder -N(C₁-C₈ Alkyl)-Gruppe ist, und
R7' ausgewählt ist aus der Gruppe bestehend aus H, Alkyl, Cycloalkyl, Aryl, Alkylcycloalkyl, Arylalkyl, Heterocyclus, Hetercycloalkyl, substituiertes Alkyl, substituiertes Cycloalkyl, substituiertes Aryl, substituiertes Arylalkyl, substituiertes Heterocyclus, substituiertes Heteroaryl, substituiertes Heteroarylalkyl, substituiertes Heteroalkyl, substituiertes Cycloalkylalkyl, und substituiertes Cycloheteroalkylalkyl, zur Herstellung einer Zusammensetzung für die Behandlung einer hyperproliferativen Hauterkrankung bei Menschen, die unter einer solchen Krankheit wie aktinische Keratose, Bowen-Krankheit, Papillom, seborrhoische Keratose, toxisches Ekzem, atopische Dermatitis und Ichthyosis leiden.

13. Verwendung eines 3-,7-disubstituierten Pyrazolo[4,3-d]Pyrimidines der Formel I, 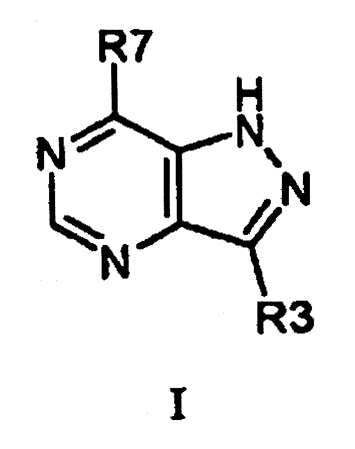 und pharmazeutisch geeigneter Salze davon, wobei
R3 ausgewählt ist aus der Gruppe bestehend aus
C₁-C₈ Alkyl, C₂-C₈ Alkenyl, C₃-C₈Alkinyl, wobei jede der Gruppen optional substituiert sein kann,
R7
R7'-X ist, wobei X eine -NH-, oder -N(Alkyl)-Gruppe ist, und
R7' ausgewählt ist aus der Gruppe bestehend aus H, Alkyl, Cycloalkyl, Aryl, Alkylcycloalkyl, Arylalkyl, Heterocyclus, Hetercycloalkyl, substituiertes Alkyl, substituiertes Cycloalkyl, substituiertes Aryl, substituiertes Arylalkyl, substituierter Heterocyclus, substituiertes Heteroaryl, substituiertes Heteroarylalkyl, substituiertes Heteroalkyl, substituiertes Cycloalkylalkyl, und substituiertes Cycloheteroalkylalkyl, zur Herstellung einer Zusammensetzung für die Behandlung von Virusinfektionen.

14. 3-,7-disubstituierte Pyrazolo[4,3-d]Pyrimidine 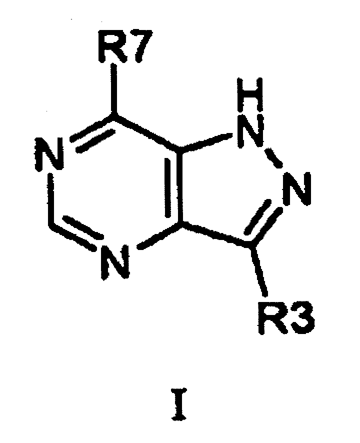 und pharmazeutisch geeigneter Salze davon, wobei
R3 ausgewählt ist aus der Gruppe bestehend aus
C₁-C₈ Alkyl, C₂-C₈ Alkenyl, C₃-C₈Alkinyl, wobei jede der Gruppen optional substituiert sein kann,
R7
R7'-X ist, wobei X eine -NH-, oder -N(Alkyl)-Gruppe ist, und
R7' ausgewählt ist aus der Gruppe bestehend aus H, Alkyl, Cycloalkyl, Aryl, Alkylcycloalkyl, Arylalkyl, Heterocyclus, Hetercycloalkyl, substituiertes Alkyl, substituiertes Cycloalkyl, substituiertes Aryl, substituiertes Arylalkyl, substituierter Heterocyclus, substituiertes Heteroaryl, substituiertes Heteroarylalkyl, substituiertes Heteroalkyl, substituiertes Cycloalkylalkyl, und substituiertes Cycloheteroalkylalkyl, für die Herstellung einer Zusammensetzung als Therapeutikum.

15. Verwendung der 3-,7-disubstituierten Pyrazolo[4,3-d]Pyrimidine gemäß den Ansprüchen 1 bis 4 zur Herstellung eines Medikamentes für die Behandlung von Krebs, oder Psoriasis, rheumatoider Arthritis, Lupus, Diabetes Typ I, multipler Sklerose, Restenose, polyzystischer Nierenkrankheit, Transplantatabstoßung, Graft-Versus-Host Krankheit und Gicht, Parasitosen, wie solche, die durch Pilze oder Protisten verursacht werden, oder M. Alzheimer, Asthma, aktinischer Keratose, Bowen-Krankheit, Papillom, seborrhoischer Keratose, toxische Ekzeme, atopischer Dermatitis und Ichthyosis, kardiovaskularen, neurodegenerativen, viralen und inflammatorischen Krankheiten.

16. 3-,7-disubstituierte Pyrazolo[4,3-d]Pyrimidine gemäß einem der Ansprüche 1 bis 4 für die Verwendung als Therapeutikum.

## Revendications

1. Pyrazolo[4,3-d]pyrimidines 3-,7-disubstitués, representés par la formule générale I 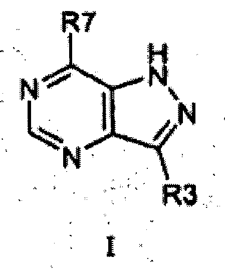 et leurs sels pharmaceutiquement acceptables, où
R3 est ethyle, isopropyle, n-butyle, isobutyle, tert-butyle, 3-pentyle, benzyle, cyclopropyle, alkényle, alkynyle, où chaque groupe peut éventuellement être substitué par halogène, hydroxyle, amino, mercapto, carboxyle, cyano, nitro, amido, sulpho, sulphamido, carbamino, alkyle, alcoxy et/ou un groupe alkyle substitué par un de ces substituants;
R7 est
R7'-X-, où X est un groupe -NH- ou -N(alkyle)-, et
R7' est choisi dans le groupe comprenant H, alkyle, cycloalkyle, aryle, alkylcycloalkyle, arylalkyle, hétérocycle, hétérocycloalkyle, alkyle substitué, cycloalkyle substitué, aryle substitué, arylalkyle substitué, hétérocycle substitué, hétéroaryle substitué, hétéroarylalkyle substitué, hétéroalkyle substitué, cycloalkylalkyle substitué, et cyclohétéroalkylalkyle substitué.

2. Pyrazolo[4,3-d]pyrimidines 3-,7-disubstitués selon la revendication 1, où les groupes sont substitués par plus d'un halogène, hydroxyle, amino, mercapto, carboxyle, cyano, nitro, amido, sulpho, sulphamido, carbamino, alkyle, alcoxy et/ou un groupe alkyle substitué.

3. Pyrazolo[4,3-d]pyrimidines 3-,7-disubstitués selon la revendication 1 ou 2, où
R3 est
ethyle, isopropyle, n-butyle, isobutyle, tert-butyle, 3-pentyle, benzyle, cyclopropyle, C₂₋₈ alkényle, C₃₋₈ alkynyle, qui peuvent être substitués par 1-3 halogènes comme fluoro et chloro;
R7 est
R7'-X, où X est -NH- ou -N(alkyle)- et
R7'-X, où X est de préférence -N(alkyle)- choisi à chaque occurence dans un groupe méthyle, ethyle, propyle, isopropyle, ethynyle, allyle, propargyle, isopent-2-én-1-yle.

4. Pyrazolo[4,3-d]pyrimidines 3-,7-disubstitués selon une quelconque des revendications précédentes, qui ont indépendamment à chaque occurence la configuration (R) ou (S) à R3 ou R7 dans le cas de chiralité.

5. Procédé de préparation d'un pyrazolo[4,3-d]pyrimidine 3,7-disubstitué de la formule I 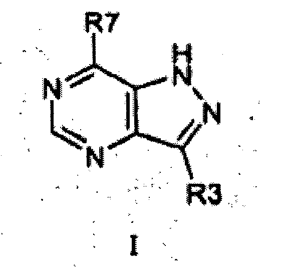 et ses sels pharmaceutiquement acceptables, où
R3 est choisi dans le groupe comprenant
C₁₋₈ alkyle, C₂₋₈ alkényle, C₃₋₈ alkynyle, où chaque groupe peut éventuellement être substitué,
R7 est
R7'-X-, où X est un groupe -NH- ou -N(C₁₋₈ alkyle)-, et
R7' est choisi dans le groupe comprenant H, alkyle, cycloalkyle, aryle, alkylcycloalkyle, arylalkyle, hétérocycle, hétérocycloalkyle, alkyle substitué, cycloalkyle substitué, aryle substitué, arylalkyle substitué, hétérocycle substitué, hétéroaryle substitué, hétéroarylalkyle substitué, hétéroalkyle substitué, cycloalkylalkyle substitué, et cyclohétéroalkylalkyle substitué, où 7-hydroxypyrazolo[4,3-d]pyrimidines 3-substitués sont chlorés, de préférence avec SOCl₂ ou POCl₃ ou PCl₅, ou le groupe 7-hydroxy est remplacé par un groupe mercapto, de préférence par le traitement avec P₂S₅,
les 7-chloropyrazolo[4,3-d]pyrimidines 3-substitués ainsi obtenus sont puis traités avec un agent nucléophile approprié, ou les 7-mercaptopyrazolo[4,3-d]pyrimidines 3-substitués sont traités avec un agent d'alkylation, et
le pyrazolo[4,3-d]pyrimidine 3,7-disubstitué résultant peut éventuellement être isolé.

6. Le procédé selon la revendication 5, où le nucléophile approprié R7'-X-Y est choisi dans le groupe comprenant
1) amine, hydroxyde d'ammonium, hydrazine, hydroxylamine, benzylamine; 2-, 3-, 4-hydroxybenzylamine, dihydroxybenzylamine, et 3-chloroaniline, ou
2) sel de lithim, sodium, potassium d'un alcool ou mercaptane.

7. Le procédé selon les revendications 5 ou 6, où l'agent d'alkylation est choisi dans la groupe comprenant halogenide d'alkyle, toluenesulphonate d'alkyle, et mésylate d'alkyle.

8. Le procédé selon les revendication 5 à 7, où le pyrazolo[4,3-d]pyrimidine 3,7-disubstitué résultant est isolé par la chromatographie sur le gel de silice, suivie par la crystallisation, ou seulement par la crystallisation.

9. Utilisation d'un pyrazolo[4,3-d]pyrimidine 3,7-disubstitué de la formule I 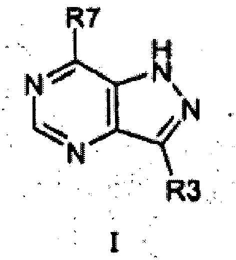 et ses sels pharmaceutiquement acceptables, où
R3 est choisi dans le groupe comprenant
C₁₋₈ alkyle, C₂₋₈ alkényle, C₃₋₈ alkynyle, où chaque groupe peut éventuellement être substitué,
R7 est
R7'-X-, où X est un groupe -NH- ou -N(C₁₋₈ alkyle)-, et
R7' est choisi dans le groupe comprenant H, alkyle, cycloalkyle, aryle, alkylcycloalkyle, arylalkyle, hétérocycle, hétérocycloalkyle, alkyle substitué, cycloalkyle substitué, aryle substitué, arylalkyle substitué, hétérocycle substitué, hétéroaryle substitué, hétéroarylalkyle substitué, hétéroalkyle substitué, cycloalkylalkyle substitué, et cyclohétéroalkylalkyle substitué, pour la préparation d'une composition destinée à l'inhibition de la prolifération des cellules dans les mammifères.

10. Utilisation d'un pyrazolo[4,3-d]pyrimidine 3,7-disubstitué de la formule I 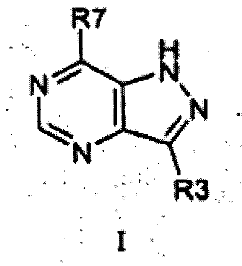 et ses sels pharmaceutiquement acceptables, où
R3 est choisi dans le groupe comprenant
C₁₋₈ alkyle, C₂₋₈ alkényle, C₃₋₈ alkynyle, où chaque groupe peut éventuellement être substitué,
R7 est
R7'-X-, où X est un groupe -NH-, ou -N(C₁₋₈ alkyle)-, et
R7' est choisi dans le groupe comprenant H, alkyle, cycloalkyle, aryle, alkylcycloalkyle, arylalkyle, hétérocycle, hétérocycloalkyle, alkyle substitué, cycloalkyle substitué, aryle substitué, arylalkyle substitué, hétérocycle substitué, hétéroaryle substitué, hétéroarylalkyle substitué, hétéroalkyle substitué, cycloalkylalkyle substitué, et cyclohétéroalkylalkyle substitué, pour la préparation d'une composition destinée au traitement du cancer ou psoriasis, polyarthrite rhumatoïde, lupus, diabète de type I, sclerose en plaque, resténose, maladie polycystique du rein, rejet de greffe, maladie du greffon contre l'hôte et goutte, infections parasitaires, comme fongiques ou causées par les protistes ou maladie d'Alzheimer, ou les médicaments anti-neurodégénératives ou pour la suppréssion de l'immunostimulation.

11. Utilisation d'un pyrazolo[4,3-d]pyrimidine 3,7-disubstitué de la formule I 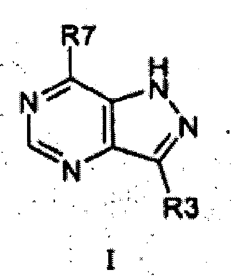 et ses sels pharmaceutiquement acceptables, où
R3 est choisi dans le groupe comprenant
C₁₋₈ alkyle, C₂₋₈ alkényle, C₃₋₈ alkynyle, où chaque groupe peut éventuellement être substitué,
R7 est
R7'-X-, où X est un groupe -NH-, ou -N(C₁₋₈ alkyle)-, et
R7' est choisi dans le groupe comprenant H, alkyle, cycloalkyle, aryle, alkylcycloalkyle, arylalkyle, hétérocycle, hétérocycloalkyle, alkyle substitué, cycloalkyle substitué, aryle substitué, arylalkyle substitué, hétérocycle substitué, hétéroaryle substitué, hétéroarylalkyle substitué, hétéroalkyle substitué, cycloalkylalkyle substitué, et cyclohétéroalkylalkyle substitué, pour la préparation d'une composition destinée au traitement du cancer, comprenant l'administration d'une quantité efficace du pyrazolo[4,3-d]pyrimidine 3,7-disubstitué ou son sel pharmaceutiquement acceptable, en combinaison avec les cytostatiques habituellement utilisés, comme mitoxantrone, cisplatine, methotrexate, taxole, ou doxorubicine.

12. Utilisation d'un pyrazolo[4,3-d]pyrimidine 3,7-disubstitué de la formule I 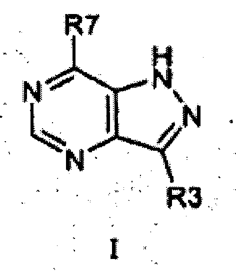 et ses sels pharmaceutiquement acceptables, où
R3 est choisi dans le groupe comprenant
C₁₋₈ alkyle, C₂₋₈ alkényle, C₃₋₈ alkynyle, où chaque groupe peut éventuellement être substitué,
R7 est
R7'-X-, où X est un groupe -NH-, ou -N(C₁₋₈ alkyle)-, et
R7' est choisi dans le groupe comprenant H, alkyle, cycloalkyle, aryle, alkylcycloalkyle, arylalkyle, hétérocycle, hétérocycloalkyle, alkyle substitué, cycloalkyle substitué, aryle substitué, arylalkyle substitué, hétérocycle substitué, hétéroaryle substitué, hétéroarylalkyle substitué, hétéroalkyle substitué, cycloalkylalkyle substitué, et cyclohétéroalkylalkyle substitué, pour la préparation d'une composition destinée au traitement d'une maladie hyperproliferative de la peau dans un humain souffrant de cela, telle que kératose actinique, maladie de Bowen, papilloma, kératose séborrhéique, eczéma toxique, dermatite atopique, et ichthyosis.

13. Utilisation d'un pyrazolo[4,3-d]pyrimidine 3,7-disubstitué de la formule I 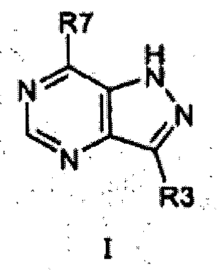 et ses sels pharmaceutiquement acceptables, où
R3 est choisi dans le groupe comprenant
C₁₋₈ alkyle, C₂₋₈ alkényle, C₃₋₈ alkynyle, où chaque groupe peut éventuellement être substitué,
R7 est
R7'-X-, où X est un groupe -NH-, ou -N(alkyle)-, et
R7' est choisi dans le groupe comprenant H, alkyle, cycloalkyle, aryle, alkylcycloalkyle, arylalkyle, hétérocycle, hétérocycloalkyle, alkyle substitué, cycloalkyle substitué, aryle substitué, arylalkyle substitué, hétérocycle substitué, hétéroaryle substitué, hétéroarylalkyle substitué, hétéroalkyle substitué, cycloalkylalkyle substitué, et cyclohétéroalkylalkyle substitué, pour la préparation d'une composition destinée au traitement des infections virales.

14. Pyrazolo[4,3-d]pyrimidines 3,7-disubstitués 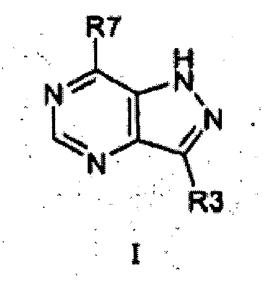 et leurs sels pharmaceutiquement acceptables, où
R3 est choisi dans le groupe comprenant
C₁₋₈ alkyle, C₂₋₈ alkényle, C₃₋₈ alkynyle, où chaque groupe peut éventuellement être substitué,
R7 est
R7'-X-, où X est un groupe -NH-, ou -N(alkyle)-, et
R7' est choisi dans le groupe comprenant H, alkyle, cycloalkyle, aryle, alkylcycloalkyle, arylalkyle, hétérocycle, hétérocycloalkyle, alkyle substitué, cycloalkyle substitué, aryle substitué, arylalkyle substitué, hétérocycle substitué, hétéroaryle substitué, hétéroarylalkyle substitué, hétéroalkyle substitué, cycloalkylalkyle substitué, et cyclohétéroalkylalkyle substitué, pour la préparation d'une composition comme un agent thérapeutique.

15. Utilisation des pyrazolo[4,3-d]pyrimidines 3,7-disubstitués selon les revendications 1 à 4 pour la préparation d'un médicament destiné au traitement du cancer, psoriasis, polyarthrite rhumatoïde, lupus, diabète de type I, sclerose en plaque, resténose, maladie polycystique du rein, rejet de greffe, maladie du greffon contre l'hôte et goutte, infections parasitaires, comme fongiques ou causées par les protistes, ou maladie d'Alzheimer, asthme, kératose actinique, maladie de Bowen, papilloma, kératose séborrhéique, eczéma toxique, dermatite atopique et ichthyosis, maladies cardiovasculaires, neurodégénératives, virales et inflammatoires.

16. Pyrazolo[4,3-d]pyrimidines 3,7-disubstitués selon une quelconque des revendications 1 à 4 pour l'utilisation comme un agent thérapeutique.
